# EUROPEAN PATENT APPLICATION

(11) **EP 4 101 848 A1**
(43) Date of publication of application: **14.12.2022**
(21) Application number: 21759622.0
(22) Date of filing: 22.02.2021
(51) Int. Cl.: C07D 401/08, C07D 471/04, C07D 209/46, A61K 31/4184, A61K 31/4035, A61K 31/4436, A61P 37/02, A61P 29/00

(54) **PDE4 INHIBITOR COMPOUND AND MEDICAL USE THEREOF**

(30) Priority: 24.02.2020 CN 202010114076; 28.07.2020 CN 202010748673; 28.08.2020 CN 202010881875
(71) Applicant: Suzhou Longbotai Pharmaceuticals Co., Ltd., Suzhou, Jiangsu 215126 (CN)
(72) Inventor: LI, Shuxin, Suzhou, Jiangsu 215126 (CN); YANG, Hengying, Suzhou, Jiangsu 215126 (CN); KUANG, Sheng, Suzhou, Jiangsu 215126 (CN); SHE, Sifan, Suzhou, Jiangsu 215126 (CN); WU, Kuiwang, Suzhou, Jiangsu 215126 (CN); LI, Shiwei, Suzhou, Jiangsu 215126 (CN)
(74) Representative: Maiwald GmbH
(86) International application number: PCT/CN2021/077232
(87) International publication number: WO 2021/169913

(57) **Abstract**

A class of PDE4 inhibitor compounds, and an isomer, solvate, deuterated derivative, or pharmaceutically acceptable salt thereof. A drug that comprises the compound, isomer, solvate, deuterated derivative, and pharmaceutically acceptable salt thereof, and a use thereof in preparing a drug for treating diseases related to PDE4 inhibition, such as autoimmune diseases and cancer.

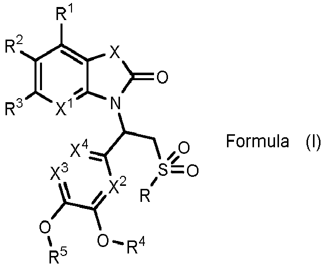

## Description

### REFERENCE TO RELATED APPLICATIONS

This application claims the priority of Chinese patent applications CN202010114076.2, under the title of "PDE4 Inhibitor Compound And Medical Use Thereof" filed on February 24, 2020; CN202010748673.0, under the title of "PDE4 Inhibitor Compound And Medical Use Thereof" filed on July 28, 2020; and CN202010881875.2, under the title of "PDE4 Inhibitor Compound And Medical Use Thereof" filed on August 28, 2020, the entire content of each of which is incorporated herein by reference in its entirety.

### FIELD OF THE INVENTION

The present disclosure relates to a class of PDE4 inhibitor compounds, and isomers, solvates, deuterated derivatives, or pharmaceutically acceptable salts thereof, to a medicament comprising the compound, or the salt thereof as an active ingredient, and to a use of the same in the manufacture of a medicament for treating diseases related to PDE4, such as autoimmune diseases and cancers.

### SUMMARY OF THE INVENTION

A purpose of the present disclosure is to provide a compound of formula (I) and an isomer, solvate, deuterated derivative or pharmaceutically acceptable salt thereof: 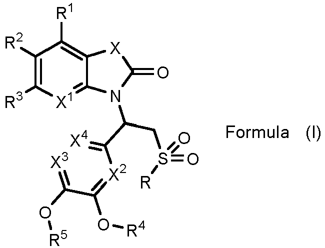 wherein:
X¹ is selected from N or CH;
one of X², X³, and X⁴ is selected from N, and the others are CH;
X is selected from O, -N(R⁶), and-CH(R⁶)-;
R is selected from hydrogen, C₁-C₃ alkyl, and C₃-C₆ cycloalkyl, and may be substituted with halogen;
R¹, R², and R³ are each independently selected from hydrogen, halogen, hydroxyl, -CN, -NH₂, C1-C6 alkyl, C1-6 alkoxy, C1-C6 alkylamino, C2-6 alkenyl, C3-6 cycloalkenyl, 3- to 6-membered heterocycloalkenyl, C3-C6 cycloalkyl, 3- to 6-membered heterocycloalkyl, phenyl, 5- to 6-membered heteroaryl, and -COOR⁸, which may be substituted with halogen, C1-C6 alkyl, optionally halogenated C1-C6 alkyl, C3-C6 cycloalkyl, or optionally halogenated C3-C6 cycloalkyl;
R⁴ and R⁵ are independently selected from C1-C6 alkyl and C3-C6 cycloalkyl, and may be optionally substituted with deuterium, tritium, or halogen;
R⁶ is selected from H, halogen, hydroxyl, -CN, -NH₂,-COOH, and R⁷-L¹-, or selected from C1-6 alkyl, C1-C6 heteroalkyl, C3-C6 cycloalkyl, 3- to 6-membered heterocycloalkyl, phenyl, 5- to 6-membered heteroaryl, -COOR⁹, -SO₂R¹⁰, -SO₂NHR¹¹, and -NHCONHR¹², which may be substituted with halogen, C1-C6 alkyl, optionally halogenated C1-C6 alkyl, C3-C6 cycloalkyl, or optionally halogenated C3-C6 cycloalkyl;
R⁷ is selected from C3-C6 cycloalkyl, 3- to 6-membered heterocycloalkyl, 3- to 6-membered heterocycloalkenyl, phenyl, 5- to 6-membered heteroaryl, and -COOR⁸, and may be substituted with halogen, C1-C6 alkyl, or C3-C6 cycloalkyl;
L¹ is selected from -CH₂-, -CH₂CH₂-, O, S, and NH;
R⁸, R⁹, R¹⁰, R¹¹, and R¹² are C1-C6 alkyl, halogenated C1-C6 alkyl, or C3-C6 cycloalkyl.

Alternatively, the compound of formula (I) and an isomer, solvate, deuterated derivative or pharmaceutically acceptable salt thereof are provided, wherein X is selected from:

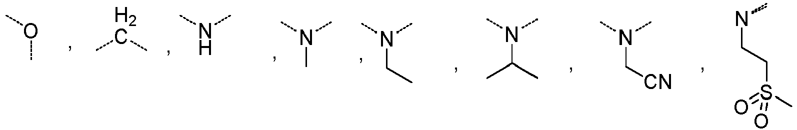

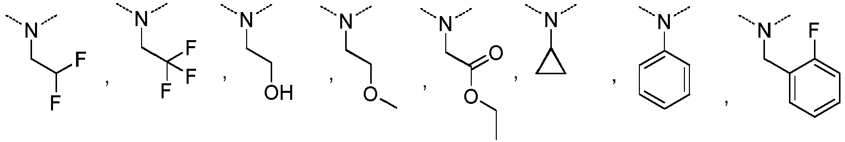

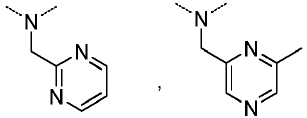

Alternatively, the compound of formula (I) and an isomer, solvate, deuterated derivative or pharmaceutically acceptable salt thereof are provided, wherein R¹, R², and R³ are independently selected from the following groups: 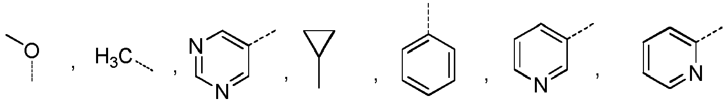 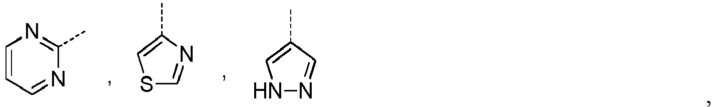 which may be substituted with halogen, -OH, -NH₂, -CN, C1-C6 alkyl, optionally halogenated C1-C6 alkyl, C3-C6 cycloalkyl, or optionally halogenated C3-C6 cycloalkyl.

Alternatively, the compound of formula (I) and an isomer, solvate, deuterated derivative or pharmaceutically acceptable salt thereof are provided, wherein the compound of formula (I) comprises the following structures:
3-(1-(6-ethoxy-5-methoxypyridin-2-yl)-2-(methylsulfonyl)ethyl)-6-(2-fluorophenyl)-7-methyl-1H-imidazo[4,5-b]pyridin-2(3H)-one;
(S)-3-(1-(6-ethoxy-5-methoxypyridin-2-yl)-2-(methylsulfonyl)ethyl)-6-(2-fluorophenyl) -7-methyl-1H-imidazo[4,5-b]pyridin-2(3H)-one;
(R)-3-(1-(6-ethoxy-5-methoxypyridin-2-yl)-2-(methylsulfonyl)ethyl)-6-(2-fluorophenyl) -7-methyl-1H-imidazo[4,5-b]pyridin-2(3H)-one;
(S)-3-(1-(6-ethoxy-5-methoxypyridin-2-yl)-2-(methylsulfonyl)ethyl)-7-methyl-6-phenyl -1H-imidazo[4,5-b]pyridin-2(3H)-one;
(R)-3-(1-(6-ethoxy-5-methoxypyridin-2-yl)-2-(methylsulfonyl)ethyl)-7-methyl-6-phenyl -1H-imidazo[4,5-b]pyridin-2(3H)-one;
(S)-3-(1-(6-ethoxy-5-methoxypyridin-2-yl)-2-(methylsulfonyl)ethyl)-6-(4-fluorophenyl) -7-methyl-1H-imidazo[4,5-b]pyridin-2(3H)-one;
(R)-3-(1-(6-ethoxy-5-methoxypyridin-2-yl)-2-(methylsulfonyl)ethyl)-6-(4-fluorophenyl) -7-methyl-1H-imidazo[4,5-b]pyridin-2(3H)-one;
(S)-3-(1-(6-ethoxy-5-methoxypyridin-2-yl)-2-(methylsulfonyl)ethyl)-7-methyl-6-(*o*-met hylphenyl)-1H-imidazo[4,5-b]pyridin-2(3H)-one;
(R)-3-(1-(6-ethoxy-5-methoxypyridin-2-yl)-2-(methylsulfonyl)ethyl)-7-methyl-6-(*o*-met hylphenyl)-1H-imidazo[4,5-b]pyridin-2(3H)-one;
S)-3-(1-(6-ethoxy-5-methoxypyridin-2-yl)-2-(methylsulfonyl)ethyl)-6-(2-ethylphenyl)-7-methyl-1H-imidazo[4,5-b]pyridin-2(3H)-one;
(S)-3-(1-(6-ethoxy-5-methoxypyridin-2-yl)-2-(methylsulfonyl)ethyl)-6-(2-isopropylphen yl)-7-methyl-1H-imidazo[4,5-b]pyridin-2(3H)-one;
(S)-3-(1-(6-ethoxy-5-methoxypyridin-2-yl)-2-(methylsulfonyl)ethyl)-6-(2-methoxyphen yl)-7-methyl-1H-imidazo[4,5-b]pyridin-2(3H)-one;
(R)-3-(l-(6-ethoxy-5-methoxypyridin-2-yl)-2-(methylsulfonyl)ethyl)-6-(2-methoxyphen yl)-7-methyl-1H-imidazo[4,5-b]pyridin-2(3H)-one;
(S)-3-(1-(6-ethoxy-5-methoxypyridin-2-yl)-2-(methylsulfonyl)ethyl)-7-methyl-6-(2-(trifl uoromethyl)phenyl)-1H-imidazo[4,5-b]pyridin-2(3H)-one;
(S)-2-(3-(l-(6-ethoxy-5-methoxypyridin-2-yl)-2-(methylsulfonyl)ethyl)-7-methyl-2-oxo-2,3-dihydro-1H-imidazo[4,5-b]pyridin-6-yl)benzonitrile;
(R)-2-(3-(1-(6-ethoxy-5-methoxypyridin-2-yl)-2-(methylsulfonyl)ethyl)-7-methyl-2-oxo -2,3-dihydro-1H-imidazo[4,5-b]pyridin-6-yl)benzonitrile;
(S)-3-(1-(6-ethoxy-5-methoxypyridin-2-yl)-2-(methylsulfonyl)ethyl)-7-methyl-6-(pyridi n-2-yl)-1H-imdazo[4,5-b]pyridin-2(3H)-one;
(R)-3-(1-(6-ethoxy-5-methoxypyridin-2-yl)-2-(methylsulfonyl)ethyl)-7-methyl-6-(pyridi n-2-yl)-1H-imidazo 4,5-b]pyridin-2(3H)-one;
(S)-3-(1-(6-ethoxy-5-methoxypyridin-2-yl)-2-(methylsulfonyl)ethyl)-1,7-dimethyl-6-(py ridin-2-yl)-1H-imidazo[4,5-b]pyridin-2(3H)-one;
(S)-3-(1-(6-ethoxy-5-methoxypyridin-2-yl)-2-(methylsulfonyl)ethyl)-1-(2-methoxyethyl )-7-methyl-6-(pyridin-2-yl)-1H-imidazo[4,5-b]pyridin-2(3H)-one;
(S)-3-(1-(6-ethoxy-5-methoxypyridin-2-yl)-2-(methylsulfonyl)ethyl)-6-(2-fluorophenyl) -1,7-dimethyl-1H-imidazo[4,5-b]pyridin-2(3H)-one;
(R)-3-(1-(6-ethoxy-5-methoxypyridin-2-yl)-2-(methylsulfonyl)ethyl)-6-(2-fluorophenyl) -1,7-dimethyl-1H-imidazo[4,5-b]pyridin-2(3H)-one;
(S)-6-(2,6-difluorophenyl)-3-(1-(6-ethoxy-5-methoxypyridin-2-yl)-2-(methylsulfonyl)et hyl)-7-methyl-1H-imidazo[4,5-b]pyridin-2(3H)-one;
(R)(S)-6-(2,6-difluorophenyl)-3-(1-(6-ethoxy-5-methoxypyridin-2-yl)-2-(methylsulfonyl )ethyl)-7-methyl-1H-imidazo[4,5-b]pyridin-2(3H)-one;
(S)-6-(2,3-difluorophenyl)-3-(1-(6-ethoxy-5-methoxypyridin-2-yl)-2-(methylsulfonyl)et hyl)-7-methyl-1H-imidazo[4,5-b]pyridin-2(3H)-one;
(R)-6-(2,3-difluorophenyl)-3-(1-(6-ethoxy-5-methoxypyridin-2-yl)-2-(methylsulfonyl)et hyl)-7-dimethyl-1H-imidazo[4,5-b]pyridin-2(3H)-one;
(S)-6-(2,5-difluorophenyl)-3-(1-(6-ethoxy-5-methoxypyridin-2-yl)-2-(methylsulfonyl)et hyl)-7-methyl-1H-imidazo[4,5-b]pyridin-2(3H)-one;
(S)-6-(2,4-difluorophenyl)-3-(1-(6-ethoxy-5-methoxypyridin-2-yl)-2-(methylsulfonyl)et hyl)-7-methyl-1H-imidazo[4,5-b]pyridin-2(3H)-one;
2,4,5-trifluorophenyl)-3-(1-(6-ethoxy-5-methoxypyridin-2-yl)-2-(methylsulfonyl) ethyl)-7-methyl-1H-imidazo[4,5-b]pyridin-2(3H)-one;
(R)-6-(2,5-difluorophenyl)-3-(1-(6-ethoxy-5-methoxypyridin-2-yl)-2-(methylsulfonyl)et hyl)-7-methyl-1H-imidazo[4,5-b]pyridin-2(3H)-one;
(S)-6-(2,4-dimethylphenyl)-3-(1-(6-ethoxy-5-methoxypyridin-2-yl)-2-(methylsulfonyl)et hyl)-7-methyl-1H-imidazo[4,5-b]pyridin-2(3H)-one;
(S)-6-(2,5-dimethylphenyl)-3-(1-(6-ethoxy-5-methoxypyridin-2-yl)-2-(methylsulfonyl)et hyl)-7-methyl-1H-imidazo[4,5-b]pyridin-2(3H)-one;
(S)-6-(2,3-dimethylphenyl)-3-(l-(6-ethoxy-5-methoxypyridin-2-yl)-2-(methylsulfonyl)et hyl)-7-methyl-1H-imidazo[4,5-b]pyridin-2(3H)-one;
(S)-6-(2,6-dimethylphenyl)-3-(1-(6-ethoxy-5-methoxypyridin-2-yl)-2-(methylsulfonyl)et hyl)-7-methyl-1H-imidazo[4,5-b]pyridin-2(3H)-one;
(S)-6-(4-fluoro-2-methylphenyl)-3-(1-(6-ethoxy-5-methoxypyridin-2-yl)-2-(methylsulfo nyl)ethyl)-7-methyl-1H-imidazo[4,5-b]pyridin-2(3H)-one;
(S)-6-(5-fluoro-2-methylphenyl)-3-(1-(6-ethoxy-5-methoxypyridin-2-yl)-2-(methylsulfo nyl)ethyl)-7-methyl-1H-imidazo[4,5-b]pyridin-2(3H)-one;
(S)-6-(2-fluoro-5-methylphenyl)-3-(1-(6-ethoxy-5-methoxypyridin-2-yl)-2-(methylsulfo nyl)ethyl)-7-methyl-1H-imidazo[4,5-b]pyridin-2(3H)-one;
(S)-6-(2-fluoro-3-methylphenyl)-3-(1-(6-ethoxy-5-methoxypyridin-2-yl)-2-(methylsulfo nyl)ethyl)-7-methyl-1H-imidazo[4,5-b]pyridin-2(3H)-one;
(S)-6-(2-fluoro-4-methylphenyl)-3-(1-(6-ethoxy-5-methoxypyridin-2-yl)-2-(methylsulfo nyl)ethyl)-7-methyl-1H-imidazo[4,5-b]pyridin-2(3H)-one;
(S)-6-(2-fluoro-5-methoxyphenyl)-3-(l-(6-ethoxy-5-methoxypyridin-2-yl)-2-(methylsulf onyl)ethyl)-7-methyl-1H-imidazo[4,5-b]pyridin-2(3H)-one;
(S)-6-(2-fluoro-3-methoxyphenyl)-3-(1-(6-ethoxy-5-methoxypyridin-2-yl)-2-(methylsulf onyl)ethyl)-7-methyl-1H-imidazo[4,5-b]pyridin-2(3H)-one;
(S)-6-(2-fluoro-4-methoxyphenyl)-3-(1-(6-ethoxy-5-methoxypyridin-2-yl)-2-(methylsulf onyl)ethyl)-7-methyl-1H-imidazo[4,5-b]pyridin-2(3H)-one;
(S)-6-(5-fluoro-2-methoxyphenyl)-3-(l-(6-ethoxy-5-methoxypyridin-2-yl)-2-(methylsulf onyl)ethyl)-7-methyl-1H-imidazo[4,5-b]pyridin-2(3H)-one;
(S)-6-(4-fluoro-2-methoxyphenyl)-3-(l-(6-ethoxy-5-methoxypyridin-2-yl)-2-(methylsulf onyl)ethyl)-7-methyl-1H-imidazo[4,5-b]pyridin-2(3H)-one;
(S)-6-(6-fluoro-2-methoxyphenyl)-3-(l-(6-ethoxy-5-methoxypyridin-2-yl)-2-(methylsulf onyl)ethyl)-7-methyl-1H-imidazo[4,5-b]pyridin-2(3H)-one;
(R)-3-(1-(6-ethoxy-5-methoxypyridin-2-yl)-2-(methylsulfonyl)ethyl)-l-ethyl-7-methyl-6 -phenyl-1H-imidazo[4,5-b]pyridin-2(3H)-one;
(S)-3-(l-(6-ethoxy-5-methoxypyridin-2-yl)-2-(methylsulfonyl)ethyl)-l-ethyl-6-(4-fluoro phenyl)-7-methyl-1H-imidazo[4,5-b]pyridin-2(3H)-one;
(R)-3-(1-(6-ethoxy-5-methoxypyridin-2-yl)-2-(methylsulfonyl)ethyl)-1-ethyl-6-(4-fluoro phenyl)-7-methyl-1H-imidazo[4,5-b]pyridin-2(3H)-one;
(S)-3-(1-(6-ethoxy-5-methoxypyridin-2-yl)-2-(methylsulfonyl)ethyl)-1-ethyl-7-methyl-6 -(*o*-methylphenyl)-1H-imidazo[4,5-b]pyridin-2(3H)-one;
(S)-3-(1-(6-ethoxy-5-methoxypyridin-2-yl)-2-(methylsulfonyl)ethyl)-6-(2-fluorophenyl) -7-methyl-1-(2,2,2-trifluoroethyl)-1H-imidazo[4,5-b]pyridin-2(3H)-one;
(R)-3-(1-(6-ethoxy-5-methoxypyridin-2-yl)-2-(methylsulfonyl)ethyl)-6-(2-fluorophenyl) -7-methyl-1-(2,2,2-trifluoroethyl)-1H-imidazo[4,5-b]pyridin-2(3H)-one;
(S)-3-(1-(6-ethoxy-5-methoxypyridin-2-yl)-2-(methylsulfonyl)ethyl)-7-methyl-6-phenyl -1-(2,2,2-trifluoroethyl)-1H-imidazo[4,5-b]pyridin-2(3H)-one;
(R)-3-(1-(6-ethoxy-5-methoxypyridin-2-yl)-2-(methylsulfonyl)ethyl)-7-methyl-6-phenyl -1-(2,2,2-trifluoroethyl)-1H-imidazo[4,5-b]pyridin-2(3H)-one;
(S)-3-(1-(6-ethoxy-5-methoxypyridin-2-yl)-2-(methylsulfonyl)ethyl)-7-methyl-6-(*o*-met hylphenyl)-1-(2,2,2-trifluoroethyl)-1H-imidazo[4,5-b]pyridin-2(3H)-one;
(S)-1-(2,2-difluoroethyl)-3-(1-(6-ethoxy-5-methoxypyridin-2-yl)-2-(methylsulfonyl)ethy l)-6-(2-fluorophenyl)-7-methyl-1H-imidazo[4,5-b]pyridin-2(3H)-one;
(S)-1-(2,2-difluoroethyl)-3-(1-(6-ethoxy-5-methoxypyridin-2-yl)-2-(methylsulfonyl)ethy 1)-7-methyl-6-phenyl-1H-imidazo[4,5-b]pyridin-2(3H)-one;
(S)-1-(2,2-difluoroethyl)-3-(1-(6-ethoxy-5-methoxypyridin-2-yl)-2-(methylsulfonyl)ethy l)-6-(4-fluorophenyl)-7-methyl-1H-imidazo[4,5-b]pyridin-2(3H)-one;
(S)-1-(2,2-difluoroethyl)-3-(1-(6-ethoxy-5-methoxypyridin-2-yl)-2-(methylsulfonyl)ethy 4,5-b]pyridin-2(3H)-one;
(S)-3-(1-(5-(difluoromethoxy)-6-ethoxypyridin-2-yl)-2-(methylsulfonyl)ethyl)-6-(2-fluo rophenyl)-7-methyl-1H-imidazo[4,5-b]pyridin-2(3*H*)-one;
(S)-3-(1-(5-(difluoromethoxy)-6-ethoxypyridin-2-yl)-2-(methylsulfonyl)ethyl)-7-methyl -6-phenyl-1H-imidazo[4,5-b]pyridin-2(3H)-one;
(R)-3-(1-(5-(difluoromethoxy)-6-ethoxypyridin-2-yl)-2-(methylsulfonyl)ethyl)-7-methyl -6-phenyl-1H-imidazo[4,5-b]pyridin-2(3H)-one;
(S)-3-(1-(5-(difluoromethoxy)-6-ethoxypyridin-2-yl)-2-(methylsulfonyl)ethyl)-6-(4-fluo rophenyl)-7-methyl-1H-imidazo[4,5-b]pyridin-2(3H)-one;
(S)-3-(l-(5-(difluoromethoxy)-6-ethoxypyridin-2-yl)-2-(methylsulfonyl)ethyl)-7-methyl -6-(*o*-methylphenyl)-1H-imidazo[4,5-b]pyridin-2(3H)-one;
(S)-3-(1-(5-(difluoromethoxy)-6-ethoxypyridin-2-yl)-2-(methylsulfonyl)ethyl)-6-(2-fluo rophenyl)-1,7-dimethyl-1H-imidazo[4,5-b]pyridin-2(3H)-one;
(S)-3-(l-(5-(difluoromethoxy)-6-ethoxypyridin-2-yl)-2-(methylsulfonyl)ethyl)-l,7-dimet hyl-6-phenyl-1H-imidazo[4,5-b]pyridin-2(3H)-one;
(S)-3-(1-(5-(difluoromethoxy)-6-ethoxypyridin-2-yl)-2-(methylsulfonyl)ethyl)-6-(4- fluo rophenyl)-1,7-dimethyl-1H-imidazo[4,5-b]pyridin-2(3H)-one;
(S)-3-(1-(5-(difluoromethoxy)-6-ethoxypyridin-2-yl)-2-(methylsulfonyl)ethyl)-1,7-dimet hyl-6-(*o*-methylphenyl)-1H-imidazo[4,5-b]pyridin-2(3H)-one;
(S)-1-(2,2-difluoroethyl)-3-(1-(6-ethoxy-5-isopropoxypyridin-2-yl)-2-(methylsulfonyl)et hyl)-6-(2-fluorophenyl)-7-methyl-1H-imidazo[4,5-b]pyridin-2(3H)-one;
(S)-1-(2,2-difluoroethyl)-3-(1-(6-ethoxy-5-isopropoxypyridin-2-yl)-2-(methylsulfonyl)et hyl)-7-methyl-6-phenyl-1H-imidazo[4,5-b]pyridin-2(3H)-one;
(S)-1-(2,2-difluoroethyl)-3-(1-(6-ethoxy-5-isopropoxypyridin-2-yl)-2-(methylsulfonyl)et hyl)-6-(4-fluorophenyl)-7-methyl-1H-imidazo[4,5-b]pyridin-2(3H)-one;
(S)-1-(2,2-difluoroethyl)-3-(1-(6-ethoxy-5-isopropoxypyridin-2-yl)-2-(methylsulfonyl)et hyl)-7-methyl-6-(*o*-methylphenyl)-1H-imidazo[4,5-b]pyridin-2(3H)-one;
(S)-3-(1-(6-ethoxy-5-methoxypyridin-2-yl)-2-(methylsulfonyl)ethyl)-6-bromo-7-methyl-1H-imidazo[4,5-b]pyridin-2(3H)-one;
(S)-3-(1-(6-ethoxy-5-methoxypyridin-2-yl)-2-(methylsulfonyl)ethyl)-6-(2-fluorophenyl) -1H-imidazo[4,5-b]pyridin-2(3H)-one;
(S)-3-(1-(6-ethoxy-5-methoxypyridin-2-yl)-2-(methylsulfonyl)ethyl)-6-(2-fluorophenyl) -1-methyl-1H-imidazo[4,5-b]pyridin-2(3H)-one;
(S)-3-(1-(6-ethoxy-5-methoxypyridin-2-yl)-2-(methylsulfonyl)ethyl)-6-phenyl-1H-imida zo[4,5-b]pyridin-2(3H)-one;
(S)-3-(1-(6-ethoxy-5-methoxypyridin-2-yl)-2-(methylsulfonyl)ethyl)-6-phenyl-1-methyl -1H-imidazo[4,5-b]pyridin-2(3H)-one;
(S)-3-(1-(6-ethoxy-5-methoxypyridin-2-yl)-2-(methylsulfonyl)ethyl)-6-(4-fluorophenyl) -1H-imidazo[4,5-b]pyridin-2(3H)-one;
(S)-3-(1-(6-ethoxy-5-methoxypyridin-2-yl)-2-(methylsulfonyl)ethyl)-6-(*o*-methylphenyl )-1H-imidazo[4,5-b]pyridin-2(3H)-one;
(S)-3-(1-(6-ethoxy-5-methoxypyridin-2-yl)-2-(methylsulfonyl)ethyl)-1-methyl-6-(*o*-met hylphenyl)-1H-imidazo[4,5-b]pyridin-2(3H)-one;
(S)-3-(1-(6-ethoxy-5-methoxypyridin-2-yl)-2-(methylsulfonyl)ethyl)-6-(2-ethylphenyl)-1H-imidazo[4,5-b]pyridin-2(3H)-one;
(S)-3-(1-(6-ethoxy-5-methoxypyridin-2-yl)-2-(methylsulfonyl)ethyl)-6-(2-ethylphenyl)-1-methyl-1H-imidazo[4,5-b]pyridin-2(3H)-one;
(S)-3-(1-(6-ethoxy-5-methoxypyridin-2-yl)-2-(methylsulfonyl)ethyl)-6-(2-methoxyphen yl)-1H-imidazo[4,5-b]pyridin-2(3H)-one;
(S)-3-(1-(6-ethoxy-5-methoxypyridin-2-yl)-2-(methylsulfonyl)ethyl)-6-(2-methoxyphen yl)-1-methyl-1H-imidazo[4,5-b]pyridin-2(3H)-one;
(S)-3-(1-(6-ethoxy-5-methoxypyridin-2-yl)-2-(methylsulfonyl)ethyl)-6-(2-(trifluorometh yl)phenyl)-1H-imidazo[4,5-b]pyridin-2(3H)-one;
(S)-3-(1-(6-ethoxy-5-methoxypyridin-2-yl)-2-(methylsulfonyl)ethyl)-6-(2-(trifluorometh yl)phenyl)-1-methyl-1H-imidazo[4,5-b]pyridin-2(3H)-one;
(S)-6-(2-cyclopropylphenyl)-3-(1-(6-ethoxy-5-methoxypyridin-2-yl)-2-(methylsulfonyl) ethyl)-1H-imidazo[4,5-b]pyridin-2(3H)-one;
(S)-6-(2,6-difluorophenyl)-3-(1-(6-ethoxy-5-methoxypyridin-2-yl)-2-(methylsulfonyl)et hyl)-1H-imidazo[4,5-b]pyridin-2(3H)-one;
(S)-6-(2,6-difluorophenyl)-3-(1-(6-ethoxy-5-methoxypyridin-2-yl)-2-(methylsulfonyl)et hyl)-1-methyl-1H-imidazo[4,5-b]pyridin-2(3H)-one;
(S)-6-(2,3-difluorophenyl)-3-(1-(6-ethoxy-5-methoxypyridin-2-yl)-2-(methylsulfonyl)et hyl)-1H-imidazo[4,5-b]pyridin-2(3H)-one;
(S)-6-(2,3-difluorophenyl)-3-(1-(6-ethoxy-5-methoxypyridin-2-yl)-2-(methylsulfonyl)et hyl)-1-methyl-1H-imidazo[4,5-b]pyridin-2(3H)-one;
(S)-6-(2,5-difluorophenyl)-3-(l-(6-ethoxy-5-methoxypyridin-2-yl)-2-(methylsulfonyl)et hyl)-1H-imidazo[4,5-b]pyridin-2(3H)-one;
(S)-6-(2,5-difluorophenyl)-3-(l-(6-ethoxy-5-methoxypyridin-2-yl)-2-(methylsulfonyl)et hyl)-1-methyl-1H-imidazo[4,5-b]pyridin-2(3H)-one;
(S)-6-(2,4-difluorophenyl)-3-(1-(6-ethoxy-5-methoxypyridin-2-yl)-2-(methylsulfonyl)et hyl)-1H-imidazo[4,5-b]pyridin-2(3H)-one;
(S)-6-(2,4-difluorophenyl)-3-(1-(6-ethoxy-5-methoxypyridin-2-yl)-2-(methylsulfonyl)et hyl)-1-methyl-1H-imidazo[4,5-b]pyridin-2(3H)-one;
(S)-6-(2,4,5-trifluorophenyl)-3-(1-(6-ethoxy-5-methoxypyridin-2-yl)-2-(methylsulfonyl) ethyl)-1H-imidazo[4,5-b]pyridin-2(3H)-one;
(S)-6-(2,4,5-trifluorophenyl)-3-(1-(6-ethoxy-5-methoxypyridin-2-yl)-2-(methylsulfonyl) ethyl)-1-methyl-1H-imidazo[4,5-b]pyridin-2(3H)-one;
(R)-6-(2,5-difluorophenyl)-3-(1-(6-ethoxy-5-methoxypyridin-2-yl)-2-(methylsulfonyl)et hyl)-1H-imidazo[4,5-b]pyridin-2(3H)-one;
(S)-6-(2,4-dimethylphenyl)-3-(1-(6-ethoxy-5-methoxypyridin-2-yl)-2-(methylsulfonyl)et hyl)-1H-imidazo[4,5-b]pyridin-2(3H)-one;
(S)-6-(2,4-dimethylphenyl)-3-(1-(6-ethoxy-5-methoxypyridin-2-yl)-2-(methylsulfonyl)et hyl)-1-methyl-1H-imidazo[4,5-b]pyridin-2(3H)-one;
(S)-6-(2,5-dimethylphenyl)-3-(1-(6-ethoxy-5-methoxypyridin-2-yl)-2-(methylsulfonyl)et hyl)-1H-imidazo[4,5-b]pyridin-2(3H)-one;
(S)-6-(2,5-dimethylphenyl)-3-(1-(6-ethoxy-5-methoxypyridin-2-yl)-2-(methylsulfonyl)et hyl)-1-methyl-1H-imidazo[4,5-b]pyridin-2(3H)-one;
(S)-6-(2,3-dimethylphenyl)-3-(l-(6-ethoxy-5-methoxypyridin-2-yl)-2-(methylsulfonyl)et hyl)-1H-imidazo[4,5-b]pyridin-2(3H)-one;
(S)-6-(2,3-dimethylphenyl)-3-(l-(6-ethoxy-5-methoxypyridin-2-yl)-2-(methylsulfonyl)et hyl)-1-methyl-1H-imidazo[4,5-b]pyridin-2(3H)-one;
(S)-6-(2,6-dimethylphenyl)-3-(1-(6-ethoxy-5-methoxypyridin-2-yl)-2-(methylsulfonyl)et hyl)-1H-imidazo[4,5-b]pyridin-2(3H)-one;
(S)-6-(2,6-dimethylphenyl)-3-(1-(6-ethoxy-5-methoxypyridin-2-yl)-2-(methylsulfonyl)et hyl)-1-methyl-1H-imidazo[4,5-b]pyridin-2(3H)-one;
(S)-6-(4-fluoro-2-methylphenyl)-3-(1-(6-ethoxy-5-methoxypyridin-2-yl)-2-(methylsulfo nyl)ethyl)-1H-imidazo[4,5-b]pyridin-2(3H)-one;
(S)-6-(4-fluoro-2-methylphenyl)-3-(1-(6-ethoxy-5-methoxypyridin-2-yl)-2-(methylsulfo nyl)ethyl)-1-methyl-1H-imidazo[4,5-b]pyridin-2(3H)-one;
(S)-6-(5-fluoro-2-methylphenyl)-3-(1-(6-ethoxy-5-methoxypyridin-2-yl)-2-(methylsulfo nyl)ethyl)-1H-imidazo[4,5-b]pyridin-2(3H)-one;
(S)-6-(5-fluoro-2-methylphenyl)-3-(1-(6-ethoxy-5-methoxypyridin-2-yl)-2-(methylsulfo nyl)ethyl)-1-methyl-1H-imidazo[4,5-b]pyridin-2(3H)-one;
(S)-6-(3-fluoro-2-methylphenyl)-3-(1-(6-ethoxy-5-methoxypyridin-2-yl)-2-(methylsulfo nyl)ethyl)-1H-imidazo[4,5-b]pyridin-2(3H)-one;
(S)-6-(3-fluoro-2-methylphenyl)-3-(1-(6-ethoxy-5-methoxypyridin-2-yl)-2-(methylsulfo nyl)ethyl)-1-methyl-1H-imidazo[4,5-b]pyridin-2(3H)-one;
(S)-6-(2-fluoro-5-methylphenyl)-3-(1-(6-ethoxy-5-methoxypyridin-2-yl)-2-(methylsulfo nyl)ethyl)-1H-imidazo[4,5-b]pyridin-2(3H)-one;
(S)-6-(2-fluoro-5-methylphenyl)-3-(1-(6-ethoxy-5-methoxypyridin-2-yl)-2-(methylsulfo nyl)ethyl)-1-methyl-1H-imidazo[4,5-b]pyridin-2(3H)-one;
(S)-6-(2-fluoro-4-methylphenyl)-3-(1-(6-ethoxy-5-methoxypyridin-2-yl)-2-(methylsulfo nyl)ethyl)-1H-imidazo[4,5-b]pyridin-2(3H)-one;
(S)-6-(2-fluoro-4-methylphenyl)-3-(1-(6-ethoxy-5-methoxypyridin-2-yl)-2-(methylsulfo nyl)ethyl)-1-methyl-1H-imidazo[4,5-b]pyridin-2(3H)-one;
(S)-6-(5-fluoro-2-methoxyphenyl)-3-(l-(6-ethoxy-5-methoxypyridin-2-yl)-2-(methylsulf onyl)ethyl)-1H-imidazo[4,5-b]pyridin-2(3H)-one;
(S)-6-(5-fluoro-2-methoxyphenyl)-3-(1-(6-ethoxy-5-methoxypyridin-2-yl)-2-(methylsulf onyl)ethyl)-1-methyl-1H-imidazo[4,5-b]pyridin-2(3H)-one;
(S)-6-(4-fluoro-2-methoxyphenyl)-3-(1-(6-ethoxy-5-methoxypyridin-2-yl)-2-(methylsulf onyl)ethyl)-1H-imidazo[4,5-b]pyridin-2(3H)-one;
(S)-6-(4-fluoro-2-methoxyphenyl)-3-(1-(6-ethoxy-5-methoxypyridin-2-yl)-2-(methylsulf onyl)ethyl)-1-methyl-1H-imidazo[4,5-b]pyridin-2(3H)-one;
(S)-6-(6-fluoro-2-methoxyphenyl)-3-(1-(6-ethoxy-5-methoxypyridin-2-yl)-2-(methylsulf onyl)ethyl)-1H-imidazo[4,5-b]pyridin-2(3H)-one;
(S)-6-(pyridin-2-yl)-3-(1-(6-ethoxy-5-methoxypyridin-2-yl)-2-(methylsulfonyl)ethyl)-1 H-imidazo[4,5-b]pyridin-2(3H)-one;
(S)-3-(1-(6-ethoxy-5-methoxypyridin-2-yl)-2-(methylsulfonyl)ethyl)-1-methyl-6-(pyridi n-2-yl)-1H-imidazo[4,5-b]pyridin-2(3H)-one;
(S)-3-(1-(6-ethoxy-5-methoxypyridin-2-yl)-2-(methylsulfonyl)ethyl)-1-ethyl-6-(pyridin-2-yl)-1H-imidazo[4,5-b]pyridin-2(3H)-one;
(S)-3-(1-(6-ethoxy-5-methoxypyridin-2-yl)-2-(methylsulfonyl)ethyl)-1-(2-methoxyethyl )-6-(pyridin-2-yl)-1H-imidazo[4,5-b]pyridin-2(3H)-one;
(S)-3-(1-(6-ethoxy-5-methoxypyridin-2-yl)-2-(methylsulfonyl)ethyl)-6-(2-fluorophenyl) -1-methyl-1H-imidazo[4,5-b]pyridin-2(3H)-one;
(S)-3-(1-(6-ethoxy-5-methoxypyridin-2-yl)-2-(methylsulfonyl)ethyl)-1-methyl-6-phenyl -1H-imidazo[4,5-b]pyridin-2(3H)-one;
(S)-3-(1-(6-ethoxy-5-methoxypyridin-2-yl)-2-(methylsulfonyl)ethyl)-6-(4-fluorophenyl) -1-methyl-1H-imidazo[4,5-b]pyridin-2(3H)-one;
(S)-5-bromo-1-(1-(6-ethoxy-5-methoxypyridin-2-yl)-2-(methylsulfonyl)ethyl)-1H-benzo [d]imidazol-2(3H)-one;
(S)-1-(1-(1-(6-ethoxy-5-methoxypyridin-2-yl)-2-(methylsulfonyl)ethyl)-5-phenyl-1H-be nzo[d]imidazol-2(3H)-one;
(S)-1-(1-(1-(6-ethoxy-5-methoxypyridin-2-yl)-2-(methylsulfonyl)ethyl)-5-phenyl-3-met hyl-1H-benzo[d]imidazol-2(3H)-one;
(S)-1-(1-(1-(6-ethoxy-5-methoxypyridin-2-yl)-2-(methylsulfonyl)ethyl)-5-(2-fluorophen yl)-1H-benzo[d]imidazol-2(3H)-one;
(S)-1-(1-(1-(6-ethoxy-5-methoxypyridin-2-yl)-2-(methylsulfonyl)ethyl)-5-(2-fluorophen yl)-3 -methyl-1H-benzo[d]imidazol-2(3H)-one;
(R)-1-(1-(1-(6-ethoxy-5-methoxypyridin-2-yl)-2-(methylsulfonyl)ethyl)-5-(2-fluorophen yl)-1H-benzo[d]imidazol-2(3H)-one;
(S)-1-(1-(1-(6-ethoxy-5-methoxypyridin-2-yl)-2-(methylsulfonyl)ethyl)-5-(4-fluorophen yl)-1H-benzo[d]imidazol-2(3H)-one;
(R)-1-(1-(1-(6-ethoxy-5-methoxypyridin-2-yl)-2-(methylsulfonyl)ethyl)-5-(4-fluorophen yl)-1H-benzo[d]imidazol-2(3H)-one;
(S)-1-(1-(1-(6-ethoxy-5-methoxypyridin-2-yl)-2-(methylsulfonyl)ethyl)-3-methyl-5-(4-fl uorophenyl)-1H-benzo[d]imidazol-2(3H)-one;
(R)-1-(1-(1-(6-ethoxy-5-methoxypyridin-2-yl)-2-(methylsulfonyl)ethyl)-3-methyl-5-(4-f luorophenyl)-1H-benzo[d]imidazol-2(3H)-one;
(S)-1-(1-(6-ethoxy-5-methoxypyridin-2-yl)-2-(methylsulfonyl)ethyl)-5-(o-methylphenyl )-1H-benzo[d]imidazol-2(3H)-one;
(S)-1-(1-(6-ethoxy-5-methoxypyridin-2-yl)-2-(methylsulfonyl)ethyl)-5-(o-methylphenyl )-3-methyl-1H-benzo[d]imidazol-2(3H)-one;
(R)-1-(1-(6-ethoxy-5-methoxypyridin-2-yl)-2-(methylsulfonyl)ethyl)-5-(o-methylphenyl )-1H-benzo[d]imidazol-2(3H)-one;
(S)-5-(2-chlorophenyl)-1-(1-(6-ethoxy-5-methoxypyridin-2-yl)-2-(methylsulfonyl)ethyl) -1H-benzo[d]imidazol-2(3H)-one;
(S)-1-(1-(1-(6-ethoxy-5-methoxypyridin-2-yl)-2-(methylsulfonyl)ethyl)-5-(2-methoxyph enyl)-1H-benzo[d]imidazol-2(3H)-one;
(S)-1-(1-(1-(6-ethoxy-5-methoxypyridin-2-yl)-2-(methylsulfonyl)ethyl)-5-(2-methoxyph enyl)-3 -methyl-1H-benzo[d]imidazol-2(3H)-one;
(R)-1-(1-(1-(6-ethoxy-5-methoxypyridin-2-yl)-2-(methylsulfonyl)ethyl)-5-(2-methoxyph enyl)-1H-benzo[d]imidazol-2(3H)-one;
(S)-1-(1-(1-(6-ethoxy-5-methoxypyridin-2-yl)-2-(methylsulfonyl)ethyl)-5-(2-(trifluorom ethyl)phenyl)-1H-benzo[d]imidazol-2(3H)-one;
(S)-1-(1-(1-(6-ethoxy-5-methoxypyridin-2-yl)-2-(methylsulfonyl)ethyl)-5-(2-(trifluorom ethyl)phenyl)-3-methyl-1H-benzo[d]imidazol-2(3H)-one;
(S)-5-(2-cyclopropylphenyl)-1-(1-(6-ethoxy-5-methoxypyridin-2-yl)-2-(methylsulfonyl) ethyl)-1H-benzo[d]imidazol-2(3H)-one;
(S)-1-(1-(1-(6-ethoxy-5-methoxypyridin-2-yl)-2-(methylsulfonyl)ethyl)-5-(2-isopropylp henyl)-4-methyl-1H-benzo[d]imidazol-2(3H)-one;
(S)-1-(1-(6-ethoxy-5-methoxypyridin-2-yl)-2-(methylsulfonyl)ethyl)-5-(2-ethylphenyl)-1H-benzo[d]imidazol-2(3H)-one;
(S)-1-(1-(6-ethoxy-5-methoxypyridin-2-yl)-2-(methylsulfonyl)ethyl)-5-(2-ethylphenyl)-3 -methyl-1H-benzo[d]imidazol-2(3H)-one;
(S)-5-(2,3-difluorophenyl)-1-(1-(6-ethoxy-5-methoxypyridin-2-yl)-2-(methylsulfonyl)et hyl)-1H-benzo[d]imidazol-2(3H)-one;
(S)-5-(2,3-difluorophenyl)-1-(1-(6-ethoxy-5-methoxypyridin-2-yl)-2-(methylsulfonyl)et hyl)-3 -methyl-1H-benzo[d]imidazol-2(3H)-one;
(S)-5-(2,6-difluorophenyl)-1-(1-(6-ethoxy-5-methoxypyridin-2-yl)-2-(methylsulfonyl)et hyl)-1H-benzo[d]imidazol-2(3H)-one;
(S)-5-(2,6-difluorophenyl)-1-(1-(6-ethoxy-5-methoxypyridin-2-yl)-2-(methylsulfonyl)et hyl)-3 -methyl-1H-benzo[d]imidazol-2(3H)-one;
(S)-5-(2,4-difluorophenyl)-1-(1-(6-ethoxy-5-methoxypyridin-2-yl)-2-(methylsulfonyl)et hyl)-1H-benzo[d]imidazol-2(3H)-one;
(S)-5-(2,4-difluorophenyl)-1-(1-(6-ethoxy-5-methoxypyridin-2-yl)-2-(methylsulfonyl)et hyl)-3 -methyl-1H-benzo[d]imidazol-2(3H)-one;
(S)-5-(2,4,5-trifluorophenyl)-1-(1-(6-ethoxy-5-methoxypyridin-2-yl)-2-(methylsulfonyl) ethyl)-1H-benzo[d]imidazol-2(3H)-one;
(S)-5-(2,4,5-trifluorophenyl)-1-(1-(6-ethoxy-5-methoxypyridin-2-yl)-2-(methylsulfonyl) ethyl)-3-methyl-1H-benzo[d]imidazol-2(3H)-one;
(S)-5-(2,5-difluorophenyl)-1-(1-(6-ethoxy-5-methoxypyridin-2-yl)-2-(methylsulfonyl)et hyl)-1H-benzo[d]imidazol-2(3H)-one;
(S)-5-(2,5-difluorophenyl)-1-(1-(6-ethoxy-5-methoxypyridin-2-yl)-2-(methylsulfonyl)et hyl)-3 -methyl-1H-benzo[d]imidazol-2(3H)-one;
(S)-5-(2,4-dimethylphenyl)-1-(1-(6-ethoxy-5-methoxypyridin-2-yl)-2-(methylsulfonyl)et hyl)-1H-benzo[d]imidazol-2(3H)-one;
(S)-5-(2,4-dimethylphenyl)-1-(1-(6-ethoxy-5-methoxypyridin-2-yl)-2-(methylsulfonyl)et hyl)-3 -methyl-1H-benzo[d]imidazol-2(3H)-one;
(S)-5-(2,5-dimethylphenyl)-1-(1-(6-ethoxy-5-methoxypyridin-2-yl)-2-(methylsulfonyl)et hyl)-1H-benzo[d]imidazol-2(3H)-one;
(S)-5-(2,5-dimethylphenyl)-1-(1-(6-ethoxy-5-methoxypyridin-2-yl)-2-(methylsulfonyl)et hyl)-3 -methyl-1H-benzo[d]imidazol-2(3H)-one;
(S)-5-(2,3-dimethylphenyl)-1-(1-(6-ethoxy-5-methoxypyridin-2-yl)-2-(methylsulfonyl)et hyl)-1H-benzo[d]imidazol-2(3H)-one;
(S)-5-(2,3-dimethylphenyl)-1-(1-(6-ethoxy-5-methoxypyridin-2-yl)-2-(methylsulfonyl)et hyl)-3 -methyl-1H-benzo[d]imidazol-2(3H)-one;
(S)-5-(2,6-dimethylphenyl)-1-(1-(6-ethoxy-5-methoxypyridin-2-yl)-2-(methylsulfonyl)et hyl)-1H-benzo[d]imidazol-2(3H)-one;
(S)-5-(4-fluoro-2-methylphenyl)-1-(1-(6-ethoxy-5-methoxypyridin-2-yl)-2-(methylsulfo nyl)ethyl)-1H-benzo[d]imidazol-2(3H)-one;
(S)-5-(4-fluoro-2-methylphenyl)-1-(1-(6-ethoxy-5-methoxypyridin-2-yl)-2-(methylsulfo nyl)ethyl)-3-methyl-1H-benzo[d]imidazol-2(3H)-one;
(S)-5-(5-fluoro-2-methylphenyl)-1-(1-(6-ethoxy-5-methoxypyridin-2-yl)-2-(methylsulfo nyl)ethyl)-1H-benzo[d]imidazol-2(3H)-one;
(S)-5-(5-fluoro-2-methylphenyl)-1-(1-(6-ethoxy-5-methoxypyridin-2-yl)-2-(methylsulfo nyl)ethyl)-3-methyl-1H-benzo[d]imidazol-2(3H)-one;
(S)-5-(3-fluoro-2-methylphenyl)-1-(1-(6-ethoxy-5-methoxypyridin-2-yl)-2-(methylsulfo nyl)ethyl)-1H-benzo[d]imidazol-2(3H)-one;
(S)-5-(3-fluoro-2-methylphenyl)-1-(1-(6-ethoxy-5-methoxypyridin-2-yl)-2-(methylsulfo nyl)ethyl)-3-methyl-1H-benzo[d]imidazol-2(3H)-one;
(S)-5-(2-fluoro-5-methylphenyl)-1-(1-(6-ethoxy-5-methoxypyridin-2-yl)-2-(methylsulfo nyl)ethyl)-1H-benzo[d]imidazol-2(3H)-one;
(S)-5-(2-fluoro-5-methylphenyl)-1-(1-(6-ethoxy-5-methoxypyridin-2-yl)-2-(methylsulfo nyl)ethyl)-3-methyl-1H-benzo[d]imidazol-2(3H)-one;
(S)-5-(2-fluoro-3-methylphenyl)-1-(1-(6-ethoxy-5-methoxypyridin-2-yl)-2-(methylsulfo nyl)ethyl)-1H-benzo[d]imidazol-2(3H)-one;
(S)-5-(2-fluoro-3-methylphenyl)-1-(1-(6-ethoxy-5-methoxypyridin-2-yl)-2-(methylsulfo nyl)ethyl)-3-methyl-1H-benzo[d]imidazol-2(3H)-one;
(S)-5-(2-fluoro-4-methylphenyl)-1-(1-(6-ethoxy-5-methoxypyridin-2-yl)-2-(methylsulfo nyl)ethyl)-1H-benzo[d]imidazol-2(3H)-one;
(S)-5-(2-fluoro-4-methylphenyl)-1-(1-(6-ethoxy-5-methoxypyridin-2-yl)-2-(methylsulfo nyl)ethyl)-3-methyl-1H-benzo[d]imidazol-2(3H)-one;
(S)-5-(2-fluoro-5-methoxyphenyl)-1-(1-(6-ethoxy-5-methoxypyridin-2-yl)-2-(methylsulf onyl)ethyl)-1H-benzo[d]imidazol-2(3H)-one;
(S)-5-(2-fluoro-5-methoxyphenyl)-1-(1-(6-ethoxy-5-methoxypyridin-2-yl)-2-(methylsulf onyl)ethyl)-3-methyl-1H-benzo[d]imidazol-2(3H)-one;
(S)-5-(2-fluoro-3-methoxyphenyl)-1-(1-(6-ethoxy-5-methoxypyridin-2-yl)-2-(methylsulf onyl)ethyl)-1H-benzo[d]imidazol-2(3H)-one;
(S)-5-(2-fluoro-4-methoxyphenyl)-1-(1-(6-ethoxy-5-methoxypyridin-2-yl)-2-(methylsulf onyl)ethyl)-1H-benzo[d]imidazol-2(3H)-one;
(S)-5-(2-fluoro-4-methoxyphenyl)-1-(1-(6-ethoxy-5-methoxypyridin-2-yl)-2-(methylsulf onyl)ethyl)-3-methyl-1H-benzo[d]imidazol-2(3H)-one;
(S)-5-(5-fluoro-2-methoxyphenyl)-1-(1-(6-ethoxy-5-methoxypyridin-2-yl)-2-(methylsulf onyl)ethyl)-1H-benzo[d]imidazol-2(3H)-one;
(S)-5-(5-fluoro-2-methoxyphenyl)-1-(1-(6-ethoxy-5-methoxypyridin-2-yl)-2-(methylsulf onyl)ethyl)-3-methyl-1H-benzo[d]imidazol-2(3H)-one;
(S)-5-(4-fluoro-2-methoxyphenyl)-1-(1-(6-ethoxy-5-methoxypyridin-2-yl)-2-(methylsulf onyl)ethyl)-1H-benzo[d]imidazol-2(3H)-one;
(S)-5-(4-fluoro-2-methoxyphenyl)-1-(1-(6-ethoxy-5-methoxypyridin-2-yl)-2-(methylsulf onyl)ethyl)-3-methyl-1H-benzo[d]imidazol-2(3H)-one;
(S)-5-(6-fluoro-2-methoxyphenyl)-1-(1-(6-ethoxy-5-methoxypyridin-2-yl)-2-(methylsulf onyl)ethyl)-1H-benzo[d]imidazol-2(3H)-one;
(S)-5-(6-fluoro-2-methoxyphenyl)-1-(1-(6-ethoxy-5-methoxypyridin-2-yl)-2-(methylsulf onyl)ethyl)-3-methyl-1H-benzo[d]imidazol-2(3H)-one;
(S)-1-(1-(1-(6-ethoxy-5-methoxypyridin-2-yl)-2-(methylsulfonyl)ethyl)-5-(2-ethoxyphe nyl)-1H-benzo[d]imidazol-2(3H)-one;
(R)-1-(1-(1-(6-ethoxy-5-methoxypyridin-2-yl)-2-(methylsulfonyl)ethyl)-5-(2-ethoxyphe nyl)-1H-benzo[d]imidazol-2(3H)-one;
(S)-1-(1-(1-(6-ethoxy-5-methoxypyridin-2-yl)-2-(methylsulfonyl)ethyl)-5-(pyridin-2-yl) -1H-benzo[d]imidazol-2(3H)-one;
(R)-1-(1-(1-(6-ethoxy-5-methoxypyridin-2-yl)-2-(methylsulfonyl)ethyl)-5-(pyridin-2-yl) -1H-benzo[d]imidazol-2(3H)-one;
(S)-1-(1-(6-ethoxy-5-methoxypyridin-2-yl)-2-(methylsulfonyl)ethyl)-3-methyl-5-(pyridi n-2-yl)-1H-benzo[d]imidazol-2(3H)-one;
(S)-1-(1-(1-(6-ethoxy-5-methoxypyridin-2-yl)-2-(methylsulfonyl)ethyl)-3-ethyl-5-(pyrid in-2-yl)-1H-benzo[d]imidazol-2(3H)-one;
(S)-1-(1-(6-ethoxy-5-methoxypyridin-2-yl)-2-(methylsulfonyl)ethyl)-3-(2-methoxyethyl )-5-(pyridin-2-yl)-1H-benzo[d]imidazol-2(3H)-one;
(S)-1-(1-(6-ethoxy-5-methoxypyridin-2-yl)-2-(methylsulfonyl)ethyl)-5-(4-fluoro-2-meth ylphenyl)-1H-benzo[d]imidazol-2(3H)-one;
(S)-1-(1-(6-ethoxy-5-methoxypyridin-2-yl)-2-(methylsulfonyl)ethyl)-5-(4-fluoro-2-meth ylphenyl)-3-methyl-1H-benzo[d]imidazol-2(3H)-one;
(S)-1-(1-(6-ethoxy-5-methoxypyridin-2-yl)-2-(methylsulfonyl)ethyl)-5-(2-fluoro-5-meth ylphenyl)-1H-benzo[d]imidazol-2(3H)-one;
(S)-1-(1-(6-ethoxy-5-methoxypyridin-2-yl)-2-(methylsulfonyl)ethyl)-5-(2-fluoro-5-meth ylphenyl)-3-methyl-1H-benzo[d]imidazol-2(3H)-one;
(S)-1-(1-(1-(6-ethoxy-5-methoxypyridin-2-yl)-2-(methylsulfonyl)ethyl)-4-(2-methoxyph enyl)-1H-benzo[d]imidazol-2(3H)-one;
(S)-1-(1-(1-ethoxy-5-methoxypyridin-2-yl)-2-(methylsulfonyl)ethyl)-4-(o-methylphenyl )-1H-benzo[d]imidazol-2(3H)-one;
(S)-1-(1-(1-(6-ethoxy-5-methoxypyridin-2-yl)-2-(methylsulfonyl)ethyl)-4-(2-fluorophen yl)-1H-benzo[d]imidazol-2(3H)-one;
(S)-1-(1-(1-(6-ethoxy-5-methoxypyridin-2-yl)-2-(methylsulfonyl)ethyl)-4-phenyl-1H-be nzo[d]imidazol-2(3H)-one;
(S)-1-(1-(1-(6-ethoxy-5-methoxypyridin-2-yl)-2-(methylsulfonyl)ethyl)-4-(2-fluorobenz yl)-1H-benzo[d]imidazol-2(3H)-one;
(S)-3-cyclopropyl-1-(1-(6-ethoxy-5-methoxypyridin-2-yl)-2-(methylsulfonyl)ethyl)-5-(2 -fluorophenyl)-1H-benzo[d]imidazol-2(3H)-one;
(S)-3-cyclopropyl-1-(1-(6-ethoxy-5-methoxypyridin-2-yl)-2-(methylsulfonyl)ethyl)-5-m ethyl-1H-benzo[d]imidazol-2(3H)-one;
(S)-5-bromo-1-(1-(6-ethoxy-5-methoxypyridin-2-yl)-2-(methylsulfonyl)ethyl)-3-(2-(met hylsulfonyl)ethyl)-1H-benzo[d]imidazol-2(3H)-one;
(S)-3-cyclopropyl-1-(1-(6-ethoxy-5-methoxypyridin-2-yl)-2-(methylsulfonyl)ethyl)-5-(tr ifluoromethyl)-1H-benzo[d]imidazol-2(3H)-one;
(S)-ethyl 1-(1-(1-(6-ethoxy-5-methoxypyridin-2-yl)-2-(methylsulfonyl)ethyl)-2-oxo-2,3 -dihydro-1H-benzo[d]imidazole-5-carboxylate;
(S)-isopropyl 1-(1-(1-(6-ethoxy-5-methoxypyridin-2-yl)-2-(methylsulfonyl)ethyl)-2-oxo -2,3-dihydro-1H-benzo[d]imidazole-5-carboxylate; and
(S)-2,2,2-trifluoroethyl 1-(1-(1-ethoxy-5-methoxypyridin-2-yl)-2-(methylsulfonyl)ethyl) -2-oxo-2,3-dihydro-1H-benzo[d]imidazole-5-carboxylate.

Another purpose of the present disclosure is to provide a compound of formula (II) and an isomer, solvate, deuterated derivative or pharmaceutically acceptable salt thereof: 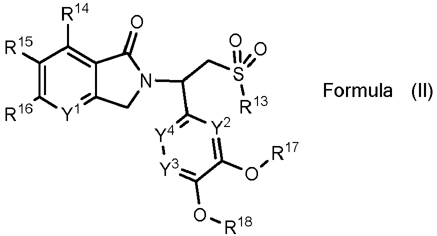 wherein:
Y¹ is selected from N or CH;
one of Y², Y³, and Y⁴ is selected from N, and the others are CH;
R¹³ is selected from C1-C3 alkyl and C1-C6 cycloalkyl, and may be substituted with halogen;
R¹⁴, R¹⁵, and R¹⁶ are independently selected from hydrogen, halogen, hydroxyl, CN, -NO₂, -NH₂, C1-C6 alkyl, C1-C6 alkoxy, C1-C6 alkylamino, C1-C6 alkylamido, C3-C6 cycloalkylamido, C2-C6 alkenyl, C3-C6 cycloalkenyl, 3- to 6-membered heterocycloalkenyl, C3-C6 cycloalkyl, 3- to 6-membered heterocyclyl, phenyl, and 5- to 6-membered heteroaryl, which may be optionally substituted with halogen, C3-C6 cycloalkyl, C1-C8 alkoxy, C3-C8 cycloalkoxy, 3- to 12-membered heterocyclyl, C1-C8 alkylamino, di(C1-C8 alkyl) substituted amino, phenyl, or 5- to 6-membered heteroaryl;
R¹⁷ and R¹⁸ are independently selected from C1-C6 alkyl and C3-C6 cycloalkyl, and may be optionally substituted with deuterium, tritium, or halogen.

Alternatively, the compound of formula (II) and an isomer, solvate, deuterated derivative or pharmaceutically acceptable salt thereof are provided, wherein the compound of formula (II) comprises the following structures:
(S)-*N*-(2-(1-(6-ethoxy-5-methoxypyridin-2-yl)-2-(methylsulfonyl)ethyl)-3-oxoisoindolin -4-yl)acetamide;
(R)-*N*-(2-(1-(6-ethoxy-5-methoxypyridin-2-yl)-2-(methylsulfonyl)ethyl)-3-oxoisoindolin -4-yl)acetamide;
(S)-*N*-(2-(1-(6-ethoxy-5-methoxypyridin-2-yl)-2-(methylsulfonyl)ethyl)-3-oxoisoindolin -4-yl)cyclopropanecarboxamide;
(R)-*N*-(2-(1-(6-ethoxy-5-methoxypyridin-2-yl)-2-(methylsulfonyl)ethyl)-3-oxoisoindolin -4-yl)cyclopropanecarboxamide;
(S)-*N*-(2-(1-(6-ethoxy-5-methoxypyridin-2-yl)-2-(methylsulfonyl)ethyl)-3-oxoisoindolin -4-yl)propionamide; or
(R)-*N*-(2-(1-(6-ethoxy-5-methoxypyridin-2-yl)-2-(methylsulfonyl)ethyl)-3-oxoisoindolin -4-yl)propionamide.

Still another purpose of the present disclosure is to provide a compound of formula (III) and an isomer, solvate, deuterated derivative or pharmaceutically acceptable salt thereof: 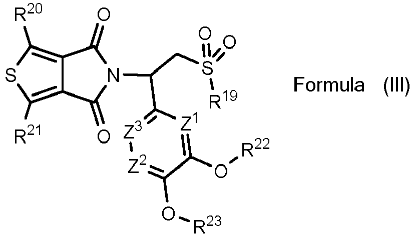 wherein:
R¹⁹ is selected from C₁-C₃ alkyl and C₃-C₆ cycloalkyl, and may be substituted with halogen;
R²⁰ and R²¹ are each selected from hydrogen, halogen, C1-C8 alkyl, C1-C8 alkoxy, hydroxyl, cyano, nitro, -NHC(O)R²⁶, -NHSO₂R²⁶, or -NR²⁴R²⁵;
R²² and R²³ are independently selected from C1-C6 alkyl, and may be optionally substituted with deuterium, tritium, or halogen;
R²⁴ and R²⁵ are each selected from hydrogen, C1-C8 alkyl, C6-C12 aryl, C(O)R²⁶, and SO₂R²⁶; alternatively, R²⁴ and R²⁵ are taken together with the N to which they are attached to form a 4-to 8-membered ring or -CH₂CH₂ZCH₂CH₂-;
Z is selected from O, S, and NR²⁶;
R²⁶ is hydrogen, C1-C6 alkyl, C3-C6 cycloalkyl, or C1-C8 alkylamino, and may be optionally substituted with halogen, C1-C8 alkoxy, C3-C8 cycloalkoxy, 3- to 12-membered heterocyclyl, C1-C8 alkylamino, or di(C1-C8 alkyl) substituted amino.

Alternatively, the compound of formula (III) and an isomer, solvate, deuterated derivative or pharmaceutically acceptable salt thereof are provided, wherein the compound of formula (III) comprises, but is not limited to, the following structures:
5-(1-(6-ethoxy-5-methoxypyridin-2-yl)-2-(methylsulfonyl)ethyl)-1-nitro-4H-thieno[3,4-c]pyrrol-4,6 (5H)-dione;
(S)-5-(1-(6-ethoxy-5-methoxypyridin-2-yl)-2-(methylsulfonyl)ethyl)-1-nitro-4H-thieno[ 3,4-c]pyrrol-4,6 (5H)-dione;
(S)-1-amino-5-(1-(6-ethoxy-5-methoxypyridin-2-yl)-2-(methylsulfonyl)ethyl)-4H-thieno [3,4-c]pyrrol-4,6 (5H)-dione;
N-(5-(1-(6-ethoxy-5-methoxypyridin-2-yl)-2-(methylsulfonyl)ethyl)-4,6-dioxo-5,6-dihy dro-4H-thieno[3,4-c]pyrrol-1-yl)acetamide;
(S)-N-(5-(1-(6-ethoxy-5-methoxypyridin-2-yl)-2-(methylsulfonyl)ethyl)-4,6-dioxo-5,6-d ihydro-4H-thieno[3,4-c]pyrrol-1-yl)acetamide;
(R)-*N*-(5-(1-(6-ethoxy-5-methoxypyridin-2-yl)-2-(methylsulfonyl)ethyl)-4,6-dioxo-5,6-d ihydro-4H-thieno[3,4-c]pyrrol-1-yl)acetamide;
(S)-N-(5-(1-(6-ethoxy-5-methoxypyridin-2-yl)-2-(methylsulfonyl)ethyl)-4,6-dioxo-5,6-d ihydro-4H-thieno[3,4-c]pyrrol-1-yl)propionamide;
(S)-N-(5-(1-(6-ethoxy-5-methoxypyridin-2-yl)-2-(methylsulfonyl)ethyl)-4,6-dioxo-5,6-d ihydro-4H-thieno[3,4-c]pyrrol-l-yl)cyclopropanecarboxamide;
(S)-2-(dimethylamino)-N-(5-(1-(6-ethoxy-5-methoxypyridin-2-yl)-2-(methylsulfonyl)et hyl)-4,6-dioxo-5,6-dihydro-4H-thieno[3,4-c]pyrrol-1-yl)acetamide;
(S)-2-(diethylamino)-N-(5-(1-(6-ethoxy-5-methoxypyridin-2-yl)-2-(methylsulfonyl)ethy l)-4,6-dioxo-5,6-dihydro-4H-thieno[3,4-c]pyrrol-1-yl)acetamide;
(S)-N-(5-(1-(6-ethoxy-5-methoxypyridin-2-yl)-2-(methylsulfonyl)ethyl)-4,6-dioxo-5,6-d ihydro-4H-thieno[3,4-c]pyrrol-1-yl)-2-(piperidin-1-yl)acetamide;
(S)-N-(5-(1-(6-ethoxy-5-methoxypyridin-2-yl)-2-(methylsulfonyl)ethyl)-4,6-dioxo-5,6-d ihydro-4H-thieno[3,4-c]pyrrol-1-yl)-2-(pyrrolidin-1-yl)acetamide; or
(S)-N-(5-(1-(6-ethoxy-5-methoxypyridin-2-yl)-2-(methylsulfonyl)ethyl)-4,6-dioxo-5,6-d ihydro-4H-thieno[3,4-c]pyrrol-1-yl)-2-morpholinoacetamide.

### Terminology

The term "alkyl" refers to a straight or branched alkyl group having from 1 to 12 carbon atoms in the chain, and examples of the alkyl group include methyl (Me), ethyl (Et), n-propyl, isopropyl, butyl, isobutyl, sec-butyl, tert-butyl (t-Bu), pentyl, isopentyl, tert-pentyl, hexyl, isohexyl, and any group which is considered to be equivalent to the above examples according to those of ordinary skill in the art and the teachings provided herein.

The term "alkoxy" refers to an alkyl group as defined above which is bonded to an oxygen atom. The alkoxy group is attached to the parent structure via the oxygen atom.

The term "alkenyl" refers to an alkyl group as defined above consisting of at least two carbon atoms and at least one carbon-carbon double bond, such as vinyl, 1-propenyl, 2-propenyl, etc. It is preferably C₂₋₁₀ alkenyl, more preferably C₂₋₆ alkenyl, most preferably C₂₋₄ alkenyl, and optimally vinyl. Alkenyl may be substituted or non-substituted. When alkenyl is substituted, the substituent is preferably one or more of groups independently selected from alkyl, alkoxy, alkylamino, halogen, hydroxy, cycloalkyl, heterocycloalkyl, and heterocycloalkoxy.

The term "amino" refers to a-NH₂ group or a mono-or di-alkylamino group.

The term cycloalkyl refers to a saturated and partially saturated, monocyclic, fused polycyclic, bridged polycyclic, or spiro polycyclic carbocyclic ring having from 3 to 12 ring atoms per ring. Illustrative examples of cycloalkyl groups include the following moieties in suitable bonding form:

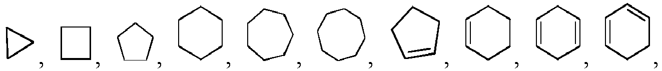

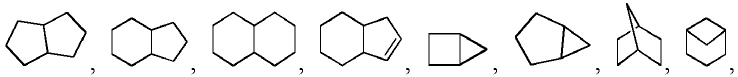

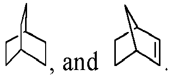

The term "heteroaryl" refers to monocyclic, fused bicyclic, or fused polycyclic aromatic heterocyclic ring (the ring structures having ring atoms selected from carbon atoms and up to four heteroatoms selected from nitrogen, oxygen, and sulfur), and each heterocyclic ring having from 3 to 12 ring atoms. Illustrative examples of heteroaryl groups include the following moieties in suitable bonding form:

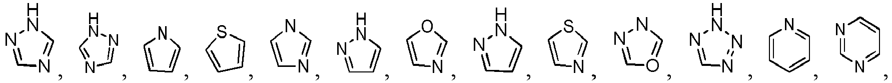

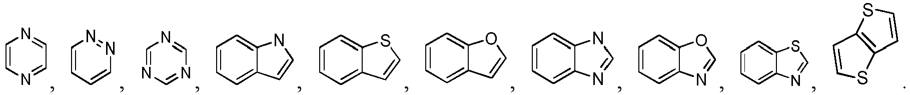

The term "aryl" refers to monocyclic, fused bicyclic, or fused polycyclic aromatic rings containing C5-C20, which is free of heteroatoms such as nitrogen, oxygen, and sulfur. Typical aromatic groups include, but are not limited to, residues derived from benzene, and substituted benzene, naphthalene, anthracene, biphenyl, etc.

The term "heterocyclyl" refers to a saturated or partially unsaturated, monocyclic or polycyclic, cyclic hydrocarbon group comprising from 3 to 20 ring atoms wherein one or more ring atoms are selected from heteroatoms nitrogen, oxygen or S(O)m (where m is an integer from 0 to 2), and the remaining ring atoms are carbon. Preferably, 3 to 12 ring atoms are included, of which 1 to 4 are heteroatoms. More preferably, a heterocycloalkyl ring contains from 3 to 10 ring atoms, and more preferably, a heterocycloalkyl ring contains from 5 to 6 ring atoms. Non-limiting examples of monocyclic heterocycloalkyl groups include pyrrolidinyl, piperidyl, morpholinyl, tetrahydrofuranyl and the like. Polycyclic heterocycloalkyl groups include spiro, fused, and bridged heterocycloalkyl groups. The heterocyclic ring may be substituted or unsubstituted. When the heterocyclic ring is substituted, the substituent is preferably one or more of groups independently selected from alkyl, haloalkyl, alkoxy, alkylamino, halogen, hydroxyl, amino, oxo, alkylamino, cycloalkyl, heterocycloalkyl, heterocycloalkoxy, hydroxyalkyl, carboxyl or carboxylic acid ester group.

The term "halogen" means chlorine, fluorine, bromine or iodine. The term "halo" means chloro, fluoro, bromo or iodo. The term "haloalkyl" refers to an alkyl group as defined above which is substituted with one or more halogen atoms.

"Pharmaceutically acceptable carrier, diluent or excipient" includes but not limited to any adjuvant, carrier, excipient, glidant, sweetening agent, diluent, preservative, dye/colorant, flavor enhancer, surfactant, wetting agent, dispersing agent, suspending agent, stabilizer, isosmotic agent, solvent, or emulsifier, etc, which has been approved by the United States Food and Drug Administration as being acceptable for use in humans or animals and has no side effects on preparing a pharmaceutical composition.

"Pharmaceutically acceptable salts" include both "pharmaceutically acceptable acid addition salts" and "pharmaceutically acceptable base addition salts".

"Pharmaceutically acceptable acid addition salt" refers to those salts which retain the biological effectiveness and properties of the free base, which are not biologically or otherwise undesirable, and which are formed with inorganic acids such as hydrochloric acid, hydrobromic acid, sulfuric acid, nitric acid, phosphoric acid and the like, and organic acids such as acetic acid, 2,2-dichloroacetic acid, adipic acid, alginic acid, ascorbic acid, aspartic acid, benzenesulfonic acid, benzoic acid, 4-acetamidobenzoic acid, camphanic acid, camphor-10-sulfonic acid, capric acid, caproic acid, caprylic acid, carbonic acid, cinnamic acid, citric acid, cyclamic acid, dodecylsulfuric acid, ethane-1,2-disulfonic acid, ethanesulfonic acid, 2-hydroxyethanesulfonic acid, formic acid, fumaric acid, galactaric acid, gentisic acid, glucoheptonic acid, gluconic acid, glucuronic acid, glutamic acid, glutaric acid, 2-oxo-glutaric acid, glycerophosphoric acid, glycolic acid, hippuric acid, isobutyric acid, lactic acid, lactobionic acid, lauric acid, maleic acid, malic acid, malonic acid, mandelic acid, methanesulfonic acid, mucic acid, naphthalene-1,5-disulfonic acid, naphthalene-2-sulfonic acid, 1-hydroxy-2-naphthoic acid, nicotinic acid, oleinic acid, orotic acid, oxalic acid, palmitic acid, pamoic acid, propionic acid, pyroglutamic acid, pyruvic acid, salicylic acid, 4-aminosalicylic acid, sebacic acid, stearic acid, succinic acid, tartaric acid, thiocyanic acid, p-toluenesulfonic acid, trifluoroacetic acid, undecylenic acid and the like.

"Pharmaceutically acceptable base addition salt" refers to those salts which retain the biological effectiveness and properties of the free acid, which are not biologically or otherwise undesirable. These salts are prepared from addition of an inorganic or an organic base to the free acid. Salts derived from an inorganic base include, but are not limited to sodium, potassium, lithium, ammonium, calcium, magnesium, iron, zinc, copper, manganese, aluminum salts and the like. Preferred inorganic salts are the ammonium, sodium, potassium, calcium, and magnesium salts. Salts derived from an organic base include, but are not limited to salts of primary, secondary and tertiary amines, substituted amines including naturally occurring substituted amines, cyclic amines and basic ion exchange resins, such as ammonia, isopropylamine, trimethylamine, diethylamine, triethylamine, tripropylamine, diethanolamine, ethanolamine, dimethylaminoethanol, 2-dimethylaminoethanol, 2-diethylaminoethanol, dicyclohexylamine, lysine, arginine, histidine, caffeine, procaine, hydrabamine, choline, betaine, benzylamine, benzathine, ethylenediamine, glucosamine, methylglucosamine, theobromine, triethanolamine, trometamol, purine, piperazine, piperidine, N-ethyl piperidine, polyamine resins and the like. Particularly preferred organic bases are isopropylamine, diethylamine, ethanolamine, trimethylamine, dicyclohexylamine, choline and caffeine.

"Pharmaceutical composition" refers to a formulation of a compound of the present disclosure and a medium generally acceptable in the art for the delivery of the biologically active compound to mammals, e.g., humans. Such a medium includes all pharmaceutically acceptable carriers, diluents or excipients.

In the second aspect, the present disclosure provides methods for the preparation of compounds of formula (I), formula (II) or formula (III), and isomers and deuterated derivatives thereof. For details, see the examples.

In the embodiments provided herein, if the compound of the present disclosure contains a basic group, it can form a salt with an acid. Salts of acyclic nucleoside derivatives and isomers can be prepared by methods well known to those skilled in the art.

Common acid salts include organic acid salts, inorganic acid salts, and the like. In general, the commonly used organic acid salts are citrate, fumarate, oxalate, malate, lactate, sulfonate (e.g., camphor sulfonate, p-toluenesulfonate, methanesulfonate), etc.; inorganic acid salts include hydrohalides, sulfates, phosphates, nitrates, and the like. For example, a lower alkylsulfonic acid such as methanesulfonic acid and trifluoromethanesulfonic acid may form mesylate salt and triflate salt; and arylsulfonic acid such as benzenesulfonic acid and p-toluenesulfonic acid may form benzenesulfonate and p-toluenesulfonate; an organic carboxylic acid such as acetic acid, fumaric acid, tartaric acid, oxalic acid, maleic acid, malic acid, succinic acid, and citric acid may form corresponding salts; an amino acid such as glutamic acid and aspartic acid may form glutamate and aspartate. An inorganic acid such as hydrohalic acids (for example, hydrofluoric acid, hydrobromic acid, hydroiodic acid, hydrochloric acid), nitric acid, carbonic acid, sulfuric acid and phosphoric acid may also form corresponding salts.

In the third aspect, the present disclosure provides a medicament comprising a compound of formula (I), formula (II) or formula (III), or an isomer, solvate, deuterated derivative or pharmaceutically acceptable salt thereof as an active ingredient. The above medicament may further comprise one or more pharmaceutically acceptable carriers, including conventional diluents, excipients, fillers, binders, wetting agents, disintegrants, absorption promoters, surfactants, adsorption carriers, lubricants, etc. in the pharmaceutical field. If necessary, flavoring agents, sweeteners or the like may be added. The medicament disclosed herein can be formulated into various forms such as tablets, powders, granules, capsules, oral liquids and injectable preparations, and the medicament in the above various dosage forms can be prepared according to a conventional method in the pharmaceutical field.

In a fourth aspect, the present disclosure provides a method of inhibiting PDE4 by a compound of formula (I), formula (II) or formula (III), or an isomer, solvate, deuterated derivative or pharmaceutically acceptable salt thereof, comprising administering to a subject in need a therapeutically effective amount of at least one compound of the present disclosure.

A preferred embodiment is the use of the medicament for the treatment of inflammatory and autoimmune diseases. For this purpose of the disclosure, the inflammatory and autoimmune diseases or conditions include systemic lupus erythematosus (SLE), lupus nephritis, cutaneous lupus, Crohn's Disease, ulcerative colitis, type 1 diabetes, psoriasis, rheumatoid arthritis, systemic-onset juvenile idiopathic arthritis, ankylosing spondylitis, multiple sclerosis, and so on.

### BACKGROUND OF THE INVENTION

Tumor necrosis factor alpha (TNF-α) is a cytokine released primarily by mononuclear phagocytes in response to immunostimulators. TNF-α is able to facilitate most cellular processes of differentiation, recruitment, proliferation, and protein degradation etc.. At low levels, TNF-α has a protective effect against infective agents, tumors, and tissue damage. However, excessive release of TNF-α may cause diseases, as it may cause or exacerbate inflammation, fever, cardiovascular effects, hemorrhage, coagulation, and acute responses similar to acute infection and shock states when administered to mammals or humans. Animals or humans with excessive or uncontrolled production of TNFα in the body chronically suffer from the following diseases: endotoxemia and/or toxic shock syndrome, cachexia, adult respiratory distress syndrome, cancers (such as solid tumors and hematological tumors), heart disease (such as congestive heart failure), viral infection, and genetic, inflammatory, allergic, or autoimmune diseases.

Cancer is a disease with particular destructiveness, and an increased level of TNFα in the blood indicates the risk of having cancer or cancer spreading. Normally, tumor cell cannot survive in the circulatory system in healthy subjects, one of the reasons is that the inner wall of blood vessels acts as a barrier to tumor-cell extravasation. ELAM-1 on endothelial cells has been shown to mediate the promotion of colon cancer cell adhesion to endothelium treated with cytokines.

Cyclic adenosine monophosphate (cAMP) functions in many diseases and disorders. Increased cAMP concentration in leukocytes during inflammation suppresses the activation of leukocytes, and then inflammatory regulatory factors including TNFα and NF-κB are released. The increased cAMP level also leads to relaxation of respiratory smooth muscles.

The main cellular mechanism of cAMP inactivation is the breakdown of cAMP by a family of isoenzymes referred to as cyclic nucleotide phosphodiesterases (PDE). There are eleven known members in the family of PDEs. To date, it has been confirmed that the inhibition of PDE4 enzyme is particularly effective both in inhibiting the release of inflammatory mediators and in relaxing respiratory smooth muscles. Therefore, PDE4 enzyme has become one of the popular drug targets. The family of PDE-4 can be divided into four subtypes (PDE-4A, B, C, D) based on different genetic codes, wherein PDE-4A, PDE-4B and PDE-4D are more strongly expressed in inflammatory cells (such as B cells, T cells and neutrophils) than PDE-4C. Inhibition of PDE4 enzyme leads to an increased cAMP level, thereby regulating the TNFα level for the purpose of disease treatment.

Inhibition of PDE4 enzyme leads to an increased cAMP level, thereby regulating the TNFα level for the treatment of inflammation, such as septic shock, ichorrhemia, endotoxin shock, hemic shock, sepsis syndrome, local ischemia reperfusion injury, mycobacterium malaria infection, meningitis, psoriasis, congestive heart failure, fibrotic disease, cachexia, graft rejection, tumor, autoimmune disorder, AIDS opportunistic infection, rheumatic arthritis, rheumatoid spondylitis, osteoarthritis, other inflammatory disease, Crohn's disease, ulcerative colitis, multiple sclerosis, systemic lupus erythematosus, atopic dermatitis, erythema nodosum leprosum, radiation damage, and hyperoxic lung injury, infective diseases, immune diseases or other malignant diseases.

### DETAILED DESCRIPTION OF THE INVENTION

The practicability of the present invention will be illustrated by the following examples, and those skilled in the art should understand that, according to the teaching of the prior art, modification or replacement of the corresponding technical features still belongs to the protection scope of the present invention.

### Example 1. 3-(1-(6-ethoxy-5-methoxypyridin-2-yl)-2-(methylsulfonyl)ethyl)-6-(2-fluorophenyl)-7-methyl-1H-imidazo[4,5-b]pyridin-2(3H)-one

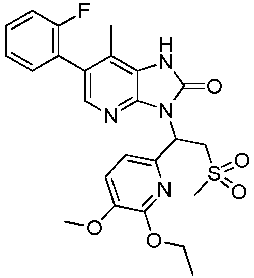

### Step 1: 5-bromo-N-(1-(6-ethoxy-5-methoxypyridin-2-yl)-2-(methylsulfonyl)ethyl)-4-methyl-3 -nitropyridin-2-amine

30.0 g of 5-bromo-2-chloro-4-methyl-3-nitropyridine, 29.4 g of 1-(6-ethoxy-5-methoxypyridin-2-yl)-2-(methylsulfonyl)ethylamine (synthesized by reference to the method disclosed in WO2018157779), and 30 g of diisopropylamine were dissolved in 150.0 mL of N,N-dimethylformamide at room temperature under nitrogen. The reaction was carried out at 120 °C for 2 hours. 1000 mL of water was added, and the mixture was extracted with ethyl acetate (250 mL x 3). The organic phases were combined, washed with saturated saline (100 mL×3), dried over anhydrous sodium sulfate, and filtered. The filtrate was concentrated under reduced pressure, and the residue was subejected to column chromatography to obtain 36.8 g of the target compound.

ESI MS m/z: 489.04[M+1]⁺

### Step 2: N-(1-(1-ethoxy-5-methoxypyridin-2-yl)-2-(methylsulfonyl)ethyl)-5-(2-fluor ophenyl)-4-methyl-3-nitropyridin-2-amine

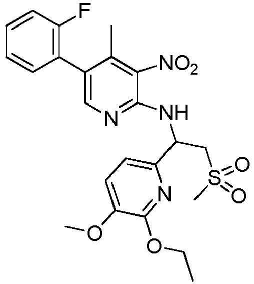

24.1 g of product of step 1 and 10.40 g of o-fluorophenylboronic acid were dissolved in 300 mL of dioxane and 100 mL of water at room temperature, and 20 g of potassium carbonate and 4 g of [1,1'-bis(diphenylphosphino)ferrocene]palladium dichloride dichloromethane complex were added under nitrogen protection. The reaction mixture was heated to 80 °C and stirred for 14 h under nitrogen protection. After the reaction was completed, the reaction mixture was cooled to room temperature, and 600 mL of water was added. The mixture was extracted with ethyl acetate (300 mL x 3). The organic phases were combined, dried over anhydrous sodium sulfate, and filtered to remove the desiccant. The filtrate was concentrated under reduced pressure, and the residue was subjected to column chromatography to obtain 16.4 g of the target compound.

ESI MS m/z: 505.15[M+1]⁺

### Step 3: N2-(1-(6-ethoxy-5-methoxypyridin-2-yl)-2-(methylsulfonyl)ethyl)-5-(2-flu orophenyl)-4-methylpyridin-2,3-diamine

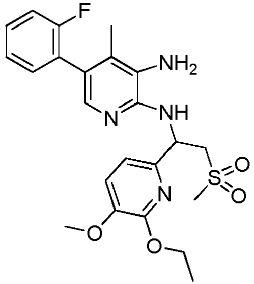

To a mixture of 12.0 g of product of Step 2 and 13 g of ammonium chloride was added ethanol (200 mL) at room temperature. 15 g of zinc powder was added in 20 batches at 0 °C. The reaction mixture was stirred at 0 °C for 16 hours. After the reaction was completed, the reaction mixture was filtered to remove the zinc powder, and the filtrate was concentrated under reduced pressure to obtain the residue. The residue was dissolved in 300 mL of dichloromethane, and the suspension was filtered to remove the insoluble matter. The filtrate was concentrated under reduced pressure to obtain 8.7 g of the compound.

ESI MS m/z: 475.17[M+1]⁺

### Step 4: 3-(1-(6-ethoxy-5-methoxypyridin-2-yl)-2-(methylsulfonyl)ethyl)-6-(2-fluorophenyl)-7-methyl-1H-imidazo[4,5-b]pyridin-2(3H)-one

4.65 g of product of Step 3 and 10g of triethylamine were dissolved in 50.0 mL of tetrahydrofuran at room temperature. The mixture was cooled to 0°C. 1.17 g of triphosgene and 10.0 mL of tetrahydrofuran were added dropwise to the above reaction solution. After the dropwise addition was completed, the reaction mixture was stirred at 0°C for 2 hours under nitrogen. After the reaction was completed, 100 mL of water was added, and the reaction mixture was extracted with ethyl acetate (20 mL x 3). The organic phases were combined, dried over anhydrous sodium sulfate, and filtered. The filtrate was concentrated under reduced pressure, and the residue was subjected to column chromatography to obtain the target compound.

ESI MS m/z: 501.15[M+1]⁺

### Example 2. (S)-3-(1-(6-ethoxy-5-methoxypyridin-2-yl)-2-(methylsulfonyl)ethyl) -6-(2-fluorophenyl)-7-methyl-1H-imidazo[4,5-b]pyridin-2(3H)-one

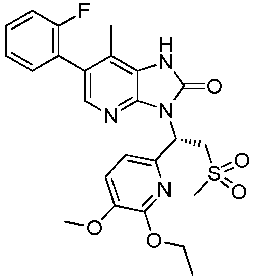

### Step 1, (S)-5-bromo-N-(1-(6-ethoxy-5-methoxypyridin-2-yl)-2-(methylsulfonyl) ethyl)-4-methyl-3-nitropyridin-2-amine

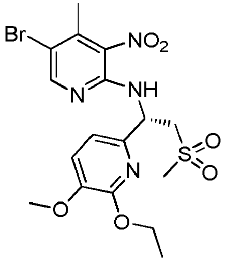

(S)-1-(6-ethoxy-5-methoxypyridin-2-yl)-2-(methylsulfonyl)ethylamine (1.43 g, 5.23 mmol) (prepared from the racemate by SFC resolution) was dissolved in 10 mL of N,N-dimethylformamide. 5-bromo -2-chloro-4-methyl-3-nitropyridine (1.93 g, 8.16 mmol) and 3 mL triethylamine were added under nitrogen. The mixture was heated to 100 °C for 16 h. The mixture was cooled to room temperature, and then quenched by adding water. The resulting mixture was extracted with ethyl acetate (50 mL^{∗}3). The organic phases were combined, washed with saturated saline, dried over anhydrous sodium sulfate and filtered. The filtrate was concentrated, and the residue was subjected to column chromatography to give 1.3 g of the target compound.

### Step 2, (S)-N-(1-(1-ethoxy-5-methoxypyridin-2-yl)-2-(methylsulfonyl)ethyl)-5-(2 -fluorophenyl)-4-methyl-3-nitropyridin-2-amine

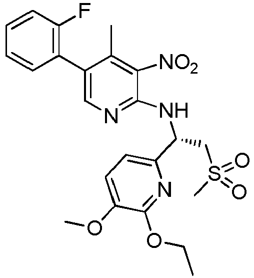

Product of Step 1 (121 mg, 0.25 mmol) was dissolved in 8 mL of mixed solvent (1,4-dioxane : water = 3:1). (2-fluorophenyl)boronic acid (52 mg, 0.37 mmol), (dppf)PdCl₂ (110 mg), and potassium carbonate (102 mg, 0.74 mmol) were added. The system was evacuated with suction and protected with nitrogen. The reaction was refluxed at 85°C for 8 hours. The mixture was cooled to room temperature, and then quenched by adding water. The resulting mixture was filtered through diatomaceous earth with suction, and the filtrate was extracted with ethyl acetate (50 mL^{∗}3). The organic phases were combined, washed with saturated saline, dried over anhydrous sodium sulfate and filtered. The filtrate was concentrated, and dried to give 1.3 g of the target compound.

### Step 3, (S)-N²-(1-(6-ethoxy-5-methoxypyridin-2-yl)-2-(methylsulfonyl)ethyl)-5-(2-fluorophenyl)-4-methylpyridin-2,3-diamine

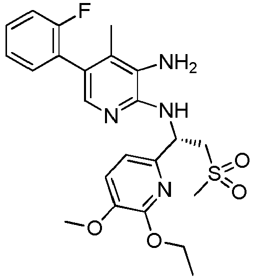

To a mixture of 1.2 g of product of Step 2 and 2.3 g of ammonium chloride was added ethanol (200 mL) at room temperature. 2.8 g of zinc powder was added in 20 batches at 0°C. The reaction mixture was stirred at 0 °C for 4 hours. After the reaction was completed, the reaction mixture was filtered to remove the zinc powder, and the filtrate was concentrated under reduced pressure to obtain the residue. The residue was dissolved in 300 mL of dichloromethane, and the mixture was washed for 2 times with aqueous sodium carbonate solution. The ethyl acetate layer was dried over anhydrous sodium sulfate, and filtered. The filtrate was concentrated under reduced pressure to give 0.8 g of the compound.

ESI MS m/z: 475.17[M+1]⁺

### Step 4: (S)-3-(1-(6-ethoxy-5-methoxypyridin-2-yl)-2-(methylsulfonyl)ethyl)-6-(2 -fluorophenyl)-7-methyl-1H-imidazo[4,5-b]pyridin-2(3H)-one

0.6 g of product of Step 3 and 0.8 g of triethylamine were dissolved in 50.0 mL of tetrahydrofuran at room temperature. The mixture was cooled to 0°C. 0.3 g of triphosgene and 10.0 mL of tetrahydrofuran were added dropwise to the above reaction solution. After the dropwise addition was completed, the reaction mixture was stirred at 0°C for 2 hours under nitrogen. After the reaction was completed, 20 mL of aqueous saturated sodium carbonate solution was added. The reaction mixture was extracted with 100 mL of ethyl acetate and washed for 2 times with 2M aqueous sodium carbonate solution. The ethyl acetate layer was dried over anhydrous sodium sulfate, and filtered. The filtrate was concentrated under reduced pressure and 0.4 g of the target compound was obtained.

¹H NMR (400 MHz, DMSO-D6) δ 11.52 (s, 1H), 7.75 (s, 1H), 7.50-7.46 (m,1H), 7.39-7.29(m,3H), 7.26-7.24(d,1H), 6.85-6.83 (d, 1H), 6.09-6.05 (q, 1H), 4.58(m,1H), 4.35-4.30(q,1H), 4.28-4.17(m,2H), 3.75(s,3H), 3.08(s,3H), 2.14(s,3H), 1.23-1.18(q,3H).

ESI MS m/z: 501.15[M+1]⁺

### Example 3. (R)-3-(1-(6-ethoxy-5-methoxypyridin-2-yl)-2-(methylsulfonyl)ethyl) -6-(2-fluorophenyl)-7-methyl-1H-imidazo[4,5-b]pyridin-2(3H)-one

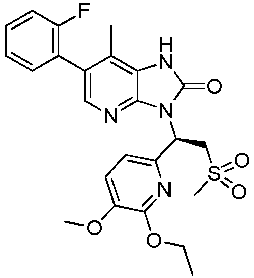

The compound was synthesized by referring to the method of Example 2.

¹H NMR (400 MHz, DMSO-D6) δ 11.50 (s, 1H), 7.75 (s, 1H), 7.50-7.46 (m, 1H), 7.39-7.29 (m, 3H), 7.26-7.24 (d, 1H), 6.85-6.83 (d, 1H), 6.09-6.05 (q, 1H), 4.65-4.59(q, 1H), 4.34-4.30(q,1H), 4.27-4.19(m,2H), 3.75(s,3H), 3.08(s,3H), 2.14(s,3H), 1.28-1.20(m,3H).

ESI MS m/z: 501.15[M+1]⁺

### Example 4. (S)-3-(1-(6-ethoxy-5-methoxypyridin-2-yl)-2-(methylsulfonyl)ethyl) -7-methyl-6-phenyl-1H-imidazo[4,5-b]pyridin-2(3H)-one

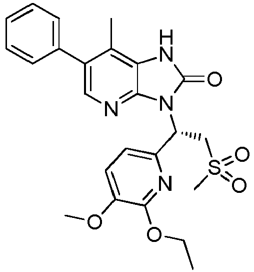

The compound was synthesized by referring to the method of Example 2.

¹H NMR (400 MHz, DMSO-D6) δ 11.47 (s, 1H), 7.76(s1H), 7.48-7.37 (m, 5H), 7.25-7.23(d, 1H), 6.82-6.79 (d,1H), 4.65-4.59(q,1H), 4.34-4.22(m,3H), 3.75(s,3H), 3.07(s,3H), 2.24(s,3H), 1.28-1.24(m,3H).

ESI MS m/z: 483.16[M+1]⁺

### Example 5. (R)-3-(1-(6-ethoxy-5-methoxypyridin-2-yl)-2-(methylsulfonyl)ethyl) -7-methyl-6-phenyl-1H-imidazo[4,5-b]pyridin-2(3H)-one

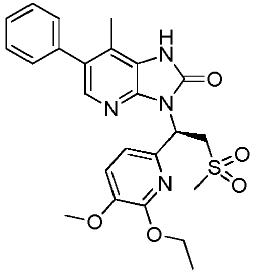

The compound was synthesized by referring to the method of Example 2.

¹H NMR (400 MHz, DMSO-D6) δ 11.47 (s, 1H), 7.76(s1H), 7.48-7.37 (m, 5H), 7.25-7.23(d, 1H), 6.82-6.79 (d,1H), 4.65-4.59(q,1H), 4.34-4.22(m,3H), 3.75(s,3H), 3.07(s,3H), 2.24(s,3H), 1.28-1.24(m,3H).

ESI MS m/z: 483.16[M+1]⁺

### Example 6. (S)-3-(1-(6-ethoxy-5-methoxypyridin-2-yl)-2-(methylsulfonyl)ethyl) -6-(4-fluorophenyl)-7-methyl-1H-imidazo[4,5-b]pyridin-2(3H)-one

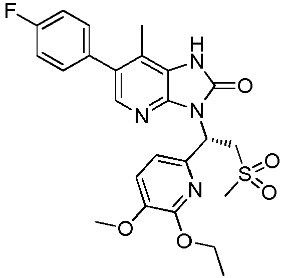

The compound was synthesized by referring to the method of Example 2.

ESI MS m/z: 501.15[M+1]⁺

### Example 7. (R)-3-(1-(6-ethoxy-5-methoxypyridin-2-yl)-2-(methylsulfonyl)ethyl) -6-(4-fluorophenyl)-7-methyl-1H-imidazo[4,5-b]pyridin-2(3H)-one

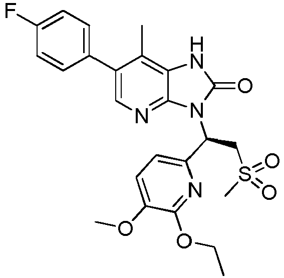

The compound was synthesized by referring to the method of Example 2.

ESI MS m/z: 501.15[M+1]⁺

### Example 8. (S)-3-(1-(6-ethoxy-5-methoxypyridin-2-yl)-2-(methylsulfonyl)ethyl) -7-methyl-6-(o-methylphenyl)-1H-imidazo[4,5-b]pyridin-2(3H)-one

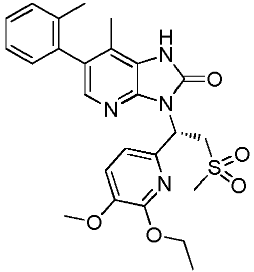

The compound was synthesized by referring to the method of Example 2.

¹H NMR (400 MHz, DMSO-D6) δ 11.44 (s, 1H), 7.63-7.62 (d, 1H), 7.33-7.24(m,4H), 7.13-7.11(q,1H), 6.89-6.86-(t,3H), 6.09-6.04(m,1H), 4.68-4.60(m,1H), 4.34-4.29(q,1H), 4.23-4.16(m,2H), 4.06-4.01(q,1H), 3.07(d,3H), 2.02-2.00(t,6H), 1.22-1.16(m,3H).

ESI MS m/z: 497.18[M+1]⁺

### Example 9. (R)-3-(1-(6-ethoxy-5-methoxypyridin-2-yl)-2-(methylsulfonyl)ethyl) -7-methyl-6-(o-methylphenyl)-1H-imidazo[4,5b]pyridin-2(3H)-one

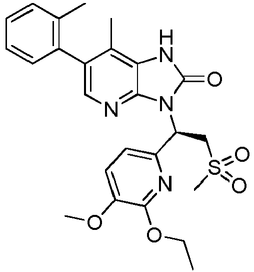

The compound was synthesized by referring to the method of Example 2.

¹H NMR (400 MHz, DMSO-D6) δ 11.44 (s, 1H), 7.63-7.62 (d, 1H), 7.32-7.24(m,4H), 7.13-7.11(q,1H), 6.89-6.86-(t,3H), 6.09-6.04(m,1H), 4.68-4.60(m,1H), 4.34-4.29(q,1H), 4.23-4.16(m,2H), 4.06-4.01(q,1H), 3.06(d,3H), 2.02-2.00(m,6H), 1.22-1.16(m,3H).

ESI MS m/z: 497.18[M+1]⁺

### Example 10. (S)-3-(1-(6-ethoxy-5-methoxypyridin-2-yl)-2-(methylsulfonyl)ethy l)-6-(2-ethylphenyl)-7-methyl-1H-imidazo[4,5-b]pyridin-2(3H)-one

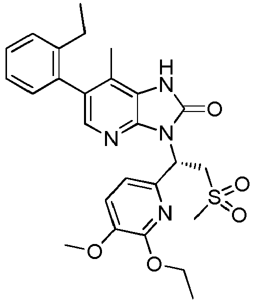

The compound was synthesized by referring to the method of Example 2.

¹H NMR (400 MHz, DMSO-D6) δ 11.43(s, 1H), 7.64-7.63(d, 1H), 7.37-7.33(m,2H), 7.28-7.23(m,2H), 7.09-7.07(d,1H), 6.92-6.88(t,1H), 6.09-6.04(m,1H), 4.66-4.63(q,1H), 4.33-4.29(t,1H), 4.22-4.18(m,2H), 3.75(s,3H), 3.06(s,3H), 2.49-1.99(m,2H), 2.05(s,3H), 1.28-1.16(m,3H), 1.00-0.94(m,3H).

ESI MS m/z: 511.19[M+1]⁺

### Example 11. (S)-3-(1-(6-ethoxy-5-methoxypyridin-2-yl)-2-(methylsulfonyl)ethyl) -6-(2-isopropylphenyl)-7-methyl-1H-imidazo[4,5-b]pyridin-2(3H)-one

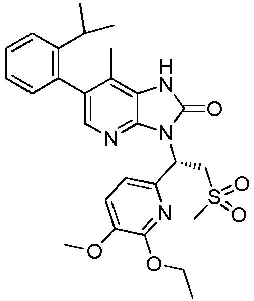

The compound was synthesized by referring to the method of Example 2.

ESI MS m/z: 525.218[M+1]⁺

### Example 12. (S)-3-(1-(6-ethoxy-5-methoxypyridin-2-yl)-2-(methylsulfonyl)ethy l)-6-(2-methoxyphenyl)-7-methyl-1H-imidazo[4,5-b]pyridin-2(3H)-one

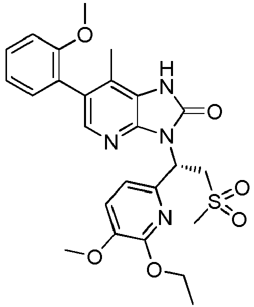

The compound was synthesized by referring to the method of Example 2.

¹H NMR (400 MHz, DMSO-D6) δ 11.39 (s, 1H), 7.63 (s, 1H), 7.42-7.38 (m,1H), 7.26-7.24(d,1H), 7.15-7.10(m,2H), 7.04-7.00(m,1H), 6.84-6.82 (d, 1H), 6.08-6.04 (q,1H), 4.63-4.60(t,1H), 4.33-4.21(m,3H), 3.75-3.73(d,6H), 3.08(s,3H), 2.05(s,3H), 1.25-1.21(t,3H).

ESI MS m/z: 513.17[M+1]⁺

### Example 13. (R)-3-(1-(6-ethoxy-5-methoxypyridin-2-yl)-2-(methylsulfonyl)ethy l)-6-(2-methoxyphenyl)-7-methyl-1H-imidazo[4,5-b]pyridin-2(3H)-one

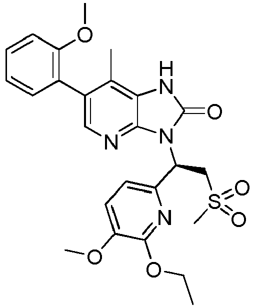

The compound was synthesized by referring to the method of Example 2.

¹H NMR (400 MHz, DMSO-D6) δ 11.39 (s, 1H), 7.63 (s, 1H), 7.42-7.38 (m,1H), 7.26-7.24(d,1H), 7.15-7.10(m,2H), 7.04-7.00(m,1H), 6.84-6.82 (d, 1H), 6.08-6.04(q,1H), 4.66-4.59(q,1H), 4.34-4.21(m,3H), 3.75-3.73(d,6H), 3.08(s,3H), 2.05(s,3H), 1.25-1.21(t,3H).

ESI MS m/z: 513.17[M+1]⁺

### Example 14. (S)-3-(1-(6-ethoxy-5-methoxypyridin-2-yl)-2-(methylsulfonyl)ethy l)-7-methyl-6-(2-(trifluoromethyl)phenyl)-1H-imidazo[ 4,5-b]pyridin-2(3H)-one

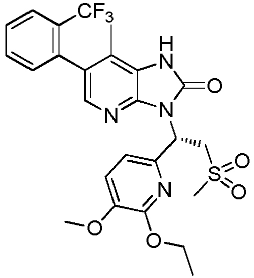

The compound was synthesized by referring to the method of Example 2.

ESI MS m/z: 551.15[M+1]⁺

### Example 15. (S)-2-(3-(1-(6-ethoxy-5-methoxypyridin-2-yl)-2-(methylsulfonyl)et hyl)-7-methyl-2-oxo-2,3-dihydro-1H-imidazo[4,5-b]pyridin-6-yl)benzonitrile

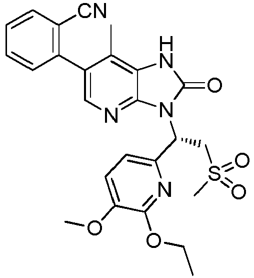

The compound was synthesized by referring to the method of Example 2.

¹H NMR (400 MHz, DMSO-D6) δ 11.36 (s, 1H), 7.89-7.86 (m, 1H), 7.79-7.77 (d, 1H), 7.47-7.43 (m, 1H), 7.23-7.21(d, 1H), 6.87-6.86(d,1H), 6.77-6.75(d,1H), 6.04-6.01(q,1H), 4.62-4.55(q,1H), 4.30-4.20(m,2H), 3.74(s,2H), 3.02(s,3H), 2.52-2.50(m,3H), 2.32(s,3H), 1.24-1.21(t,3H).

ESI MS m/z: 508.16[M+1]⁺

### Example 16. (R)-2-(3-(1-(6-ethoxy-5-methoxypyridin-2-yl)-2-(methylsulfonyl)e thyl)-7-methyl-2-oxo-2,3-dihydro-1H-imidazo[4,5-b]pyridin-6-yl)benzonitrile

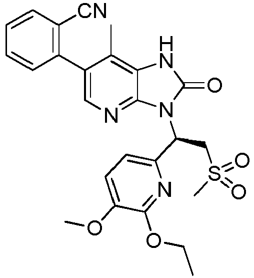

The compound was synthesized by referring to the method of Example 2.

¹H NMR (400 MHz, DMSO-D6) δ 11.36 (s, 1H), 7.89-7.86 (m, 1H), 7.79-7.77 (d, 1H), 7.46-7.43 (m, 1H), 7.23-7.21(d, 1H), 6.87-6.86 (d,1H), 6.77-6.75(d,1H), 6.04-6.01(q,1H), 4.62-4.55(q,1H), 4.30-4.20(m,2H), 3.74(s,2H), 3.02(s,3H), 2.51-2.50(m,3H), 2.32(s,3H), 1.24-1.21(t,3H).

ESI MS m/z: 508.16[M+1]⁺

### Example 17. (S)-3-(1-(6-ethoxy-5-methoxypyridin-2-yl)-2-(methylsulfonyl)ethy l)-7-methyl-6-(pyridin-2-yl)-1H-imidazo[4,5-b]pyridin-2(3H)-one

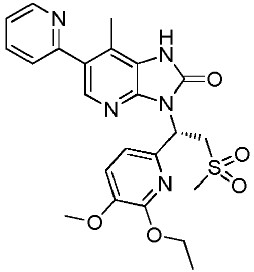

The compound was synthesized by referring to the method of Example 2.

ESI MS m/z: 484.16[M+1]⁺

### Example 18. (R)-3-(1-(6-ethoxy-5-methoxypyridin-2-yl)-2-(methylsulfonyl)ethy l)-7-methyl-6-(pyridin-2-yl)-1H-imidazo[4,5-b]pyridin-2(3H)-one

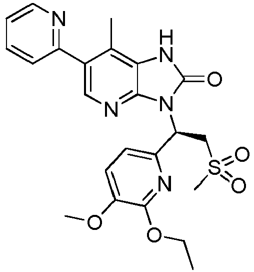

The compound was synthesized by referring to the method of Example 2.

ESI MS m/z: 484.16[M+1]⁺

### Example 19. (S)-3-(1-(6-ethoxy-5-methoxypyridin-2-yl)-2-(methylsulfonyl)ethy l)-1,7-dimethyl-6-(pyridin-2-yl)-1H-imidazo[4,5-b]pyridin-2(3H)-one

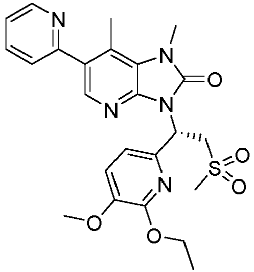

100 mg of compound of Example 17 was dissolved in 1 ml of N,N-dimethyl formamide. 100 mg of iodomethane and 0.1 mL of triethylamine were added under nitrogen. The mixture was heated to 0°C for 16 h. The mixture was cooled to room temperature, and then quenched by adding water. The resulting mixture was extracted with ethyl acetate (10 mL^{∗}3). The organic phases were combined, washed with saturated saline, dried over anhydrous sodium sulfate and filtered. The filtrate was concentrated, and the residue was subjected to column chromatography to give 53 mg of the target compound.

ESI MS m/z: 498.17[M+1]⁺

### Example 20. (S)-3-(1-(6-ethoxy-5-methoxypyridin-2-yl)-2-(methylsulfonyl)ethy l)-1-(2-methoxyethyl)-7-methyl-6-(pyridin-2-yl)-1H-imidazol[4,5-b]lpyridin-2(3H)-one

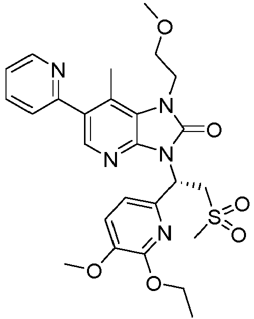

The compound was synthesized by referring to the method of Example 19.

ESI MS m/z: 542.208[M+1]⁺

### Example 21. (S)-3-(1-(6-ethoxy-5-methoxypyridin-2-yl)-2-(methylsulfonyl)ethy l)-6-(2-fluorophenyl)-1,7-dimethyl-1H-imidazo[4,5-b]pyridin-2(3H)-one

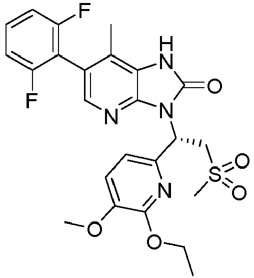

The compound was synthesized by referring to the method of Example 2.

ESI MS m/z: 519.14[M+1]⁺

### Example 22. (R)-3-(1-(6-ethoxy-5-methoxypyridin-2-yl)-2-(methylsulfonyl)ethy l)-6-(2-fluorophenyl)-1,7-dimethyl-1H-imidazo[4,5-b]pyridin-2(3H)-one

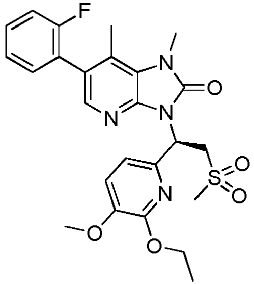

The compound was synthesized by referring to the method of Example 19.

ESI MS m/z: 515.17[M+1]⁺

### Example 23. (S)-6-(2,6-difluorophenyl)-3-(1-(6-ethoxy-5-methoxypyridin-2-yl)-2-(methylsulfonyl)ethyl)-7-methyl-1H-imidazo[4,5-b]pyridin-2(3H)-one

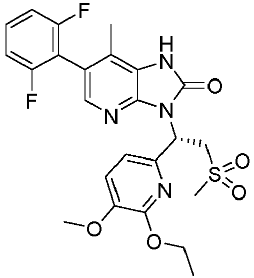

The compound was synthesized by referring to the method of Example 2.

¹H NMR (400 MHz, DMSO-D6) δ 11.38 (s, 1H), 7.79-7.77 (d, 1H), 7.73-7.68 (m,1H),7.23-7.21(d, 1H), 6.87-6.86(t,1H), 6.77-6.74(t,1H), 6.04-6.01(q,1H), 4.61-4.55(q,1H), 4.30-4.19(m,3H), 4.15-4.13(q,1H), 3.74(s,3H), 3.03(s,3H), 2.32(s,3H), 1.24-1.20(t,3H).

ESI MS m/z: 519.14[M+1]⁺

### Example 24. (R)(S)-6-(2,6-difluorophenyl)-3-(1-(6-ethoxy-5-methoxypyridin-2-yl)-2-(methylsulfonyl)ethyl)-7-methyl-1H-imidazo[4,5-b]pyridin-2(3H)-one

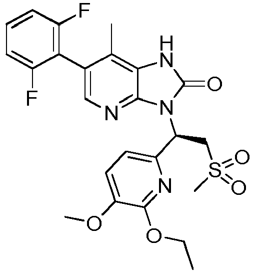

The compound was synthesized by referring to the method of Example 2.

ESI MS m/z: 519.14[M+1]⁺

### Example 25. (S)-6-(2,3-difluorophenyl)-3-(1-(6-ethoxy-5-methoxypyridin-2-yl)-2-(methylsulfonyl)ethyl)-7-methyl-1H-imidazo[4,5-b]pyridin-2(3H)-one

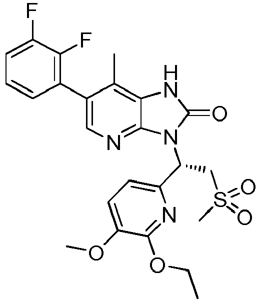

The compound was synthesized by referring to the method of Example 2.

¹H NMR (400 MHz, DMSO-D6) δ 11.38 (s, 1H), 7.78 (s, 1H), 7.74-7.67 (m, 1H), 7.23-7.21(d, 1H), 6.87-6.86(t,1H), 6.77-6.74(q,1H), 6.04-6.01(q, 1H),4.64-4.55(q,1H), 4.30-4.18(m,3H), 4.15-4.13(q,1H), 3.74(s,3H), 3.03(s,3H), 2.33(s,3H), 1.24-1.20(t,3H).

ESI MS m/z: 519.14[M+1]⁺

### Example 26. (R)-6-(2,3-difluorophenyl)-3-(1-(6-ethoxy-5-methoxypyridin-2-yl)-2-(methylsulfonyl)ethyl)-7-dimethyl-1H-imidazo[4,5-b]pyridin-2(3H)-one

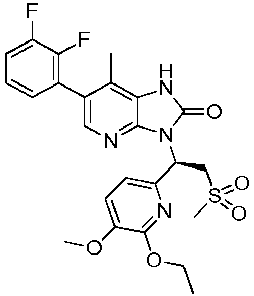

The compound was synthesized by referring to the method of Example 2.

ESI MS m/z: 519.14[M+1]⁺

### Example 27. (S)-6-(2,5-difluorophenyl)-3-(1-(6-ethoxy-5-methoxypyridin-2-yl)-2-(methylsulfonyl)ethyl)-7-methyl-1H-imidazo[4,5-b]pyridin-2(3H)-one

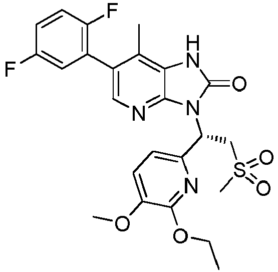

The compound was synthesized by referring to the method of Example 2.

¹H NMR (400 MHz, DMSO-D6) δ 11.53 (s, 1H), 7.78(s, 1H), 7.69-7.67 (q,1H), 7.42-7.24(m,2H), 6.85-6.83(q,1H), 6.09-6.06(q,1H), 4.64-4.58(q,1H), 4.27-4.18(m,2H), 4.15-4.13(m,1H), 4.04-4.03(d,1H), 3.75(s,3H), 3.08(s,3H), 2.16(d,3H), 1.29-1.16(m,3H).

ESI MS m/z: 519.14[M+1]⁺

### Example 28. (S)-6-(2,4-difluorophenyl)-3-(1-(6-ethoxy-5-methoxypyridin-2-yl)-2-(methylsulfonyl)ethyl)-7-methyl-1H-imidazo[4,5-b]pyridin-2(3H)-one

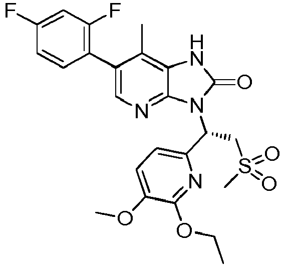

The compound was synthesized by referring to the method of Example 2.

¹H NMR (400 MHz, DMSO-D6) δ11.51(s,1H), 7.74(s,1H), 7.46-7.36(m,2H), 7.26-7.18(m,2H), 6.84(d,1H), 6.06(q,1H), 4.61(q,1H), 4.34-4.18(m,3H), 3.75(s,3H), 3.07(s,3H), 2.13(s,3H), 1.21(t,3H)

ESI MS m/z: 519.14[M+1]⁺

### Example 29. (S)-6-(2,4,5-trifluorophenyl)-3-(1-(6-ethoxy-5-methoxypyridin-2-y l)-2-(methylsulfonyl)ethyl)-7-methyl-1H-imidazo[4,5-b]pyridin-2(3H)-one

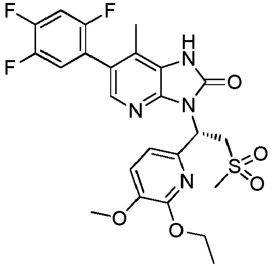

The compound was synthesized by referring to the method of Example 2.

ESI MS m/z: 537.13[M+1]⁺

### Example 30. (R)-6-(2,5-difluorophenyl)-3-(1-(6-ethoxy-5-methoxypyridin-2-yl)-2-(methylsulfonyl)ethyl)-7-methyl-1H-imidazo[4,5-b]pyridin-2(3H)-one

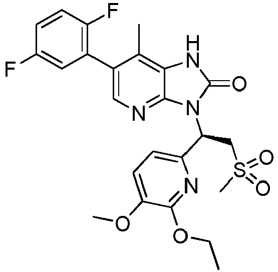

The compound was synthesized by referring to the method of Example 2.

ESI MS m/z: 519.14[M+1]⁺

### Example 31. (S)-6-(2,4-dimethylphenyl)-3-(1-(6-ethoxy-5-methoxypyridin-2-yl) -2-(methylsulfonyl)ethyl)-7-methyl-1H-imidazo[4,5-b]pyridin-2(3H)-one

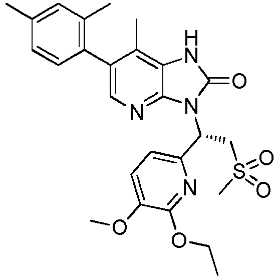

The compound was synthesized by referring to the method of Example 2.

¹H NMR (400 MHz, DMSO-D6) δ 11.41(s, 1H), 7.59(d, 1H), 7.27-7.24 (q, 1H), 7.13(s,1H), 7.07-7.05(d,1H), 7.00-6.97(q,1H), 6.89-6.85(d,1H), 6.08-6.04(m,1H), 4.64-4.59(q,1H), 4.33-4.29(t,1H), 4.23-4.16(m,2H), 3.75(s,3H), 3.06(s,3H), 2.32(s,3H), 2.01-1.97(q,6H), 1.22-1.17(m,3H).

ESI MS m/z: 511.19[M+1]⁺

### Example 32. (S)-6-(2,5-dimethylphenyl)-3-(1-(6-ethoxy-5-methoxypyridin-2-yl) -2-(methylsulfonyl)ethyl)-7-methyl-1H-imidazo[4,5-b]pyridin-2(3H)-one

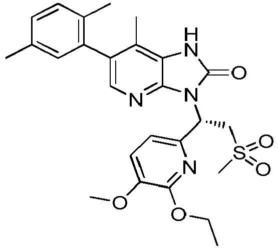

The compound was synthesized by referring to the method of Example 2.

¹H NMR (400MHz,DMSO-D6) δ11.42(s,1H), 7.61(s,1H), 7.60-7.13(m, 2H),7.11(t,1H), 6.93-6.85(m,2H), 6.09-6.05(m,1H), 4.66-4.59(m,1H), 4.33-4.23(m,1H) 4.22-4.15(m,2H), 3.75(s,3H), 3.06(s,3H), 2.29(s,3H), 2.00(d,3H), 1.96(d,3H), 1.40-1.18(m, 3H).

ESIMS m/z: 511.19[M+1]⁺

### Example 33. (S)-6-(2,3-dimethylphenyl)-3-(1-(6-ethoxy-5-methoxypyridin-2-yl) -2-(methylsulfonyl)ethyl)-7-methyl-1H-imidazo[4,5-b]pyridin-2(3H)-one

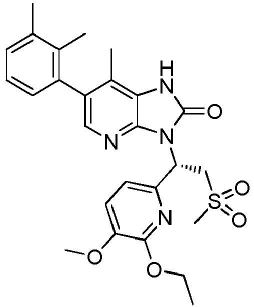

The compound was synthesized by referring to the method of Example 2.

¹H NMR (400 MHz, DMSO-D6) 811.42 (s, 1H), 7.60(s, 1H), 7.27-7.12(m, 3H), 6.96-6.85(m, 2H), 6.09-6.04(m,1H), 4.66-4.62(t,1H), 4.33-4.30(m, 1H), 4.23-4.17(m, 2H), 3.75(s, 3H), 3.07(s, 3H), 2.31(d,3H), 1.99(s,3H), 1.91(d,3H),1.26-1.18(m, 3H).

ESI MS m/z: 511.19[M+1]⁺

### Example 34. (S)-6-(2,6-dimethylphenyl)-3-(1-(6-ethoxy-5-methoxypyridin-2-yl) -2-(methylsulfonyl)ethyl)-7-methyl-1H-imidazo[4,5-b]pyridin-2(3H)-one

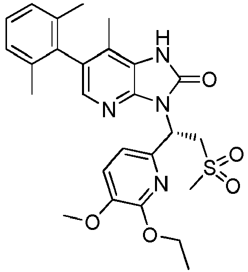

The compound was synthesized by referring to the method of Example 2.

ESI MS m/z: 511.19[M+1]⁺

### Example 35. (S)-6-(4-fluoro-2-methylphenyl)-3-(1-(6-ethoxy-5-methoxypyridin -2-yl)-2-(methylsulfonyl)ethyl)-7-methyl-1H-imidazo[4,5-b]pyridin-2(3H)-one

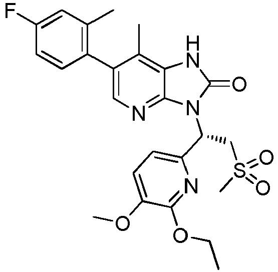

The compound was synthesized by referring to the method of Example 2.

¹H NMR (400 MHz, DMSO-D6) δ11.45(s,1H), 7.62(d,1H), 7.27-7.06 (m,4H), 6.89-6.85(d,1H), 6.08-6.04(m,1H), 4.64-4.59 (q,1H), 4.33-4.29(t,1H),4.23-4.15(m,2H), 3.75(s,3H), 3.06(s,3H)2.02-1.99(q,6H), 1.22-1.18(m,3H).

ESI MS m/z: 515.17[M+1]⁺

### Example 36. (S)-6-(5-fluoro-2-methylphenyl)-3-(1-(6-ethoxy-5-methoxypyridin -2-yl)-2-(methylsulfonyl)ethyl)-7-methyl-1H-imidazo[4,5-b[pyridin-2(3H)-one

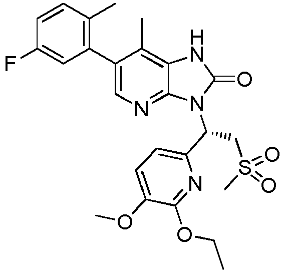

The compound was synthesized by referring to the method of Example 2.

¹H NMR (400 MHz, DMSO-D6) δ 11.47(d,1H), 7.65(s,1H), 7.64-7.35(m,1H), 7.34-7.24(m,1H), 7.18-7.13(m,1H), 7.02-6.99(m,1H), 6.98-6.85(m,1H), 6.09-6.05(m,1H), 4.60(d,1H), 4.34-4.23(m,1H), 4.22-4.16(m,2H), 3.75(s,3H), 3.07(d,3H), 2.03(d,3H), 1.98(t,3H), 1.40-1.17(m, 3H)

ESI MS m/z: 515.17[M+1]⁺

### Example 37. (S)-6-(3-fluoro-2-methylphenyl)-3-(1-(6-ethoxy-5-methoxypyridin -2-yl)-2-(methylsulfonyl)ethyl)-7-methyl-1H-imidazo[4,5-b]pyridin-2(3H)-one

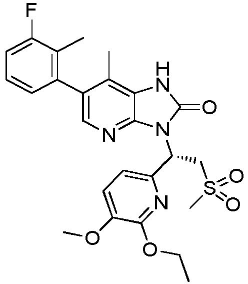

The compound was synthesized by referring to the method of Example 2.

¹H NMR (400 MHz, DMSO-D6) δ 11.49 (s, 1H), 7.65 (s, 1H), 7.33-7.19 (m, 3H), 7.02-6.99(q, 1H), 6.89-6.85(q,1H), 6.09-6.04(m,1H), 4.65-4.59(m,1H) ,4.34-4.30(m,1H), 4.23-4.18(m,2H), 3.75(s,3H), 3.08(s,3H), 2.03(s,3H), 1.93(s,3H), 1.21-1.19(t,3H).

ESI MS m/z: 515.17[M+1]⁺

### Example 38. (S)-6-(2-fluoro-5-methylphenyl)-3-(1-(6-ethoxy-5-methoxypyridin -2-yl)-2-(methylsulfonyl)ethyl)-7-methyl-1H-imidazo[4,5-b]pyridin-2(3H)-one

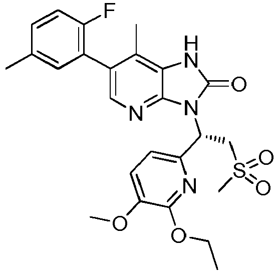

The compound was synthesized by referring to the method of Example 2.

¹H NMR (400 MHz, DMSO-D6) δ 11.51 (s, 1H), 7.74 (s, 1H), 7.27-7.15 (m, 4H), 6.84-6.82(t, 1H), 6.08-6.05 (q, 1H), 4.64-4.57(q,1H), 4.34-4.20(m,3H), 3.75(s,3H), 3.08(s,3H), 2.33(s,3H), 2.14(s,3H), 1.24-1.20(t,3H).

ESI MS m/z: 515.17[M+1]⁺

### Example 39. (S)-6-(2-fluoro-3-methylphenyl)-3-(1-(6-ethoxy-5-methoxypyridin -2-yl)-2-(methylsulfonyl)ethyl)-7-methyl-1H-imidazo[4,5-b[pyridin-2(3H)-one

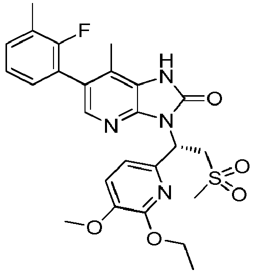

The compound was synthesized by referring to the method of Example 2.

¹H NMR (400 MHz, DMSO-D6) δ 11.50 (s, 1H), 7.73 (s, 1H), 7.36-7.33 (q, 1H), 7.26-7.16(m, 3H), 6.85-6.83 (q,1H), 6.08-6.05(q,1H), 4.64-4.58(q,1H) ,4.34-4.30(m,1H), 4.25-4.19(m,2H), 3.75(s,3H), 3.08(s,3H), 2.30(s,3H), 2.13(s,3H), 1.24-1.19(t,3H).

ESI MS m/z: 515.17[M+1]⁺

### Example 40. (S)-6-(2-fluoro-4-methylphenyl)-3-(1-(6-ethoxy-5-methoxypyridin -2-yl)-2-(methylsulfonyl)ethyl)-7-methyl-1H-imidazo[4,5-b]pyridin-2(3H)-one

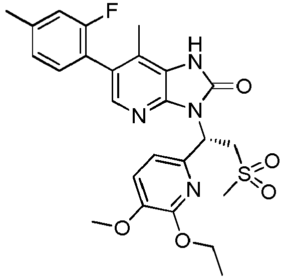

The compound was synthesized by referring to the method of Example 2.

¹H NMR (400 MHz, DMSO-D6) δ 11.50 (s, 1H), 7.72(s, 1H), 7.26-7.21 (t, 2H), 7.17-7.11(q,2H), 6.84-6.82(t, 1H), 6.08-6.05 (q,1H), 4.64-4.57(q,1H), 4.34-4.30(q,1H), 4.27-4.18(m,2H), 3.75(s,3H), 3.08(s,3H), 2.38(s,3H), 2.13(s,3H), 1.23-1.18(t,3H).

ESI MS m/z: 515.17[M+1]⁺

### Example 41. (S)-6-(2-fluoro-5-methoxyphenyl)-3-(1-(6-ethoxy-5-methoxypyridi n-2-yl)-2-(methylsulfonyl)ethyl)-7-methyl-1H-imidazo[4,5-b]pyridin-2(3H)-one

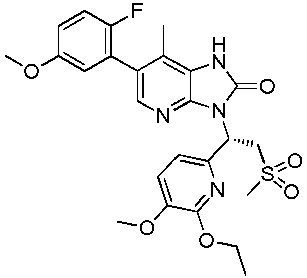

The compound was synthesized by referring to the method of Example 2.

¹HNMR (400 MHz, DMSO-D6) δ 11.50(s,1H), 7.77(d,1H), 7.27-7.20(m,2H), 7.00-6.99(m,1H), 7.98-6.82(m,2H), 6.07(m,1H), 4.62-4.58(m,1H), 4.34-4.19(m,3H), 3.77(s,3H), 3.73(d,3H), 3.05(s,3H), 2.51-2.50(m,3H), 1.20-1.09(m,3H)

ESI MS m/z: 531.16[M+1]⁺

### Example 42. (S)-6-(2-fluoro-3-methoxyphenyl)-3-(1-(6-ethoxy-5-methoxypyridi n-2-yl)-2-(methylsulfonyl)ethyl)-7-methyl-1H-imidazo[4,5-b]pyridin-2(3H)-one

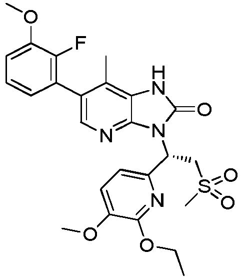

The compound was synthesized by referring to the method of Example 2.

ESI MS m/z: 531.16[M+1]⁺

### Example 43. (S)-6-(2-fluoro-4-methoxyphenyl)-3-(1-(6-ethoxy-5-methoxypyridi n-2-yl)-2-(methylsulfonyl)ethyl)-7-methyl-1H-imidazo[4,5-b]pyridin-2(3H)-one

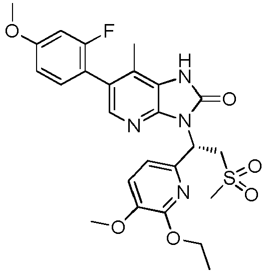

The compound was synthesized by referring to the method of Example 2.

¹HNMR (400 MHz, DMSO-D6) δ 11.49(s,1H), 7.71(s,1H), 7.29-7.23(m,2H), 6.96(q,1H), 6.88(q,1H), 6.82(q,1H), 6.06(q,1H), 4.64-4.58(m,1H), 4.34-4.18(m,3H), 3.82(s,3H), 3.74(d,3H), 3.05(d,3H), 2.14(d,3H), 1.28-1.19(m,3H)

ESI MS m/z: 531.16[M+1]⁺

### Example 44. (S)-6-(5-fluoro-2-methoxyphenyl)-3-(1-(6-ethoxy-5-methoxypyridi n-2-yl)-2-(methylsulfonyl)ethyl)-7-methyl-1H-imidazo]4,5-b[pyridin-2(3H)-one

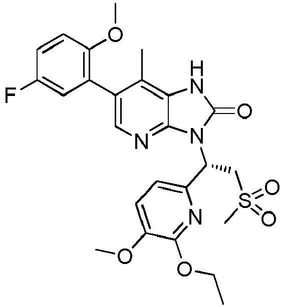

The compound was synthesized by referring to the method of Example 2.

¹HNMR (400 MHz, DMSO-D6) δ 11.40(d,1H), 7.70(d,1H), 7.26-7.21(m,2H), 7.20-7.11(m,1H), 7.10-6.99(m,1H), 6.98-6.84(m,1H), 6.07(t,1H), 4.62-4.59(m,1H), 4.33-4.03(m,3H), 3.76(d,3H), 3.72(d,3H), 3.05(d,3H), 2.07(s,3H), 1.40-1.21(m, 3H)

ESI MS m/z: 531.16[M+1]⁺

### Example 45. (S)-6-(4-fluoro-2-methoxyphenyl)-3-(1-(6-ethoxy-5-methoxypyridi n-2-yl)-2-(methylsulfonyl)ethyl)-7-methyl-1H-imidazo[4,5-b]pyridin-2(3H)-one

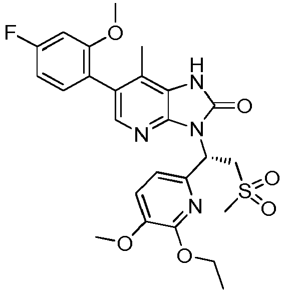

The compound was synthesized by referring to the method of Example 2.

¹HNMR (400 MHz, DMSO-D6) δ 11.40(s,1H), 7.70(d,1H), 7.26-7.16(m,2H), 7.15-7.01(m,1H), 6.87-6.81(m,2H), 6.05(q,1H), 4.65-4.59(m,1H), 4.33-4.21(m,3H), 4.74(s,3H), 3.73(s,3H), 3.03(s,3H), 2.01(d,3H), 1.28-1.16(m, 3H)

ESI MS m/z: 531.16[M+1]⁺

### Example 46. (S)-6-(6-fluoro-2-methoxyphenyl)-3-(1-(6-ethoxy-5-methoxypyridi n-2-yl)-2-(methylsulfonyl)ethyl)-7-methyl-1H-imidazo[4,5-b]pyridin-2(3H)-one

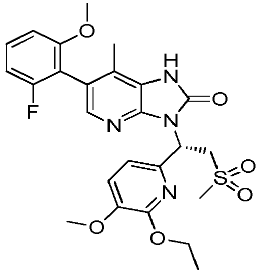

The compound was synthesized by referring to the method of Example 2.

¹HNMR (400 MHz, DMSO-D6) δ 11.36(s,1H), 7.78(d,1H), 7.21(d,1H), 6.87-6.85(q,2H), 6.77-6.75(q,1H), 6.04-6.01(q,1H), 4.62-4.55(m,1H), 4.30-4.19(m,3H), 3.74(d,3H), 3.06(t,3H), 2.32(s,3H), 1.28-1.19(m, 3H).

ESI MS m/z: 531.16[M+1]⁺

### Example 47. (R)-3-(1-(6-ethoxy-5-methoxypyridin-2-yl)-2-(methylsulfonyl)ethy l)-1-ethyl-7-methyl-6-phenyl-1H-imidazo[4,5-b]pyridin-2(3H)-one

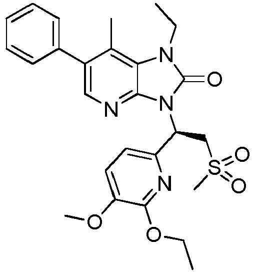

The compound was synthesized by referring to the method of Example 19.

ESI MS m/z: 511.19[M+1]⁺

### Example 48. (S)-3-(1-(6-ethoxy-5-methoxypyridin-2-yl)-2-(methylsulfonyl)ethy l)-1-ethyl-6-(4-fluorophenyl)-7-methyl-1H-imidazo[4,5-b]pyridin-2(3H)-one

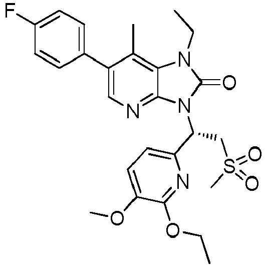

The compound was synthesized by referring to the method of Example 19.

ESI MS m/z: 529.18[M+1]⁺

### Example 49. (R)-3-(1-(6-ethoxy-5-methoxypyridin-2-yl)-2-(methylsulfonyl)ethy l)-1-ethyl-6-(4-fluorophenyl)-7-methyl-1H-imidazo[4,5-b]pyridin-2(3H)-one

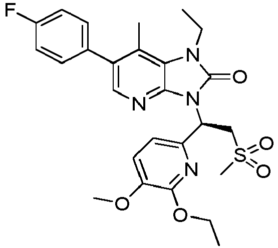

The compound was synthesized by referring to the method of Example 19.

ESI MS m/z: 529.18[M+1]⁺

### Example 50. (S)-3-(1-(6-ethoxy-5-methoxypyridin-2-yl)-2-(methylsulfonyl)ethy l)-1-ethyl-7-methyl-6-(o-methylphenyl)-1H-imidazo[4,5-b]pyridin-2(3H)-one

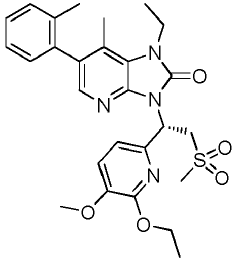

The compound was synthesized by referring to the method of Example 19.

ESI MS m/z: 525.21[M+1]⁺

### Example 51. (S)-3-(1-(6-ethoxy-5-methoxypyridin-2-yl)-2-(methylsulfonyl)ethy l)-6-(2-fluorophenyl)-7-methyl-1-(2,2,2-trifluoroethyl)-1H-imidazo[4,5-b]pyridin-2(3H)-one

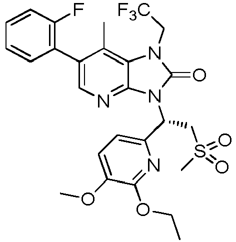

The compound was synthesized by referring to the method of Example 19.

ESI MS m/z: 583.16[M+1]⁺

### Example 52. (R)-3-(1-(6-ethoxy-5-methoxypyridin-2-yl)-2-(methylsulfonyl)ethy l)-6-(2-fluorophenyl)-7-methyl-1-(2,2,2-trifluoroethyl)-1H-imidazo[4,5-b]pyridin-2(3H)-one

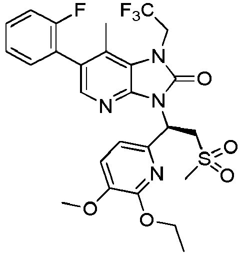

The compound was synthesized by referring to the method of Example 19.

ESI MS m/z: 583.16[M+1]⁺

### Example 53. (S)-3-(1-(6-ethoxy-5-methoxypyridin-2-yl)-2-(methylsulfonyl)ethy l)-7-methyl-6-phenyl-1-(2,2,2-trifluoroethyl)-1H-imidazo[4,5-b]pyridin-2(3H)-one

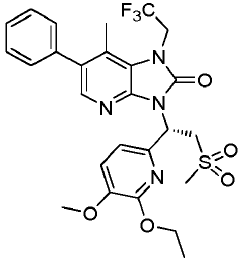

The compound was synthesized by referring to the method of Example 19.

SI MS m/z: 565.17[M+1]⁺

### Example 54. (R)-3-(1-(6-ethoxy-5-methoxypyridin-2-yl)-2-(methylsulfonyl)ethy l)-7-methyl-6-phenyl-1-(2,2,2-trifluoroethyl)-1H-imidazo[4,5-b]pyridin-2(3H)-one

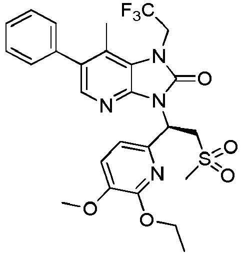

The compound was synthesized by referring to the method of Example 19.

SI MS m/z: 565.17[M+1]⁺

### Example 55. (S)-3-(1-(6-ethoxy-5-methoxypyridin-2-yl)-2-(methylsulfonyl)ethy l)-7-methyl-6-(o-methylphenyl)-1-(2,2,2-trifluoroethyl)-1H-imidazo[4,5-b]pyridin-2(3H)-one

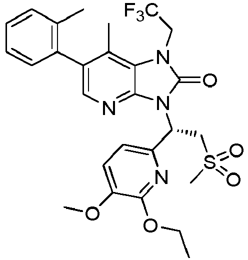

The compound was synthesized by referring to the method of Example 19.

ESI MS m/z: 579.18[M+1]⁺

### Example 56. (S)-1-(2,2-difluoroethyl)-3-(1-(6-ethoxy-5-methoxypyridin-2-yl)-2-(methylsulfonyl)ethyl)-6-(2-fluorophenyl)-7-methyl-1H-imidazo[4,5-b]pyridin-2(3H)-on e

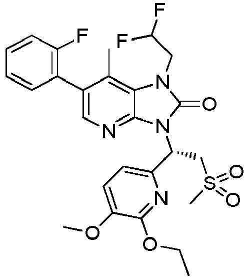

The compound was synthesized by referring to the method of Example 19.

ESI MS m/z: 565.17 [M+1]⁺

### Example 57. (S)-1-(2,2-difluoroethyl)-3-(1-(6-ethoxy-5-methoxypyridin-2-yl)-2-(methylsulfonyl)ethyl)-7-methyl-6-phenyl-1H-imidazo[4,5-b]pyridin-2(3H)-one

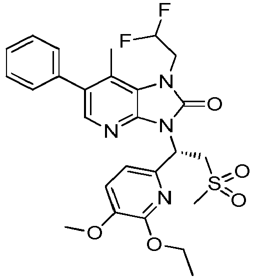

The compound was synthesized by referring to the method of Example 19.

ESI MS m/z: 547.17[M+1]⁺

### Example 58. (S)-1-(2,2-difluoroethyl)-3-(1-(6-ethoxy-5-methoxypyridin-2-yl)-2-(methylsulfonyl)ethyl)-6-(4-fluorophenyl)-7-methyl-1H-imidazo[4,5-b]pyridin-2(3H)-on e

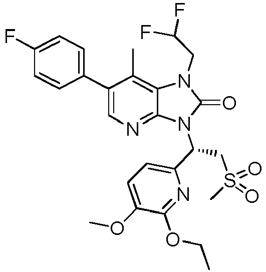

The compound was synthesized by referring to the method of Example 19.

ESI MS m/z: 565.17[M+1]⁺

### Example 59. (S)-1-(2,2-difluoroethyl)-3-(1-(6-ethoxy-5-methoxypyridin-2-yl)-2-(methylsulfonyl)ethyl)-7-methyl-6-(o-methylphenyl)-1H-imidazo[4,5-b]pyridin-2(3H)-on e

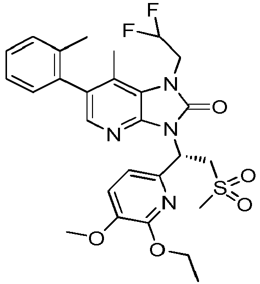

The compound was synthesized by referring to the method of Example 19.

ESI MS m/z: 561.19[M+1]⁺

### Example 60. (S)-3-(1-(5-(difluoromethoxy)-6-ethoxypyridin-2-yl)-2-(methylsulf onyl)ethyl)-6-(2-fluorophenyl)-7-methyl-1H-imidazo[4,5-b]pyridin-2(3H)-one

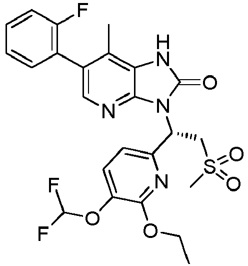

The compound was synthesized by referring to the method of Example 19.

ESI MS m/z: 537.13[M+1]⁺

### Example 61. (S)-3-(1-(5-(difluoromethoxy)-6-ethoxypyridin-2-yl)-2-(methylsulf onyl)ethyl)-7-methyl-6-phenyl-1H-imidazo[4,5-b]pyridin-2(3H)-one

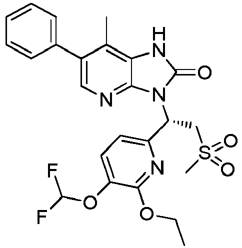

The compound was synthesized by referring to the method of Example 2.

ESI MS m/z: 519.14 [M+1]⁺

### Example 62. (R)-3-(1-(5-(difluoromethoxy)-6-ethoxypyridin-2-yl)-2-(methylsul fonyl)ethyl)-7-methyl-6-phenyl-1H-imidazo[4,5-b]pyridin-2(3H)-one

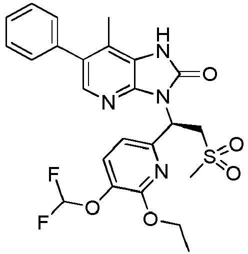

The compound was synthesized by referring to the method of Example 2.

ESI MS m/z: 519.14 [M+1]⁺

### Example 63. (S)-3-(1-(5-(difluoromethoxy)-6-ethoxypyridin-2-yl)-2-(methylsulf onyl)ethyl)-6-(4-fluorophenyl)-7-methyl-1H-imidazo[4,5-b]pyridin-2(3H)-one

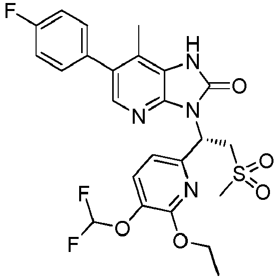

The compound was synthesized by referring to the method of Example 2.

ESI MS m/z: 537.13[M+1]⁺

### Example 64. (S)-3-(1-(5-(difluoromethoxy)-6-ethoxypyridin-2-yl)-2-(methylsulf onyl)ethyl)-7-methyl-6-(o-methylphenyl)-1H-imidazo[4,5-b]pyridin-2(3H)-one

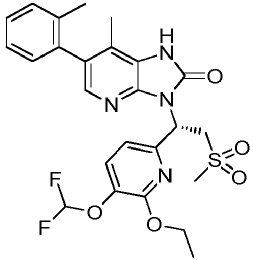

The compound was synthesized by referring to the method of Example 2.

ESI MS m/z: 533.16[M+1]⁺

### Example 65. (S)-3-(1-(5-(difluoromethoxy)-6-ethoxypyridin-2-yl)-2-(methylsulf onyl)ethyl)-6-(2-fluorophenyl)-1,7-dimethyl-1H-imidazo[4,5-b]pyridin-2(3H)-one

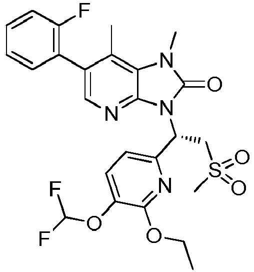

The compound was synthesized by referring to the method of Example 19.

ESI MS m/z: 551.15[M+1]⁺

### Example 66. (S)-3-(1-(5-(difluoromethoxy)-6-ethoxypyridin-2-yl)-2-(methylsulf onyl)ethyl)-1,7-dimethyl-6-phenyl-1H-imidazo[4,5-b]pyridin-2(3H)-one

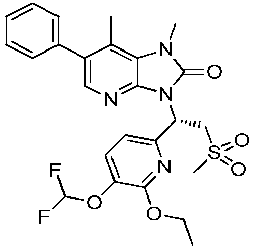

The compound was synthesized by referring to the method of Example 19.

ESI MS m/z: 533.16[M+1]⁺

### Example 67. (S)-3-(1-(5-(difluoromethoxy)-6-ethoxypyridin-2-yl)-2-(methylsulf onyl)ethyl)-6-(4-fluorophenyl)-1,7-dimethyl-1H-imidazo[4,5-b]pyridin-2(3H)-one

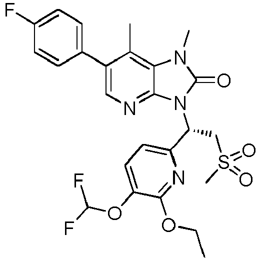

The compound was synthesized by referring to the method of Example 19.

ESI MS m/z: 551.15[M+1]⁺

### Example 68. (S)-3-(1-(5-(difluoromethoxy)-6-ethoxypyridin-2-yl)-2-(methylsulf onyl)ethyl)-1,7-dimethyl-6-(o-methylphenyl)-1H-imidazo[4,5-b]pyridin-2(3H)-one

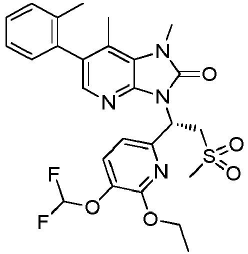

The compound was synthesized by referring to the method of Example 19.

ESI MS m/z: 547.17 [M+1]⁺

### Example 69. (S)-1-(2,2-difluoroethyl)-3-(1-(6-ethoxy-5-isopropoxypyridin-2-yl) -2-(methylsulfonyl)ethyl)-6-(2-fluorophenyl)-7-methyl-1H-imidazo[4,5-b]pyridin-2(3H)-one

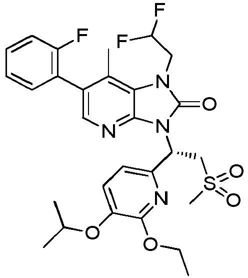

The compound was synthesized by referring to the method of Example 19.

ESI MS m/z: 593.20 [M+1]⁺

### Example 70. (S)-1-(2,2-difluoroethyl)-3-(1-(6-ethoxy-5-isopropoxypyridin-2-yl) -2-(methylsulfonyl)ethyl)-7-methyl-6-phenyl-1H-imidazo[4,5-b]pyridin-2(3H)-one

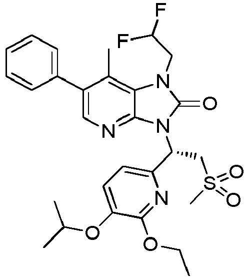

The compound was synthesized by referring to the method of Example 19.

ESI MS m/z: 575.21[M+1]⁺

### Example 71. (S)-1-(2,2-difluoroethyl)-3-(1-(6-ethoxy-5-isopropoxypyridin-2-yl) -2-(methylsulfonyl)ethyl)-6-(4-fluorophenyl)-7-methyl-1H-imidazo[4,5-b]pyridin-2(3H)-one

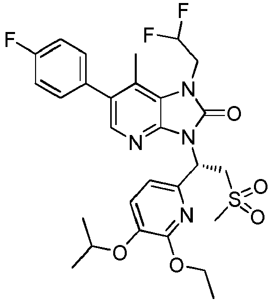

The compound was synthesized by referring to the method of Example 19.

ESI MS m/z: 593.20[M+1]⁺

### Example 72. (S)-1-(2,2-difluoroethyl)-3-(1-(6-ethoxy-5-isopropoxypyridin-2-yl) -2-(methylsulfonyl)ethyl)-7-methyl-6-(o-methylphenyl)-1H-imidazo[4,5-b]pyridin-2(3H) -one

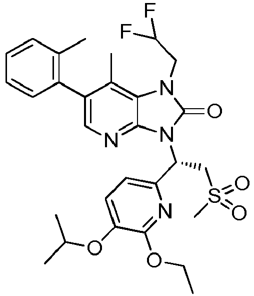

The compound was synthesized by referring to the method of Example 19.

ESI MS m/z: 589.22 [M+1]⁺

### Example 73. (S)-3-(1-(6-ethoxy-5-methoxypyridin-2-yl)-2-(methylsulfonyl)ethy l)-6-bromo-7-methyl-1H-imidazo[4,5-b]pyridin-2(3H)-one

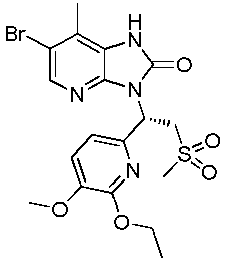

The compound was synthesized by referring to the method of Example 2.

ESI MS m/z: 485.04[M+1]⁺

### Example 74. (S)-3-(1-(6-ethoxy-5-methoxypyridin-2-yl)-2-(methylsulfonyl)ethy l)-6-(2-fluorophenyl)-1H-imidazo[4,5-b]pyridin-2(3H)-one

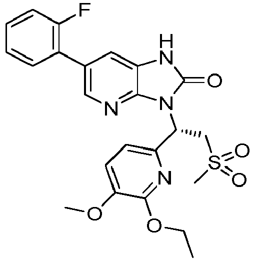

The compound was synthesized by referring to the method of Example 2.

¹H NMR (400 MHz, DMSO-D6) δ11.39(s,1H), 8.06(t,1H), 7.57-7.59(m,1H), 7.41-7.55(m,2H), 7.31-7.36(m,2H), 7.23-7.31(m,1H), 6.86(d,1H), 6.07(q,1H), 4.36-4.62(m,1H), 4.21-4.32(m,1H), 4.17-4.21(m,2H), 3.74(s,3H), 3.07(s,3H), 1.15-1.23(m,3H).

ESI MS m/z: 487.14[M+1]⁺

### Example 75. (S)-3-(1-(6-ethoxy-5-methoxypyridin-2-yl)-2-(methylsulfonyl)ethy l)-6-(2-fluorophenyl)-1-methyl-1H-imidazo[4,5-b]pyridin-2(3H)-one

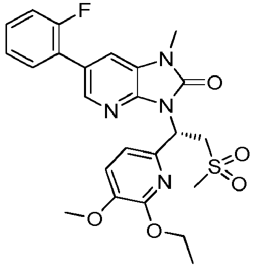

The compound was synthesized by referring to the method of Example 2.

¹H NMR (400 MHz, DMSO-D6) δ8.11(m,1H), 7.77(t,1H), 7.58-7.62 (m,1H), 7.43-7.47(m,1H), 7.32--7.38(m,2H), 6.12(dd,1H), 4.58-4.64(m,1H), 4.16-4.22(m,2H), 4.32-437(m,1H), 3.74(s,3H), 3.43(s,3H), 2.96(s,3H), 1.21(t,3H).

ESI MS m/z: 501.15[M+1]⁺

### Example 76. (S)-3-(1-(6-ethoxy-5-methoxypyridin-2-yl)-2-(methylsulfonyl)ethy l)-6-phenyl-1H-imidazo[4,5-b]pyridin-2(3H)-one

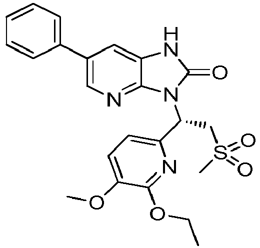

The compound was synthesized by referring to the method of Example 2.

¹H NMR (400 MHz, DMSO-D6) δ11.39(s, 1H), 8.19(d,1H), 7.66(d,2H), 7.55(d,1H), 7.47(t,2H), 7.37(t,1H), 7.24(d,1H), 7.84(d,1H), 6.07(q,1H), 4.60(q,1H), 4.32(q,1H), 4.25-4.20(m,2H), 3.74(s,3H), 3.06(s,3H), 1.22(t,3H).

ESI MS m/z: 469.15[M+1]⁺

### Example 77. (S)-3-(1-(6-ethoxy-5-methoxypyridin-2-yl)-2-(methylsulfonyl)ethy l)-6-phenyl-1-methyl-1H-imidazo[4,5-b]pyridin-2(3H)-one

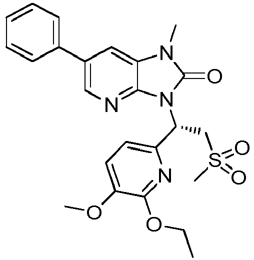

The compound was synthesized by referring to the method of Example 19.

¹HNMR (400 MHz, DMSO-D6) δ 8.25(d,1H), 7.89(d,1H), 7.72(d,2H), 7.49(t,2H), 7.39(t,1H), 7.23(d,1H), 6.86(d,1H), 6.10(q,1H), 4.60(q,1H), 4.34(q,1H), 4.23-4.18(m,2H), 3.74(s,3H), 3.45(s,3H), 3.06(s,3H), 1.21(t,3H)

ESI MS m/z: 483.16[M+1]⁺

### Example 78. (S)-3-(1-(6-ethoxy-5-methoxypyridin-2-yl)-2-(methylsulfonyl)ethy l)-6-(4-fluorophenyl)-1H-imidazo[4,5-b]pyridin-2(3H)-one

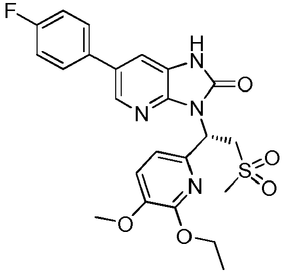

The compound was synthesized by referring to the method of Example 2.

ESI MS m/z: 487.14[M+1]⁺

### Example 79. (S)-3-(1-(6-ethoxy-5-methoxypyridin-2-yl)-2-(methylsulfonyl)ethy l)-6-(o-methylphenyl)-1H-imidazo[4,5-b]pyridin-2(3H)-one

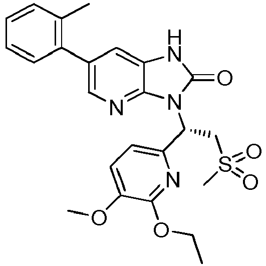

The compound was synthesized by referring to the method of Example 2.

¹H NMR (400 MHz, DMSO-D6) δ 11.31 (s, 1H), 7.71(s, 1H), 7.22-7.32 (m,6H), 6.88-6.91(m,1H), 6.06-6.09(dd,1H), 4.59-4.65(q,1H), 4.31-4.35(m,1H), 4.19(m,2H), 3.75(s,3H), 3.08(s,3H), 2.24(s,3H), 1.16-1.19(q,3H).

ESI MS m/z: 483.16 [M+1]⁺

### Example 80. (S)-3-(1-(6-ethoxy-5-methoxypyridin-2-yl)-2-(methylsulfonyl)ethy l)-1-methyl-6-(o-methylphenyl)-1H-imidazo[4,5-b]pyridin-2(3H)-one

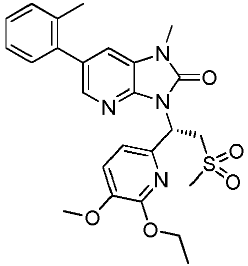

The compound was synthesized by referring to the method of Example 19.

¹H NMR (400 MHz,DMSO-D6)δ7.90(d,1H), 7.62(t,1H), 7.24-7.35(m,5H), 6.91(t,1H), 6.11(q,1H), 4.62(q,1H), 4.20-4.37(m,1H), 4.16-4.19(m,2H), 3.75(s,3H), 3.40(s,3H), 3.07(s,3H), 2.26(s,3H), 1.17(t,3H).

ESI MS m/z: 497.18 [M+1]⁺

### Example 81. (S)-3-(1-(6-ethoxy-5-methoxypyridin-2-yl)-2-(methylsulfonyl)ethy l)-6-(2-ethylphenyl)-1H-imidazo[4,5-b]pyridin-2(3H)-one

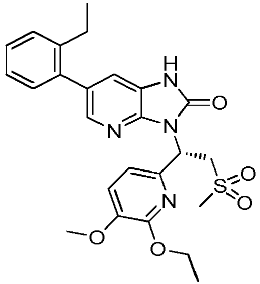

The compound was synthesized by referring to the method of Example 2.

¹HNMR (400 MHz, DMSO-D6) δ 11.28(s,1H), 7.82(d,1H), 7.38-7.18(m,7H), 6.90(d,1H), 6.07(q,1H), 4.61(t,1H), 4.35-4.22(m,1H), 4.20-4.13(m,2H), 3.76(t,3H), 3.08(q,3H), 1.21-1.16(m,4H), 1.05(t,3H).

ESI MS m/z: 497.18[M+1]⁺

### Example 82. (S)-3-(1-(6-ethoxy-5-methoxypyridin-2-yl)-2-(methylsulfonyl)ethy l)-6-(2-ethylphenyl)-1-methyl-1H-imidazo[4,5-b]pyridin-2(3H)-one

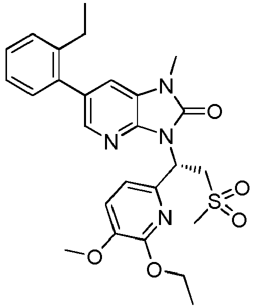

The compound was synthesized by referring to the method of Example 19.

¹HNMR (400 MHz, DMSO-D6) δ 7.87(d,1H), 7.57(d,1H), 7.36(m,2H), 7.29-7.27(m,2H), 7.23(q,1H), 6.94(d,1H), 6.11(q,1H), 4.63(q,1H), 4.34(q,1H), 4.17-4.11(m,2H), 3.75(s,3H), 3.39(s,3H), 3.07(s,3H), 2.60-2.50(m,2H), 1.17(t,3H), 1.05(t,3H).

ESI MS m/z: 511.19[M+1]⁺

### Example 83. (S)-3-(1-(6-ethoxy-5-methoxypyridin-2-yl)-2-(methylsulfonyl)ethy l)-6-(2-methoxyphenyl)-1H-imidazo[4,5-b]pyridin-2(3H)-one

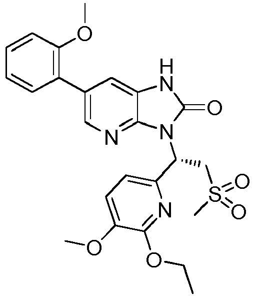

The compound was synthesized by referring to the method of Example 2.

¹H NMR (400 MHz, DMSO-D6) δ11.26(s,1H), 7.95(d,1H), 7.41(d,1H), 7.30-7.38(m,2H), 7.24(d,1H), 7.13(d,1H), 7.03(t,1H), 6.83(d,1H), 6.06(q,1H), 4.35-4.63(m,1H), 4.19-4.34(m,3H), 3.75(d,6H), 3.06(s,3H), 1.15-1.22(m,3H)

ESI MS m/z: 499.16 [M+1]⁺

### Example 84. (S)-3-(1-(6-ethoxy-5-methoxypyridin-2-yl)-2-(methylsulfonyl)ethy l)-6-(2-methoxyphenyl)-1-methyl-1H-imidazo[4,5-b[pyridin-2(3H)-one

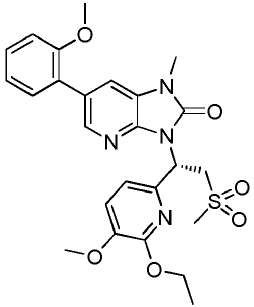

The compound was synthesized by referring to the method of Example 19.

¹H NMR (400 MHz, DMSO-D6) δ8.01(d,1H), 7.65(d,1H), 7.04-7.07 (m, 1H), 7.14-7.16(d,1H), 7.33--7.40(m,2H), 6.10 (dd,1H), 4.60-4.65(m,1H), 4.32-4.36(m,1H), 4.17-4.24(m,2H), 3.79(s,3H), 3.75(s,3H)3.40(s,3H), 3.,07(s,3H), 1.21(t,3H)..

ESI MS m/z: 513.17 [M+1]⁺

### Example 85. (S)-3-(1-(6-ethoxy-5-methoxypyridin-2-yl)-2-(methylsulfonyl)ethy l)-6-(2-(trifluoromethyl)phenyl)-1H-imidazo[4,5-b]pyridin-2(3H)-one

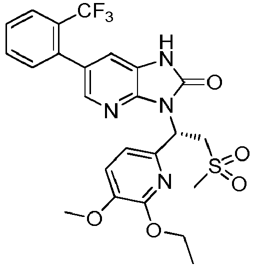

The compound was synthesized by referring to the method of Example 2.

¹HNMR (400 MHz, DMSO-D6) δ 11.33(s,1H), 7.84(q,2H), 7.74(t,1H), 7.64(t,1H), 7.47(d,1H), 7.27(t,2H), 6.93(d,1H), 6.07(q,1H), 4.61(q,1H), 4.32(q,1H), 4.17-4.08(m,2H), 3.75(s,3H), 3.07(s,3H), 1.17(t,3H)

ESI MS m/z: 537.13[M+1]⁺

### Example 86. (S)-3-(1-(6-ethoxy-5-methoxypyridin-2-yl)-2-(methylsulfonyl)ethy l)-6-(2-(trifluoromethyl)phenyl)-1-methyl-1H-imidazo[4,5-b]pyridin-2(3H)-one

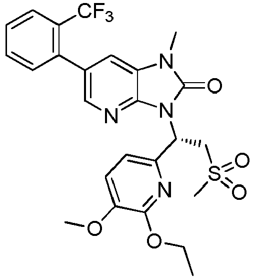

The compound was synthesized by referring to the method of Example 19.

¹HNMR (400 MHz, DMSO-D6) δ 7.88(d,2H), 7.76(t,1H), 7.66(t,1H), 7.59(s,1H), 7.49(d,1H), 7.27(d,1H), 6.96(d,1H), 6.12(q,1H), 4.63(q,1H), 4.35(q,1H), 4.12-4.06(m,2H), 3.75(s,3H), 3.30(s,3H), 3.07(s,3H), 1.15(t,3H).

ESI MS m/z: 551.15[M+1]⁺

### Example 87. (S)-6-(2-cyclopropylphenyl)-3-(1-(6-ethoxy-5-methoxypyridin-2-yl) -2-(methylsulfonyl)ethyl)-1H-imidazo[4,5-b]pyridin-2(3H)-one

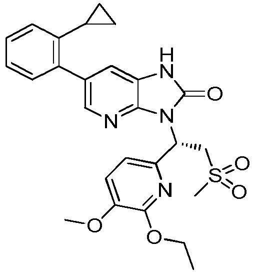

The compound was synthesized by referring to the method of Example 2.

ESI MS m/z: 509.18 [M+1]⁺

### Example 88. (S)-6-(2,6-difluorophenyl)-3-(1-(6-ethoxy-5-methoxypyridin-2-yl)-2-(methylsulfonyl)ethyl)-1H-imidazo[4,5-b]pyridin-2(3H)-one

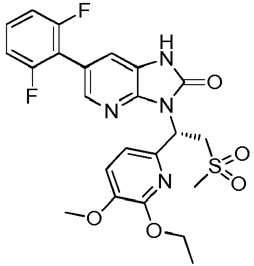

The compound was synthesized by referring to the method of Example 2.

ESI MS m/z: 505.13[M+1]⁺

### Example 89. (S)-6-(2,6-difluorophenyl)-3-(1-(6-ethoxy-5-methoxypyridin-2-yl)-2-(methylsulfonyl)ethyl)-1-methyl-1H-imidazo[4,5-b]pyridin-2(3H)-one

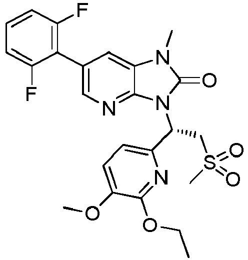

The compound was synthesized by referring to the method of Example 19.

ESI MS m/z: 519.14[M+1]⁺

### Example 90. (S)-6-(2,3-difluorophenyl)-3-(1-(6-ethoxy-5-methoxypyridin-2-yl)-2-(methylsulfonyl)ethyl)-1H-imidazo[4,5-b]pyridin-2(3H)-one

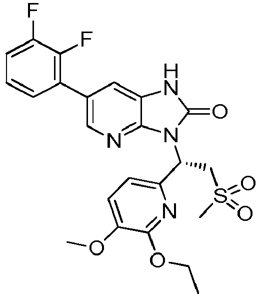

The compound was synthesized by referring to the method of Example 2.

¹HNMR (400 MHz, DMSO-D6) δ 11.45(s,1H), 8.09(s,1H), 7.52-7.38(m,3H), 7.32(t,1H), 7.25(d,1H), 6.87(d,1H), 6.08(q,1H), 4.59(q,1H), 4.34(q,1H), 4.20(q,2H), 3.75(s,3H), 3.08(s,3H), 1.24-1.18(m,3H).

ESI MS m/z: 505.13[M+1]⁺

### Example 91. (S)-6-(2,3-difluorophenyl)-3-(1-(6-ethoxy-5-methoxypyridin-2-yl)-2-(methylsulfonyl)ethyl)-1-methyl-1H-imidazo[4,5-b]pyridin-2(3H)-one

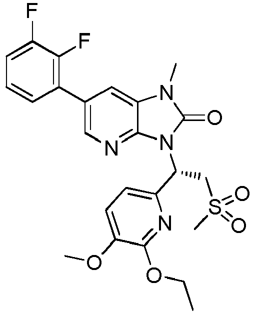

The compound was synthesized by referring to the method of Example 19.

¹HNMR (400 MHz, DMSO-D6) δ 8.15(s,1H), 7.82(s,1H), 7.52-7.41(m,2H), 7.35(t,1H), 7.24(d,1H), 6.90(d,1H), 6.12(q,1H), 4.59(q,1H), 4.35(q,1H), 4.21-4.15(m,2H), 3.74(s,3H), 3.43(s,3H), 3.07(s,3H), 1.19(t,3H).

ESI MS m/z: 519.14[M+1]⁺

### Example 92. (S)-6-(2,5-difluorophenyl)-3-(1-(6-ethoxy-5-methoxypyridin-2-yl)-2-(methylsulfonyl)ethyl)-1H-imidazo[4,5-b]pyridin-2(3H)-one

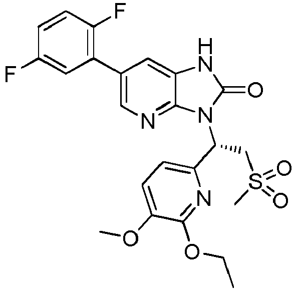

The compound was synthesized by referring to the method of Example 2.

¹HNMR (400 MHz, DMSO-D6) δ 11.44(s,1H), 8.10(s,1H), 7.52-7.47(m,2H), 7.43-7.37(m,1H), 7.29-7.23(m,2H), 6.86(d,1H), 6.07(q,1H), 4.58(t,1H), 4.36-4.23(m,1H), 4.21-4.18(m,2H), 3.75(s,3H), 3.07(s,3H), 1.21(t,3H).

ESI MS m/z: 505.13[M+1]⁺

### Example 93. (S)-6-(2,5-difluorophenyl)-3-(1-(6-ethoxy-5-methoxypyridin-2-yl)-2-(methylsulfonyl)ethyl)-1-methyl-1H-imidazo[4,5-b]pyridin-2(3H)-one

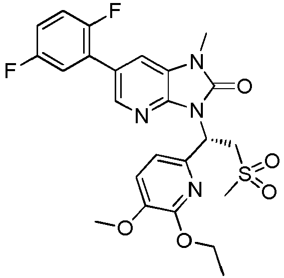

The compound was synthesized by referring to the method of Example 19.

¹HNMR (400 MHz, DMSO-D6) δ 8.15(s,1H), 7.81(s,1H), 7.54-7.50(m,1H), 7.49-7.39(m,1H), 7.32-7.23(m,2H), 6.89(d,1H), 6.11(q,1H), 4.58(t,1H), 4.35(q,1H), 4.22-4.15(m,2H), 3.74(s,3H), 3.42(s,3H), 3.07(s,3H), 1.19(t,3H).

ESI MS m/z: 519.14[M+1]⁺

### Example 94. (S)-6-(2,4-difluorophenyl)-3-(1-(6-ethoxy-5-methoxypyridin-2-yl)-2-(methylsulfonyl)ethyl)-1H-imidazo[4,5-b]pyridin-2(3H)-one

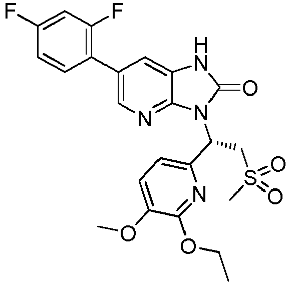

The compound was synthesized by referring to the method of Example 2.

ESI MS m/z: 505.13[M+1]⁺

### Example 95. (S)-6-(2,4-difluorophenyl)-3-(1-(6-ethoxy-5-methoxypyridin-2-yl)-2-(methylsulfonyl)ethyl)-1-methyl-1H-imidazo[4,5-b[pyridin-2(3H)-one

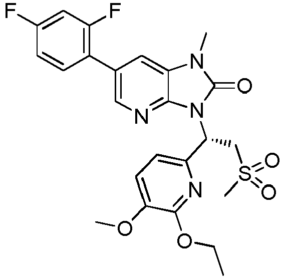

The compound was synthesized by referring to the method of Example 19.

ESI MS m/z: 519.14[M+1]⁺

### Example 96. (S)-6-(2,4,5-trifluorophenyl)-3-(1-(6-ethoxy-5-methoxypyridin-2-y l)-2-(methylsulfonyl)ethyl)-1H-imidazo[4,5-b]pyridin-2(3H)-one

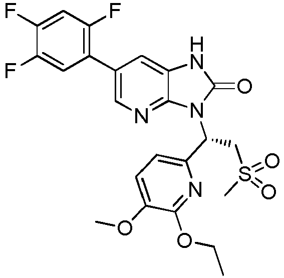

The compound was synthesized by referring to the method of Example 2.

¹H NMR (400 MHz, DMSO-D6)δ11.45(s,1H), 8.07(s,1H), 7.82-7.67(m,2H), 7.49(s,1H), 7.24(d,1H), 6.86(d,1H), 6.07(q,1H), 4.57(t,1H), 4.35-4.31(m,1H), 4.25-4.17(m,2H), 3.75(s,3H), 3.07(s,3H), 1.20(q,3H).

ESI MS m/z: 523.12[M+1]⁺

### Example 97. (S)-6-(2,4,5-trifluorophenyl)-3-(1-(6-ethoxy-5-methoxypyridin-2-y l)-2-(methylsulfonyl)ethyl)-1-methyl-1H-imidazo[4,5-b]pyridin-2(3H)-one

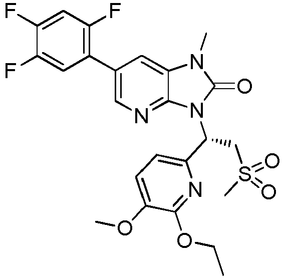

The compound was synthesized by referring to the method of Example 19.

¹H NMR (400 MHz, DMSO-D6)δ11.55(s,1H), 7.67-7.77(m,2H), 7.60(q,1H), 7.24(d,1H), 6.83(d,1H), 6.06(q,1H), 4.58(q,1H), 4.31(q,1H), 4.23(q,2H), 3.74(s,3H), 3.34(s,3H), 3.05(d,3H), 1.15-1.22(m,3H).

ESI MS m/z: 537.13[M+1]⁺

### Example 98. (R)-6-(2,5-difluorophenyl)-3-(1-(6-ethoxy-5-methoxypyridin-2-yl)-2-(methylsulfonyl)ethyl)-1H-imidazo[4,5-b]pyridin-2(3H)-one

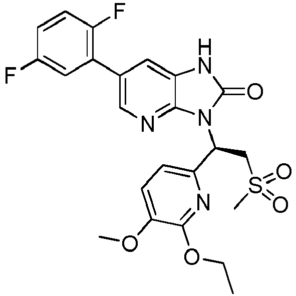

The compound was synthesized by referring to the method of Example 2.

ESI MS m/z: 505.13[M+1]⁺

### Example 99. (S)-6-(2,4-dimethylphenyl)-3-(1-(6-ethoxy-5-methoxypyridin-2-yl) -2-(methylsulfonyl)ethyl)-1H-imidazo[4,5-b]pyridin-2(3H)-one

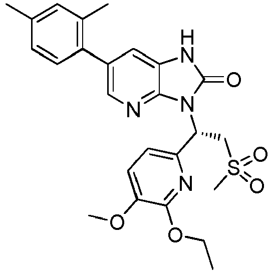

The compound was synthesized by referring to the method of Example 2.

¹HNMR (400 MHz, DMSO-D6) δ 11.27(s,1H), 7.82(d,1H), 7.28(t,3H), 7.09(m,3H), 6.88(d,1H), 6.07(q,1H), 4.62(q,1H), 4.35(q,1H), 4.21(q,1H), 3.75(s,3H), 3.06(s,3H), 2.31(s,3H), 2.20(s,3H), 1.19(t,3H)

ESI MS m/z: 497.18[M+1]⁺

### Example 100. (S)-6-(2,4-dimethylphenyl)-3-(1-(6-ethoxy-5-methoxypyridin-2-y l)-2-(methylsulfonyl)ethyl)-1-methyl-1H-imidazo[4,5-b]pyridin-2(3H)-one

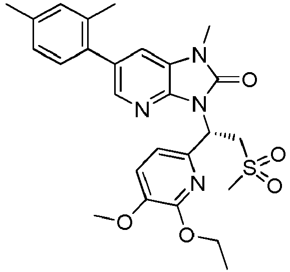

The compound was synthesized by referring to the method of Example 19.

¹HNMR (400 MHz, DMSO-D6) δ 7.87(d,1H), 7.57(d,1H), 7.25(d,1H), 7.10(q,3H), 6.91(d,1H), 6.10(q,1H), 4.62(q,1H), 4.33(q,1H), 4.19-4.13(m,2H), 3.74(s,3H), 3.40(s,3H), 3.06(s,3H), 2.32(s,3H), 2.20(d,3H), 1.18(t,3H).

ESI MS m/z: 511.19[M+1]⁺

### Example 101. (S)-6-(2,5-dimethylphenyl)-3-(1-(6-ethoxy-5-methoxypyridin-2-y l)-2-(methylsulfonyl)ethyl)-1H-imidazo[4,5-b]pyridin-2(3H)-one

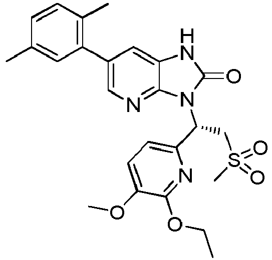

The compound was synthesized by referring to the method of Example 2.

¹HNMR (400 MHz, DMSO-D6) δ 11.29(s,1H), 7.84(d,1H), 7.26(t,2H), 7.19(d,1H), 7.10(d,1H), 7.05(s,1H), 6.88(d,1H), 6.07(q,1H), 4.62(q,1H), 4.32(q,1H), 4.23-4.16(m,2H), 3.75(s,3H), 3.07(s,3H), 2.29(s,3H), 2.18(s,3H), 1.20(t,3H).

ESI MS m/z: 497.18[M+1]⁺

### Example 102. (S)-6-(2,5-dimethylphenyl)-3-(1-(6-ethoxy-5-methoxypyridin-2-y l)-2-(methylsulfonyl)ethyl)-1-methyl-1H-imidazo[4,5-b]pyridin-2(3H)-one

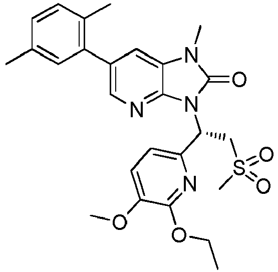

The compound was synthesized by referring to the method of Example 19.

¹HNMR (400 MHz, DMSO-D6) δ 7.88(d,1H), 7.59(d,1H), 7.23(q,2H), 7.10(t,2H), 6.91(d,1H), 6.10(q,1H), 4.62(q,1H), 4.32(q,1H), 4.19-4.13(m,2H), 3.75(s,3H), 3.40(s,3H), 3.06(s,3H), 2.31(s,3H), 2.20(s,3H), 1.18(t,3H).

ESI MS m/z: 511.19[M+1

### Example 103. (S)-6-(2,3-dimethylphenyl)-3-(1-(6-ethoxy-5-methoxypyridin-2-y l)-2-(methylsulfonyl)ethyl)-1H-imidazo[4,5-b]pyridin-2(3H)-one

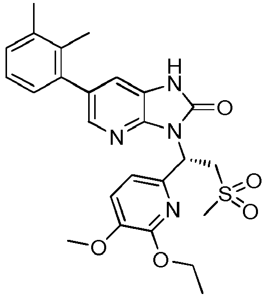

The compound was synthesized by referring to the method of Example 2.

¹HNMR (400 MHz, DMSO-D6) δ 11.29(s,1H), 7.81(d,1H), 7.27-7.13(m,4H), 7.05(d,1H), 6.89(d,1H), 6.07(q,1H), 4.62(q,1H), 4.32(q,1H), 4.22-4.16(m,2H), 3.75(s,3H), 3.07(s,3H), 2.30(s,3H), 2.11(s,3H), 1.20(t,3H).

ESI MS m/z: 497.18[M+1]⁺

### Example 104. (S)-6-(2,3-dimethylphenyl)-3-(1-(6-ethoxy-5-methoxypyridin-2-y l)-2-(methylsulfonyl)ethyl)-1-methyl-1H-imidazo[4,5-b]pyridin-2(3H)-one

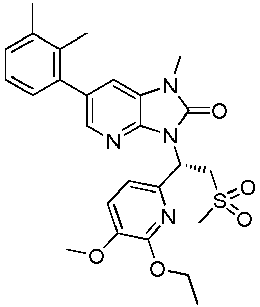

The compound was synthesized by referring to the method of Example 19.

¹HNMR (400 MHz, DMSO-D6) δ 7.85(d,1H), 7.55(d,1H), 7.26-7.14(m,3H), 7.08(d,1H), 6.92(d,1H), 6.11(q,1H), 4.63(q,1H), 4.34(q,1H), 4.19-4.12(m,2H), 3.75(s,3H), 3.39(s,3H), 3.07(s,3H), 2.31(s,3H), 2.12(s,3H), 1.18(t,3H).

ESI MS m/z: 511.19[M+1]⁺

### Example 105. (S)-6-(2,6-dimethylphenyl)-3-(1-(6-ethoxy-5-methoxypyridin-2-y l)-2-(methylsulfonyl)ethyl)-1H-imidazo[4,5-b]pyridin-2(3H)-one

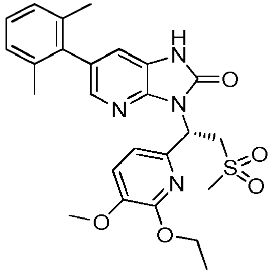

The compound was synthesized by referring to the method of Example 2.

ESI MS m/z: 497.18[M+1]⁺

### Example 106. (S)-6-(2,6-dimethylphenyl)-3-(1-(6-ethoxy-5-methoxypyridin-2-y l)-2-(methylsulfonyl)ethyl)-1-methyl-1H-imidazo[4,5-b]pyridin-2(3H)-one

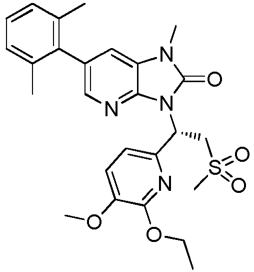

The compound was synthesized by referring to the method of Example 19.

ESI MS m/z: 511.19[M+1]⁺

### Example 107. (S)-6-(4-fluoro-2-methylphenyl)-3-(1-(6-ethoxy-5-methoxypyridi n-2-yl)-2-(methylsulfonyl)ethyl)-1H-imidazo[4,5-b]pyridin-2(3H)-one

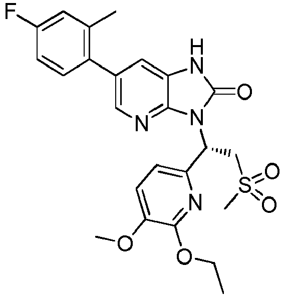

The compound was synthesized by referring to the method of Example 2.

¹H NMR (400 MHz, DMSO-D6) δ11.32(s,1H), 7.84(d,1H), 7.28-7.21(m, 3H), 7.18(t,1H), 7.12-7.07(m,1H), 6.89(d,1H), 6.07(q,1H), 4.61(q,1H), 4.35-4.22(m,1H), 4.21-4.16(m,2H), 3.75(s,3H), 3.07(s,3H), 2.24(s,3H), 1.19(t,3H).

ESI MS m/z: 501.15[M+1]⁺

### Example 108. (S)-6-(4-fluoro-2-methylphenyl)-3-(1-(6-ethoxy-5-methoxypyridi n-2-yl)-2-(methylsulfonyl)ethyl)-1-methyl-1H-imidazo[4,5-b]pyridin-2(3H)-one

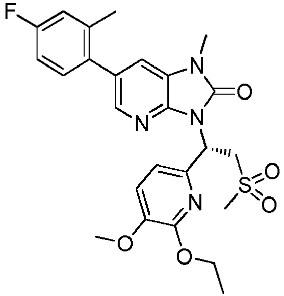

The compound was synthesized by referring to the method of Example 19.

¹H NMR (400 MHz, DMSO-D6) δ7.89(d,1H), 7.60(d,1H), 7.31-7.19(m,3H), 7.13 (q,1H), 6.92(d,1H), 6.11(q,1H), 4.61(q,1H), 4.34(q,1H), 4.17-4.13(m,2H), 3.74(s,3H), 3.40(s,3H), 3.07(s,3H), 2.26(s,3H), 1.17(t,3H).

ESI MS m/z: 515.17[M+1]⁺

### Example 109. (S)-6-(5-fluoro-2-methylphenyl)-3-(1-(6-ethoxy-5-methoxypyridi n-2-yl)-2-(methylsulfonyl)ethyl)-1H-imidazo[4,5-b]pyridin-2(3H)-one

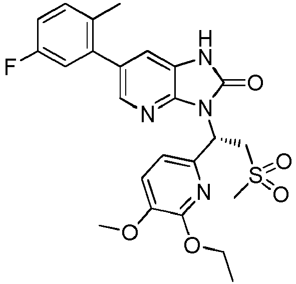

The compound was synthesized by referring to the method of Example 2.

ESI MS m/z: 501.15[M+1]⁺

### Example 110. (S)-6-(5-fluoro-2-methylphenyl)-3-(1-(6-ethoxy-5-methoxypyridi n-2-yl)-2-(methylsulfonyl)ethyl)-1-methyl-1H-imidazo[4,5-b]pyridin-2(3H)-one

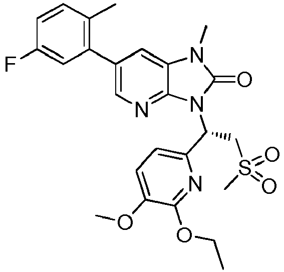

The compound was synthesized by referring to the method of Example 19.

ESI MS m/z: 515.17[M+1]⁺

### Example 111. (S)-6-(3-fluoro-2-methylphenyl)-3-(1-(6-ethoxy-5-methoxypyridi n-2-yl)-2-(methylsulfonyl)ethyl)-1H-imidazo[4,5-b]pyridin-2(3H)-one

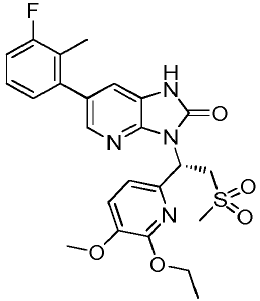

The compound was synthesized by referring to the method of Example 2.

ESI MS m/z: 501.15[M+1]⁺

### Example 112. (S)-6-(3-fluoro-2-methylphenyl)-3-(1-(6-ethoxy-5-methoxypyridi n-2-yl)-2-(methylsulfonyl)ethyl)-1-methyl-1H-imidazo[4,5-b]pyridin-2(3H)-one

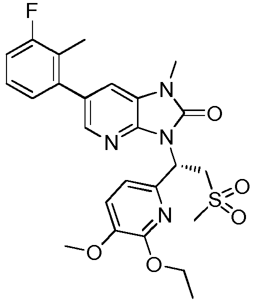

The compound was synthesized by referring to the method of Example 19.

ESI MS m/z: 515.17[M+1]⁺

### Example 113. (S)-6-(2-fluoro-5-methylphenyl)-3-(1-(6-ethoxy-5-methoxypyridi n-2-yl)-2-(methylsulfonyl)ethyl)-1H-imidazo[4,5-b]pyridin-2(3H)-one

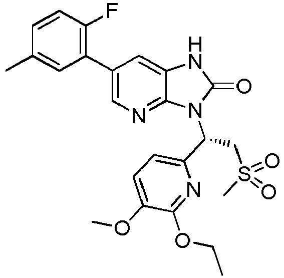

The compound was synthesized by referring to the method of Example 2.

ESI MS m/z: 501.15[M+1]⁺

### Example 114. (S)-6-(2-fluoro-5-methylphenyl)-3-(1-(6-ethoxy-5-methoxypyridi n-2-yl)-2-(methylsulfonyl)ethyl)-1-methyl-1H-imidazo[4,5-b]pyridin-2(3H)-one

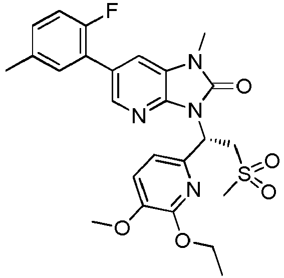

The compound was synthesized by referring to the method of Example 19.

ESI MS m/z: 515.17[M+1]⁺

### Example 115. (S)-6-(2-fluoro-4-methylphenyl)-3-(1-(6-ethoxy-5-methoxypyridi n-2-yl)-2-(methylsulfonyl)ethyl)-1H-imidazo[4,5-b]pyridin-2(3H)-one

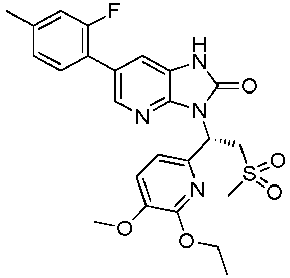

The compound was synthesized by referring to the method of Example 2.

ESI MS m/z: 501.15[M+1]⁺

### Example 116. (S)-6-(2-fluoro-4-methylphenyl)-3-(1-(6-ethoxy-5-methoxypyridi n-2-yl)-2-(methylsulfonyl)ethyl)-1-methyl-1H-imidazo[4,5-b]pyridin-2(3H)-one

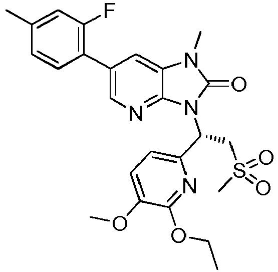

The compound was synthesized by referring to the method of Example 19.

ESI MS m/z: 515.17[M+1]⁺

### Example 117. (S)-6-(5-fluoro-2-methoxyphenyl)-3-(1-(6-ethoxy-5-methoxypyri din-2-yl)-2-(methylsulfonyl)ethyl)-1H-imidazo[4,5-b]pyridin-2(3H)-one

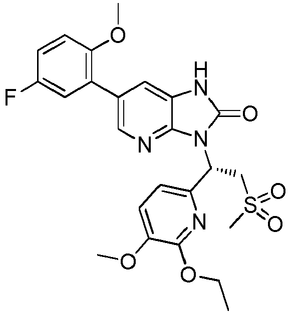

The compound was synthesized by referring to the method of Example 2.

ESI MS m/z: 517.15[M+1]⁺

### Example 118. (S)-6-(5-fluoro-2-methoxyphenyl)-3-(1-(6-ethoxy-5-methoxypyri din-2-yl)-2-(methylsulfonyl)ethyl)-1-methyl-1H-imidazo[4,5-b]pyridin-2(3H)-one

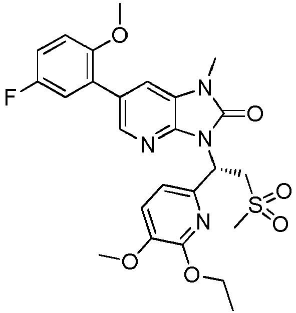

The compound was synthesized by referring to the method of Example 19.

ESI MS m/z: 531.16[M+1]⁺

### Example 119. (S)-6-(4-fluoro-2-methoxyphenyl)-3-(1-(6-ethoxy-5-methoxypyri din-2-yl)-2-(methylsulfonyl)ethyl)-1H-imidazo[4,5-b]pyridin-2(3H)-one

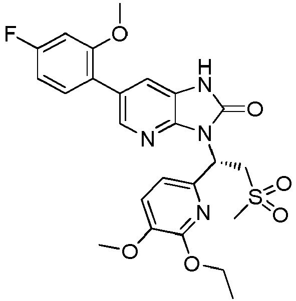

The compound was synthesized by referring to the method of Example 2.

ESI MS m/z: 517.15[M+1]⁺

### Example 120. (S)-6-(4-fluoro-2-methoxyphenyl)-3-(1-(6-ethoxy-5-methoxypyri din-2-yl)-2-(methylsulfonyl)ethyl)-1-methyl-1H-imidazo[4,5-b]pyridin-2(3H)-one

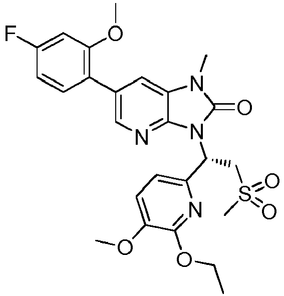

The compound was synthesized by referring to the method of Example 19.

ESI MS m/z: 531.16[M+1]⁺

### Example 121. (S)-6-(6-fluoro-2-methoxyphenyl)-3-(1-(6-ethoxy-5-methoxypyri din-2-yl)-2-(methylsulfonyl)ethyl)-1H-imidazo[4,5-b]pyridin-2(3H)-one

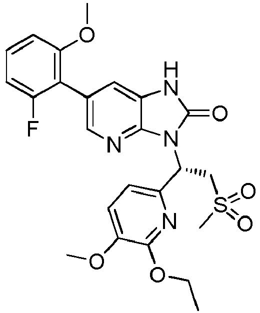

The compound was synthesized by referring to the method of Example 2.

ESI MS m/z: 517.15[M+1]⁺

### Example 122. (S)-6-(pyridin-2-yl)-3-(1-(6-ethoxy-5-methoxypyridin-2-yl)-2-(me thylsulfonyl)ethyl)-1H-imidazo[4,5-b]pyridin-2(3H)-one

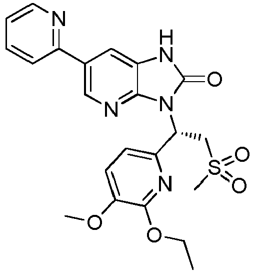

The compound was synthesized by referring to the method of Example 2.

ESI MS m/z: 470.14[M+1]⁺

### Example 123. (S)-3-(1-(6-ethoxy-5-methoxypyridin-2-yl)-2-(methylsulfonyl)eth yl)-1-methyl-6-(pyridin-2-yl)-1H-imidazo[4,5-b]pyridin-2(3H)-one

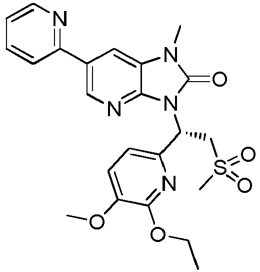

The compound was synthesized by referring to the method of Example 19.

ESI MS m/z: 484.16[M+1]⁺

### Example 124. (S)-3-(1-(6-ethoxy-5-methoxypyridin-2-yl)-2-(methylsulfonyl)eth yl)-1-ethyl-6-(pyridin-2-yl)-1H-imidazo[4,5-b]pyridin-2(3H)-one

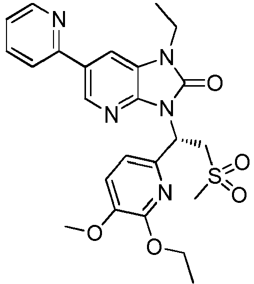

The compound was synthesized by referring to the method of Example 19.

ESI MS m/z: 498.17[M+1]⁺

### Example 125. (S)-3-(1-(6-ethoxy-5-methoxypyridin-2-yl)-2-(methylsulfonyl)eth yl)-1-(2-methoxyethyl)-6-(pyridin-2-yl)-1H-imidazo[4,5-b]pyridin-2(3H)-one

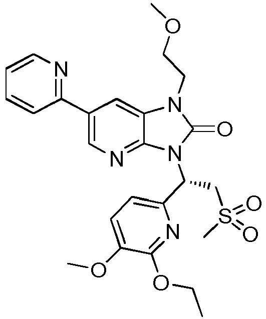

The compound was synthesized by referring to the method of Example 19.

ESI MS m/z: 528.18 [M+1]⁺

### Example 126. (S)-3-(1-(6-ethoxy-5-methoxypyridin-2-yl)-2-(methylsulfonyl)eth yl)-6-(2-fluorophenyl)-1-methyl-1H-imidazo[4,5-b]pyridin-2(3H)-one

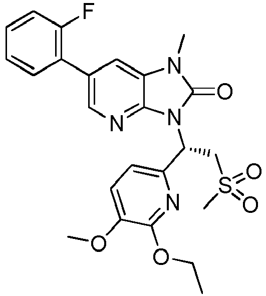

The compound was synthesized by referring to the method of Example 19.

¹H NMR (400 MHz, DMSO-D6) δ8.11(m,1H), 7.77(t,1H), 7.58-7.62 (m,1H), 7.43-7.47(m,1H), 7.32--7.38(m,2H), 6.12 (dd, 1H), 4.58-4.64(m,1H), 4.16-4.22(m, 2H), 4.32-437(m,1H), 3.74(s,3H), 3.43(s,3H), 2.96(s,3H), 1.21(t,3H)

ESI MS m/z: 501.15[M+1]⁺

### Example 127. (S)-3-(1-(6-ethoxy-5-methoxypyridin-2-yl)-2-(methylsulfonyl)eth yl)-1-methyl-6-phenyl-1H-imidazo[4,5-b]pyridin-2(3H)-one

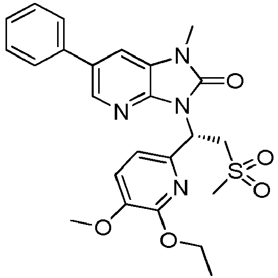

The compound was synthesized by referring to the method of Example 19.

ESI MS m/z: 496.18 [M+1]⁺

### Example 128. (S)-3-(1-(6-ethoxy-5-methoxypyridin-2-yl)-2-(methylsulfonyl)eth yl)-6-(4-fluorophenyl)-1-methyl-1H-imidazo[4,5-b]pyridin-2(3H)-one

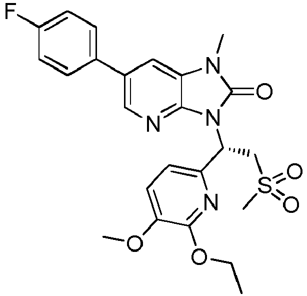

The compound was synthesized by referring to the method of Example 19.

ESI MS m/z: 501.16[M+1]⁺

### Example 129. (S)-5-bromo-1-(1-(6-ethoxy-5-methoxypyridin-2-yl)-2-(methylsul fonyl)ethyl)-1H-benzo[d]imidazol-2(3H)-one

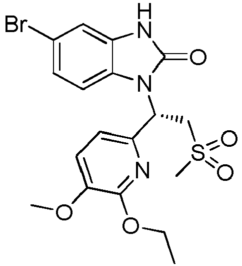

The compound was synthesized by referring to the method of Example 2.

ESI MS m/z: 470.03[M+1]⁺

### Example 130. (S)-1-(1-(1-(6-ethoxy-5-methoxypyridin-2-yl)-2-(methylsulfonyl) ethyl)-5-phenyl-1H-benzo[d]imidazol-2(3H)-one

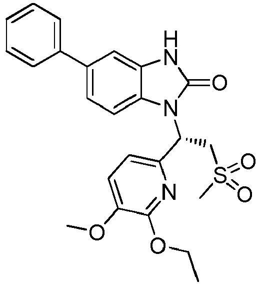

The compound was synthesized by referring to the method of Example 2.

¹H NMR (400 MHz, DMSO-D6)δ11.11(s,1H), 7.45-7.41(t,2H), 7.33-7.31(d,2H), 7.27-7.20(q,5H), 6.84-6.82(d,1H), 6.06-6.03(q,1H), 4.40-4.35(m,3H), 4.28-4.24(t,1H), 3.75(s,6H), 3.04(s,3H), 1.33-1.30(q,3H).

ESI MS m/z: 468.15 [M+1]⁺

### Example 131. (S)-1-(1-(1-(6-ethoxy-5-methoxypyridin-2-yl)-2-(methylsulfonyl) ethyl)-5-phenyl-3-methyl-1H-benzo[d]imidazol-2(3H)-one

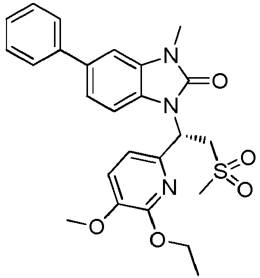

The compound was synthesized by referring to the method of Example 19.

¹HNMR (400 MHz, DMSO-D6) δ 7.68-7.66(d,2H), 7.48-7.43(q,3H), 7.34-7.24(m,4H), 6.86-6.84(d,1H), 6.09-6.06(q,1H), 4.40-4.30(q,3H), 4.28-4.24(t,1H), 3.74(s,3H), 3.41(s,3H), 3.04(s,3H), 1.32-1.29(t,3H).

ESI MS m/z: 482.17 [M+1]⁺

### Example 132. (S)-1-(1-(1-(6-ethoxy-5-methoxypyridin-2-yl)-2-(methylsulfonyl) ethyl)-5-(2-fluorophenyl)-1H-benzo[d]imidazol-2(3H)-one

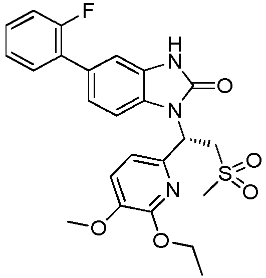

The compound was synthesized by referring to the method of Example 2.

¹H NMR (400 MHz, DMSO-D6) δ11.12(s,1H), 7.47-7.51(m,1H), 7.28-7.38 (m,1H), 7.24-7.27(m,4H), 7.13(t,2H), 6.85(d,1H), 6.05(q,1H), 4.33-4.41(m,3H), 4.22-4.28(m,1H), 3.74(s,3H), 3.03(d,3H), 1.29(t,3H)

ESI MS m/z: 486.14 [M+1]⁺

### Example 133. (S)-1-(1-(1-(6-ethoxy-5-methoxypyridin-2-yl)-2-(methylsulfonyl) ethyl)-5-(2-fluorophenyl)-3-methyl-1H-benzo[d]imidazol-2(3H)-one

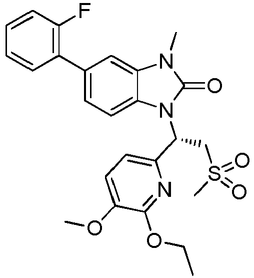

The compound was synthesized by referring to the method of Example 19.

¹H NMR (400 MHz, DMSO-D6) δ7.40-7.54(m,1H), 7.34-7.39(m,1H), 7.26-7.32(m,5H), 7.25(d,1H), 6.85(d,1H), 6.08(q,1H), 4.25-4.43(m,4H), 3.74(s,3H), 3.38(s,3H), 3.05(s,3H), 1.27(q,3H).

ESI MS m/z: 486.14 [M+1]⁺

### Example 134. (R)-1-(1-(1-(6-ethoxy-5-methoxypyridin-2-yl)-2-(methylsulfonyl) ethyl)-5-(2-fluorophenyl)-1H-benzo[d]imidazol-2(3H)-one

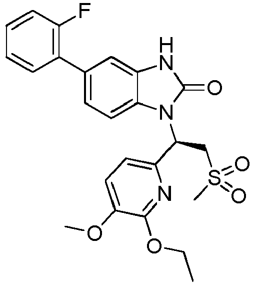

The compound was synthesized by referring to the method of Example 2.

ESI MS m/z: 486.14 [M+1]⁺

### Example 135. (S)-1-(1-(1-(6-ethoxy-5-methoxypyridin-2-yl)-2-(methylsulfonyl) ethyl)-5-(4-fluorophenyl)-1H-benzo[d]imidazol-2(3H)-one

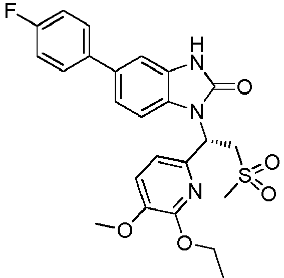

The compound was synthesized by referring to the method of Example 2.

ESI MS m/z: 486.14 [M+1]⁺

### Example 136. (R)-1-(1-(1-(6-ethoxy-5-methoxypyridin-2-yl)-2-(methylsulfonyl) ethyl)-5-(4-fluorophenyl)-1H-benzo[d]imidazol-2(3H)-one

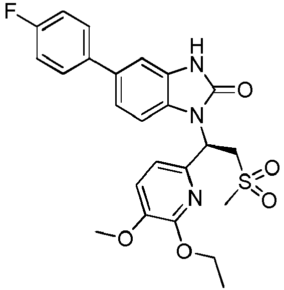

The compound was synthesized by referring to the method of Example 2.

ESI MS m/z: 486.14 [M+1]⁺

### Example 137. (S)-1-(1-(1-(6-ethoxy-5-methoxypyridin-2-yl)-2-(methylsulfonyl) ethyl)-3-methyl-5-(4-fluorophenyl)-1H-benzo[d]imidazol-2(3H)-one

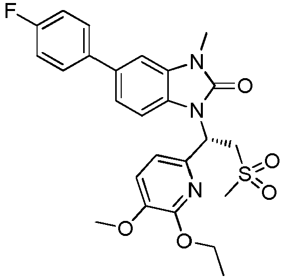

The compound was synthesized by referring to the method of Example 19.

ESI MS m/z: 500.16[M+1]⁺

### Example 138. (R)-1-(1-(1-(6-ethoxy-5-methoxypyridin-2-yl)-2-(methylsulfonyl) ethyl)-3-methyl-5-(4-fluorophenyl)-1H-benzo[d]imidazol-2(3H)-one

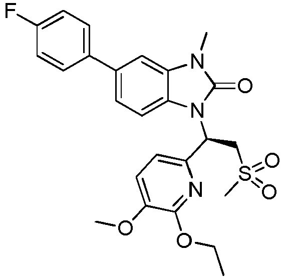

The compound was synthesized by referring to the method of Example 19.

ESI MS m/z: 500.16[M+1]⁺

### Example 139. (S)-1-(1-(6-ethoxy-5-methoxypyridin-2-yl)-2-(methylsulfonyl)eth yl)-5-(o-methylphenyl)-1H-benzo[d]imidazol-2(3H)-one

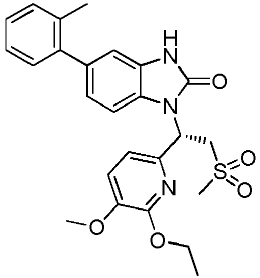

The compound was synthesized by referring to the method of Example 2.

¹H NMR (400 MHz, DMSO-D6) δ11.04(s,1H), 7.15-7.19(m,6H), 6.86-6.90(m,3H), 6.04(q,1H), 4.35(q,4H), 4.00(q,3H), 3.05(s,3H), 2.21(s,3H), 1.29(t,3H)

ESI MS m/z: 482.17 [M+1]⁺

### Example 140. (S)-1-(1-(6-ethoxy-5-methoxypyridin-2-yl)-2-(methylsulfonyl)eth yl)-5-(o-methylphenyl)-3-methyl-1H-benzo[d]imidazol-2(3H)-one

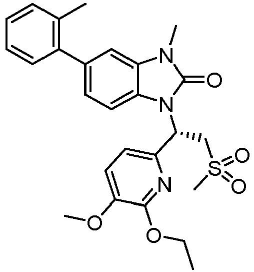

The compound was synthesized by referring to the method of Example 19.

¹H NMR (400 MHz, DMSO-D6)δ7.15-7.30(m,6H), 7.88-6.97(m.,1H), 7.16(t.,1H), 6.90(d.,1H), 6.07(q.,1H), 4.28-4.44(m.,4H), 3.75(s,3H), 3.36(s,3H), 3.34(s,3H), 2.50(t,3H), 2.45(t,3H).

ESI MS m/z: 496.18 [M+1]⁺

### Example 141. (R)-1-(1-(6-ethoxy-5-methoxypyridin-2-yl)-2-(methylsulfonyl)eth yl)-5-(o-methylphenyl)-1H-benzo[d]imidazol-2(3H)-one

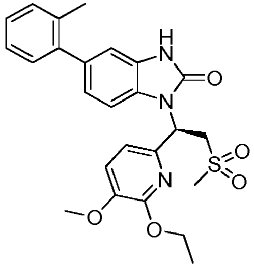

The compound was synthesized by referring to the method of Example 2.

ESI MS m/z: 482.17 [M+1]⁺

### Example 142. (S)-5-(2-chlorophenyl)-1-(1-(6-ethoxy-5-methoxypyridin-2-yl)-2-(methylsulfonyl)ethyl)-1H-benzo[d]imidazol-2(3H)-one

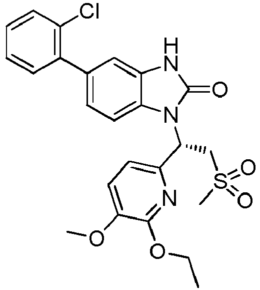

The compound was synthesized by referring to the method of Example 2.

ESI MS m/z: 502.11[M+1]⁺

### Example 143. (S)-1-(1-(1-(6-ethoxy-5-methoxypyridin-2-yl)-2-(methylsulfonyl) ethyl)-5-(2-methoxyphenyl)-1H-benzo[d]imidazol-2(3H)-one

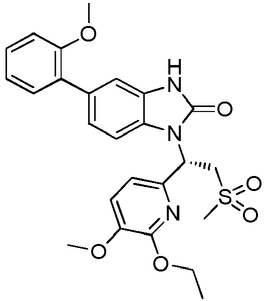

The compound was synthesized by referring to the method of Example 2.

¹H NMR (400 MHz, DMSO-D6) δ11.00(s,1H), 7.23-7.32(m,3H), 7.14 (d,1H), 7.08(t,2H), 6.97-7.02(m,2H), 6.83(d,1H), 6.03(q,1H), 4.35-4.42(m,3H), 4.21-4.27(m,1H), 3.74(d,6H), 3.05(s,3H), 1.32(t,3H).

ESI MS m/z: 498.16[M+1]⁺

### Example 144. (S)-1-(1-(1-(6-ethoxy-5-methoxypyridin-2-yl)-2-(methylsulfonyl) ethyl)-5-(2-methoxyphenyl)-3-methyl-1H-benzo[d]imidazol-2(3H)-one

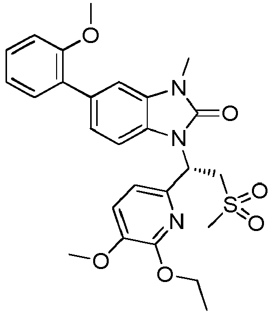

The compound was synthesized by referring to the method of Example 19.

¹H NMR (400 MHz, DMSO-D6) δ7.21-7.34(m,5H), 7.07-7.10(m,2H), 7.02(t,1H), 6.84(d,1H), 6.07(q,1H), 4.24-4.43(m,4H), 3.74(d,6H), 3.33(t,3H), 3.05(s,3H), 1.29(q,3H)

ESI MS m/z: 512.18[M+1]⁺

### Example 145. (R)-1-(1-(1-(6-ethoxy-5-methoxypyridin-2-yl)-2-(methylsulfonyl) ethyl)-5-(2-methoxyphenyl)-1H-benzo[d]imidazol-2(3H)-one

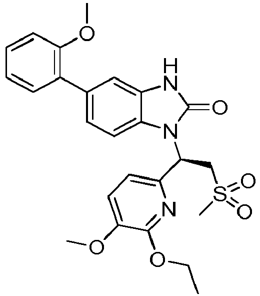

The compound was synthesized by referring to the method of Example 2.

ESI MS m/z: 498.16[M+1]⁺

### Example 146. (S)-1-(1-(1-(6-ethoxy-5-methoxypyridin-2-yl)-2-(methylsulfonyl) ethyl)-5-(2-(trifluoromethyl)phenyl)-1H-benzo[d]imidazol-2(3H)-one

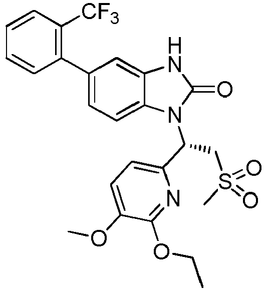

The compound was synthesized by referring to the method of Example 2.

¹H NMR (400 MHz,DMSO-D6)δ11.08(s,1H), 7.82-7.80(d,1H), 7.69-7.67 (d,1H), 7.60-7.58(d,1H), 7.41-7.39(d,1H), 7.29-7.24(q.2H), 6.91-6.87(q,2H), 6.05-6.02(q,1H), 4.38-4.27(m,5H), 3.77-3.74(t,3H), 3.05(s,3H), 1.28-1.25(t,3H).

ESI MS m/z: 536.14 [M+1]⁺

### Example 147. (S)-1-(1-(1-(6-ethoxy-5-methoxypyridin-2-yl)-2-(methylsulfonyl) ethyl)-5-(2-(trifluoromethyl)phenyl)-3-methyl-1H-benzo[d]imidazol-2(3H)-one

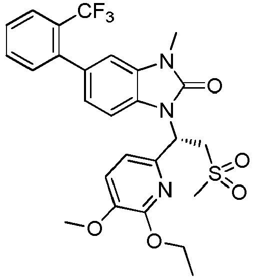

The compound was synthesized by referring to the method of Example 19.

¹HNM(400MHz,DMSO-D6)7.84-7.82(d,1H), 7.73-7.59(m,2H), 7.44-7.42(d,1H), 7.33-7.27(q,2H), 7.15(s,1H), 6.96-6.91(t,2H), 6.09-6.06(q,1H), 4.45-4.37(m,1H), 4.34-4.24(m,3H), 3.78-3.74(t,3H), 3.35(s,3H), 3.05(s,3H), 1.27-1.23(t,3H).

ESI MS m/z: 550.15 [M+1]⁺

### Example 148. (S)-5-(2-cyclopropylphenyl)-1-(1-(6-ethoxy-5-methoxypyridin-2-yl)-2-(methylsulfonyl)ethyl)-1H-benzo[d]imidazol-2(3H)-one

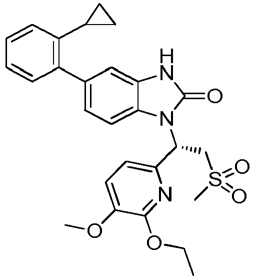

The compound was synthesized by referring to the method of Example 2.

ESI MS m/z: 508.18 [M+1]⁺

### Example 149. (S)-1-(1-(1-(6-ethoxy-5-methoxypyridin-2-yl)-2-(methylsulfonyl) ethyl)-5-(2-isopropylphenyl)-4-methyl-1H-benzo[d]imidazol-2(3H)-one

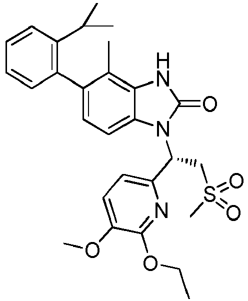

The compound was synthesized by referring to the method of Example 2.

ESI MS m/z: 524.21[M+1]⁺

### Example 150. (S)-1-(1-(6-ethoxy-5-methoxypyridin-2-yl)-2-(methylsulfonyl)eth yl)-5-(2-ethylphenyl)-1H-benzo[d]imidazol-2(3H)-one

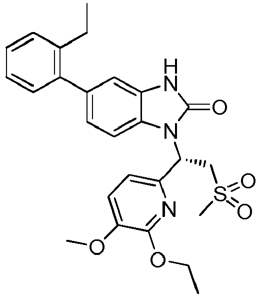

The compound was synthesized by referring to the method of Example 2.

¹H NMR (400 MHz, DMSO-D6) δ11.03(s,1H), 7.28(q,3H), 7.21(d,2H), 7.13(d,2H), 6.86(t,3H), 4.33-4.42(m,4H), 3.75(s,3H), 3.05(s,3H), 2.53(q,2H), 1.27(q,3H), 1.03(t,3H).

ESI MS m/z: 496.18[M+1]⁺

### Example 151. (S)-1-(1-(6-ethoxy-5-methoxypyridin-2-yl)-2-(methylsulfonyl)eth yl)-5-(2-ethylphenyl)-3-methyl-1H-benzo[d]imidazol-2(3H)-one

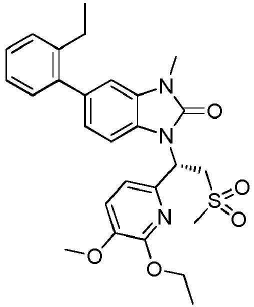

The compound was synthesized by referring to the method of Example 19.

¹H NMR (400 MHz, DMSO-D6)δ7.11-7.34(m,7H), 6.91(t,2H), 6.07(q,1H), 4.29-4.44(m,4H), 3.75(s,3H), 3.34(d,3H), 3.05(s,3H), 2.50-2.58(m,2H), 1.25(q,3H), 1.03(t,3H).

ESI MS m/z: 510.20[M+1]⁺

### Example 152. (S)-5-(2,3-difluorophenyl)-1-(1-(6-ethoxy-5-methoxypyridin-2-yl) -2-(methylsulfonyl)ethyl)-1H-benzo[d]imidazol-2(3H)-one

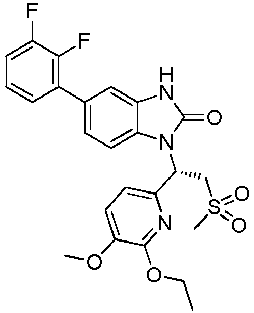

The compound was synthesized by referring to the method of Example 2.

¹H NMR (400 MHz, DMSO-D6) δ11.18(s,1H), 7.43-7.34(m,1H), 7.32-7.24(m,4H), 7.16-7.13(q,2H), 6.86-6.84(t,1H), 6.07-6.03(q,1H), 4.38-4.33(m,3H), 4.29-4.23(q,1H), 3.75(s,3H), 3.06(s,3H), 1.32-1.29(t,3H).

ESI MS m/z: 504.13[M+1]⁺

### Example 153. (S)-5-(2,3-difluorophenyl)-1-(1-(6-ethoxy-5-methoxypyridin-2-yl) -2-(methylsulfonyl)ethyl)-3-methyl-1H-benzo[d]imidazol-2(3H)-one

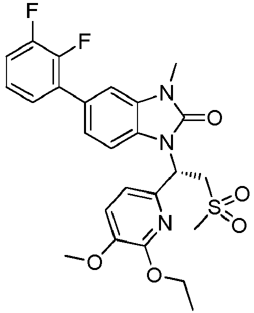

The compound was synthesized by referring to the method of Example 19.

¹HNMR (400 MHz, DMSO-D6) δ 7.43-7.20(m,7H), 6.88-6.86(d,1H), 6.11-6.07(q,1H), 4.42-4.26(m,4H), 3.75(s,3H), 3.40(s,3H), 3.06(s,3H), 1.31-1.28(t,3H).

ESI MS m/z: 518.15[M+1]⁺

### Example 154. (S)-5-(2,6-difluorophenyl)-1-(1-(6-ethoxy-5-methoxypyridin-2-yl) -2-(methylsulfonyl)ethyl)-1H-benzo[d]imidazol-2(3H)-one

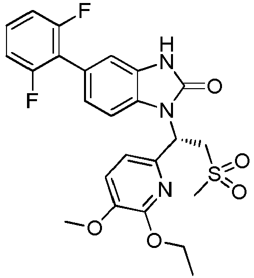

The compound was synthesized by referring to the method of Example 2.

¹H NMR (400 MHz, DMSO-D6) δ11.14-11.00(d,1H), 7.28-7.20(m,2H), 7.13-7.03(t,1H), 6.99-6.90(m,3H), 6.81-6.79(d,1H), 6.03-5.99(q,1H), 4.42-4.28(m,4H), 4.26-4.13(m,1H), 3.74(s,3H), 3.12-3.07(d,1H), 3.02(s,3H), 1.31-1.28(t,3H).

ESI MS m/z: 504.13[M+1]⁺

### Example 155. (S)-5-(2,6-difluorophenyl)-1-(1-(6-ethoxy-5-methoxypyridin-2-yl) -2-(methylsulfonyl)ethyl)-3-methyl-1H-benzo[d]imidazol-2(3H)-one

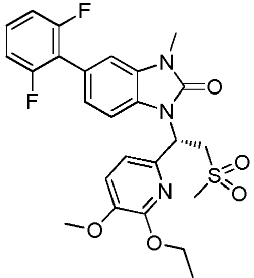

The compound was synthesized by referring to the method of Example 19.

¹HNMR (400 MHz, DMSO-D6) δ 7.27-7.15(m,4H), 7.08-7.06(t,1H), 7.04-7.00(t,1H), 6.83-6.81(d,1H), 6.07-6.03(q,1H), 4.39-4.22(m,5H), 3.74(s,3H), 3.35(s,3H), 3.02(s,3H)1.30-1.27(t,3H).

ESI MS m/z: 518.15[M+1]⁺

### Example 156. (S)-5-(2,4-difluorophenyl)-1-(1-(6-ethoxy-5-methoxypyridin-2-yl) -2-(methylsulfonyl)ethyl)-1H-benzo[d]imidazol-2(3H)-one

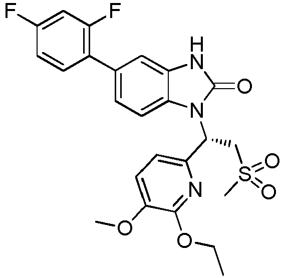

The compound was synthesized by referring to the method of Example 2.

¹H NMR (400 MHz, DMSO-D6) δ11.14(s,1H), 7.57-7.51(m,1H), 7.37-7.31(m, 1H), 7.27-7.24(q,2H), 7.22-7.17(m,1H), 7.16-7.07(m,2H), 6.85-6.83(t,1H), 6.06-6.03(q,1H), 4.38-4.34(m,3H), 4.33-4.22(m,1H), 3.75(s,3H), 3.05(s,3H), 1.32-1.29(t,3H).

ESI MS m/z: 504.13[M+1]⁺

### Example 157. (S)-5-(2,4-difluorophenyl)-1-(1-(6-ethoxy-5-methoxypyridin-2-yl) -2-(methylsulfonyl)ethyl)-3-methyl-1H-benzo[d]imidazol-2(3H)-one

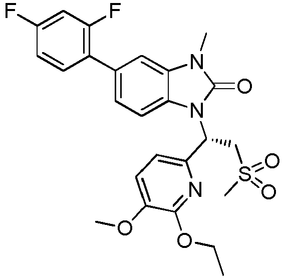

The compound was synthesized by referring to the method of Example 19.

¹HNMR (400 MHz, DMSO-D6) δ 7.38-7.31(m,1H), 7.29-7.13(m,6H), 6.87-6.85(d,1H), 6.10-6.06(q,1H), 4.42-4.28(m,4H), 3.75(s,3H), 3.38(s,3H), 3.05(s,3H), 1.31-1.24(q,3H).

ESI MS m/z: 518.15[M+1]⁺

### Example 158. (S)-5-(2,4,5-trifluorophenyl)-1-(1-(6-ethoxy-5-methoxypyridin-2-yl)-2-(methylsulfonyl)ethyl)-1H-benzo[d]imidazol-2(3H)-one

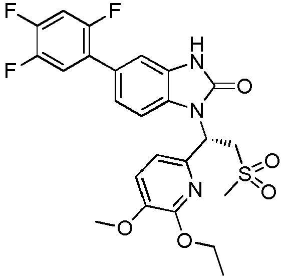

The compound was synthesized by referring to the method of Example 2.

¹H NMR (400 MHz, DMSO-D6) δ11.17(s,1H), 7.68-7.62(m,2H), 7.27-7.23 (t,2H)7.14-7.11(m,2H), 6.85-6.83(d,1H), 6.06-6.03(q,1H), 4.40-4.34(m,3H), 4.28-4.25(t,1H), 3.75(s,3H), 3.05(s,3H), 1.32-1.29(t,3H).

ESI MS m/z: 522.12[M+1]⁺

### Example 159. (S)-5-(2,4,5-trifluorophenyl)-1-(1-(6-ethoxy-5-methoxypyridin-2-yl)-2-(methylsulfonyl)ethyl)-3-methyl-1H-benzo[d]imidazol-2(3H)-one

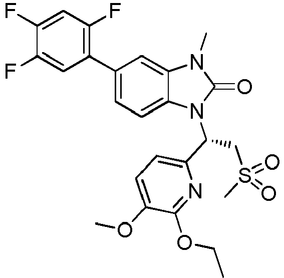

The compound was synthesized by referring to the method of Example 19.

¹HNMR (400 MHz, DMSO-D6) δ 7.75-7.63(m,2H), 7.38(s,1H), 7.33-7.31(d,1H), 7.27-7.25(d,1H), 7.20-7.18(d,1H), 6.87-6.85(d,1H), 6.10-6.07(q,1H), 4.41-4.25(m,4H), 3.75(s,3H), 3.39(s,3H), 3.05(s,3H), 1.31-1.28(t,3H).

ESI MS m/z: 536.14[M+1]⁺

### Example 160. (S)-5-(2,5-difluorophenyl)-1-(1-(6-ethoxy-5-methoxypyridin-2-yl) -2-(methylsulfonyl)ethyl)-1H-benzo[d]imidazol-2(3H)-one

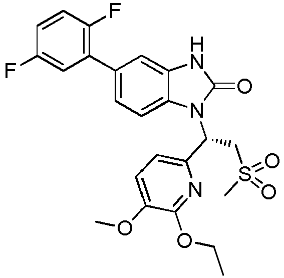

The compound was synthesized by referring to the method of Example 2.

¹H NMR (400 MHz, DMSO-D6)δ11.17(s,1H), 7.39-7.25(m,2H), 7.24-7.1(m,3H), 7.16-7.14(q,2H), 6.85-6.83(t,1H), 6.07-6.03(q,1H), 4.40-4.35(m,3H), 4.33-4.22(m,1H), 3.75(s,3H), 3.05(s,3H), 1.32-1.29(t,3H).

ESI MS m/z: 504.13[M+1]⁺

### Example 161. (S)-5-(2,5-difluorophenyl)-1-(1-(6-ethoxy-5-methoxypyridin-2-yl) -2-(methylsulfonyl)ethyl)-3-methyl-1H-benzo[d]imidazol-2(3H)-one

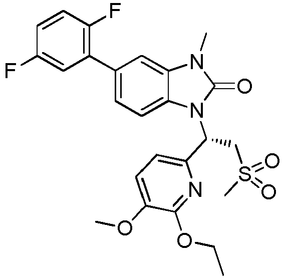

The compound was synthesized by referring to the method of Example 19.

¹HNMR (400 MHz, DMSO-D6) δ 7.46-7.31(m,4H), 7.26-7.21(m,3H), 6.87-6.85(d,1H), 6.10-6.07(q,1H), 4.37-4.25(m,4H), 3.74(s,3H), 3.39(s,3H), 3.05(s,3H), 1.31-1.27(q,3H).

ESI MS m/z: 518.15[M+1]⁺

### Example 162. (S)-5-(2,4-dimethylphenyl)-1-(1-(6-ethoxy-5-methoxypyridin-2-y l)-2-(methylsulfonyl)ethyl)-1H-benzo[d]imidazol-2(3H)-one

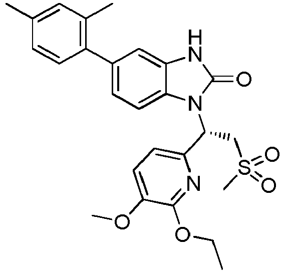

The compound was synthesized by referring to the method of Example 2.

¹H NMR (400 MHz, DMSO-D6) δ11.00(s,1H), 7.36-7.26(m,1H), 7.19-7.17(t,2H), 7.11-7.01(m,1H), 6.99-6.94(t,3H), 6.87-6.85(q,1H), 6.05-6.01(q,1H), 4.51-4.26(m,3H), 4.41-4.22(q,1H), 3.77-3.74(q,3H), 3.07-3.02(t,3H), 2.30-2.29(d,3H), 2.20-2.28(d,3H), 1.31-1.25(q,3H).

ESI MS m/z: 496.18[M+1]⁺

### Example 163. (S)-5-(2,4-dimethylphenyl)-1-(1-(6-ethoxy-5-methoxypyridin-2-y l)-2-(methylsulfonyl)ethyl)-3-methyl-1H-benzo[d]imidazol-2(3H)-one

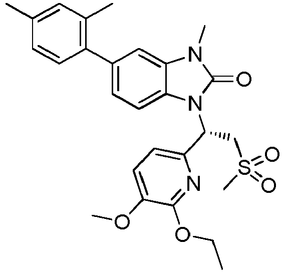

The compound was synthesized by referring to the method of Example 19.

¹HNMR (400 MHz, DMSO-D6) δ 7.27-7.24(q,2H), 7.11-7.05(q,5H), 6.93-6.87(q,1H), 6.08-6.05(q,1H), 4.42-4.31(m,4H), 3.77-3.75(d,3H), 3.35(s,3H), 3.07-3.05(s,3H), 2.30(s,3H), 1.29-1.23(q,3H).

ESI MS m/z: 510.20[M+1]+

### Example 164. (S)-5-(2,5-dimethylphenyl)-1-(1-(6-ethoxy-5-methoxypyridin-2-y l)-2-(methylsulfonyl)ethyl)-1H-benzo[d]imidazol-2(3H)-one

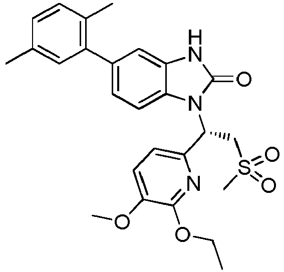

The compound was synthesized by referring to the method of Example 2.

¹H NMR (400 MHz, DMSO-D6) δ11.02(s,1H), 7.28-7.26(d,1H), 7.20-7.13(q,2H), 7.05-7.03(d,1H), 6.98(s,1H), 6.88-6.86(q,3H), 6.05-6.02(q,1H), 4.42-4.33(m,3H), 4.28-4.26(d,1H), 3.78-3.75(d,3H), 3.08-3.05(d,3H), 2.27(s,3H), 2.19-2.16(d,3H), 1.31-1.26(q,3H) .

ESI MS m/z: 496.18[M+1]⁺

### Example 165. (S)-5-(2,5-dimethylphenyl)-1-(1-(6-ethoxy-5-methoxypyridin-2-y l)-2-(methylsulfonyl)ethyl)-3-methyl-1H-benzo[d]imidazol-2(3H)-one

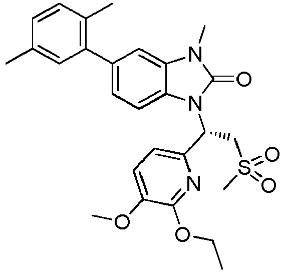

The compound was synthesized by referring to the method of Example 19.

¹HNMR (400 MHz, DMSO-D6) δ 7.27-7.25(q,2H), 7.17-7.13(t,2H), 7.06-7.02(t,2H), 6.94-6.92(d,1H), 6.89-6.87(d,1H), 6.09-6.05(q,1H), 4.44-4.27(m,4H), 3.75(s,3H), 3.36(s,3H), 3.05(s,3H), 2.28(s,3H), 2.18(s,3H), 1.30-1.26(t,3H).

ESI MS m/z: 510.20[M+1]+

### Example 166. (S)-5-(2,3-dimethylphenyl)-1-(1-(6-ethoxy-5-methoxypyridin-2-y l)-2-(methylsulfonyl)ethyl)-1H-benzo[d]imidazol-2(3H)-one

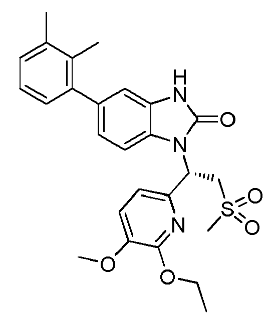

The compound was synthesized by referring to the method of Example 2.

¹H NMR (400 MHz, DMSO-D6)δ11.02(s,1H), 7.28-7.26(d,1H), 7.18-7.08(m,3H), 7.00-6.99(d,1H), 6.88-6.83(m.3H), 6.05-6.02(q,1H), 4.42-4.33(m,3H), 4.29-4.27(d,1H), 3.75(s,3H), 3.05(s,3H), 2.08(s,3H), 2.09(s,3H), 1.31-1.24(q,3H).

ESI MS m/z: 496.18[M+1]⁺

### Example 167. (S)-5-(2,3-dimethylphenyl)-1-(1-(6-ethoxy-5-methoxypyridin-2-y l)-2-(methylsulfonyl)ethyl)-3-methyl-1H-benzo[d]imidazol-2(3H)-one

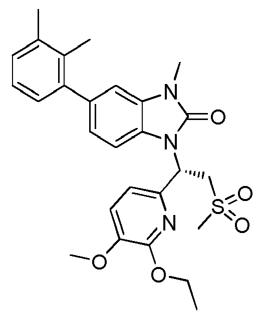

The compound was synthesized by referring to the method of Example 19.

¹H NMR (400 MHz, DMSO-D6)7.28-7.26(d,2H), 7.17-7.10(m,3H), 7.04-7.03(d,1H), 6.91-6.88(m,2H), 6.10-6.06(q,1H), 4.44-4.30(m,4H), 3.75(s,3H), 3.36(s,3H), 3.05(s,3H), 2.29(s,3H), 2.11(s,3H), 1.30-1.26(t,3H).

ESI MS m/z: 510.20[M+1]⁺

### Example 168. (S)-5-(2,6-dimethylphenyl)-1-(1-(6-ethoxy-5-methoxypyridin-2-y l)-2-(methylsulfonyl)ethyl)-1H-benzo[d]imidazol-2(3H)-one

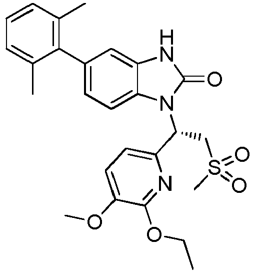

The compound was synthesized by referring to the method of Example 2.

ESI MS m/z: 496.18[M+1]⁺

### Example 169. (S)-5-(4-fluoro-2-methylphenyl)-1-(1-(6-ethoxy-5-methoxypyridi n-2-yl)-2-(methylsulfonyl)ethyl)-1H-benzo[d]imidazol-2(3H)-one

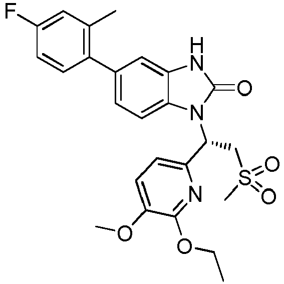

The compound was synthesized by referring to the method of Example 2.

¹H NMR (400 MHz, DMSO-D6) δ11.01(s,1H), 7.25-7.20(t,1H), 7.12-7.10(d,2H), 7.09-7.07(d,2H), 6.84-6.82(d,1H), 6.05-6.02(q,1H), 4.40-4.33(m,3H), 4.28-4.22(q,1H), 3.75(s,3H), 3.05-3.01(d,3H), 2.34(s,3H), 1.33-1.29(q,3H).

ESI MS m/z: 500.16[M+1]⁺

### Example 170. (S)-5-(4-fluoro-2-methylphenyl)-1-(1-(6-ethoxy-5-methoxypyridi n-2-yl)-2-(methylsulfonyl)ethyl)-3-methyl-1H-benzo[d]imidazol-2(3H)-one

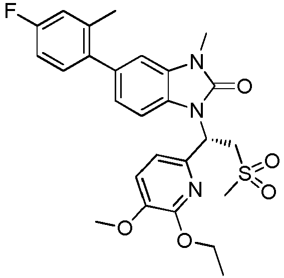

The compound was synthesized by referring to the method of Example 19.

ESI MS m/z: 514.17[M+1]⁺

### Example 171. (S)-5-(5-fluoro-2-methylphenyl)-1-(1-(6-ethoxy-5-methoxypyridi n-2-yl)-2-(methylsulfonyl)ethyl)-1H-benzo[d]imidazol-2(3H)-one

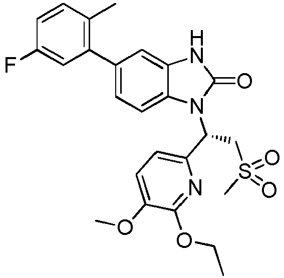

The compound was synthesized by referring to the method of Example 2.

ESI MS m/z: 500.16[M+1]⁺

### Example 172. (S)-5-(5-fluoro-2-methylphenyl)-1-(1-(6-ethoxy-5-methoxypyridi n-2-yl)-2-(methylsulfonyl)ethyl)-3-methyl-1H-benzo[d]imidazol-2(3H)-one

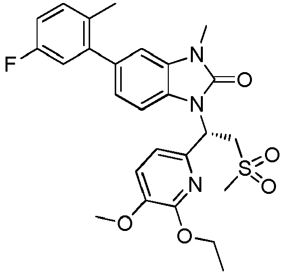

The compound was synthesized by referring to the method of Example 19.

¹HNMR (400 MHz, DMSO-D6) δ 7.34-7.26(m,3H), 7.20(s,1H), 7.12-7.03(m,2H), 7.00-6.98(q,1H), 6.90-6.88(d,1H), 6.10-6.06(q,1H), 4.44-4.26(m,4H), 3.75(s,3H), 3.37(s,3H), 3.06(s,3H), 2.21(s,3H), 1.30-1.26(t,3H).

ESI MS m/z: 514.17[M+1]⁺

### Example 173. (S)-5-(3-fluoro-2-methylphenyl)-1-(1-(6-ethoxy-5-methoxypyridi n-2-yl)-2-(methylsulfonyl)ethyl)-1H-benzo[d]imidazol-2(3H)-one

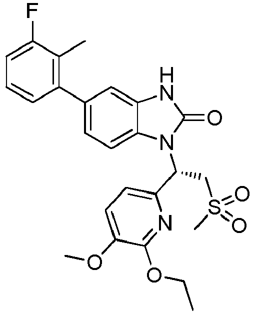

The compound was synthesized by referring to the method of Example 2.

¹H NMR (400 MHz, DMSO-D6) δ11.08(s,1H), 7.16-7.11(t,3H), 7.06-7.04(d,1H), 6.91-6.90(t,1H), 6.88-6.86(d,3H), 6.05-6.02(q,1H), 4.42-4.23(m,4H), 3.75(s,3H), 3.05(s,3H), 2.12(s,1H), 1.31-1.27(t,3H).

ESI MS m/z: 500.16[M+1]⁺

### Example 174. (S)-5-(3-fluoro-2-methylphenyl)-1-(1-(6-ethoxy-5-methoxypyridi n-2-yl)-2-(methylsulfonyl)ethyl)-3-methyl-1H-benzo[d]imidazol-2(3H)-one

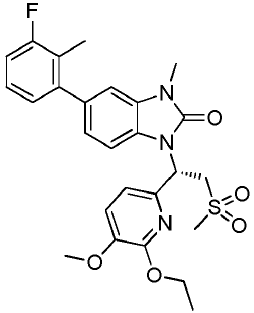

The compound was synthesized by referring to the method of Example 19.

¹HNMR (400 MHz, DMSO-D6) δ 7.30-7.25(q,3H), 7.18-7.13(t,2H), 7.10-7.08(d,1H), 6.98-6.96(q,1H), 6.90-6.88(d,1H), 6.09-6.06(q,1H), 4.44-4.26(m,4H), 3.75(s,3H), 3.37(s,3H), 3.05(s,3H), 2.14(s,3H), 1.29-1.26(t,3H).

ESI MS m/z: 514.17[M+1]⁺

### Example 175. (S)-5-(2-fluoro-5-methylphenyl)-1-(1-(6-ethoxy-5-methoxypyridi n-2-yl)-2-(methylsulfonyl)ethyl)-1H-benzo[d]imidazol-2(3H)-one

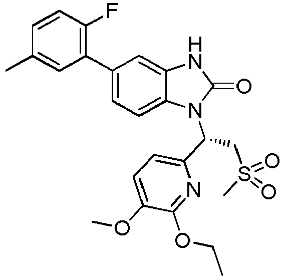

The compound was synthesized by referring to the method of Example 2.

¹H NMR (400 MHz, DMSO-D6) δ11.11(s,1H), 7.30-7.21(m,3H), 7.16-7.09(q,4H), 6.85-6.83(d,1H), 6.06-6.03(q,1H), 4.40-4.33(m,3H), 4.28-4.22(q,1H), 3.75(s,3H), 3.05(s,3H), 2.32(s,3H), 1.34-1.24(q,3H)

ESI MS m/z: 500.16[M+1]⁺

### Example 176. (S)-5-(2-fluoro-5-methylphenyl)-1-(1-(6-ethoxy-5-methoxypyridi n-2-yl)-2-(methylsulfonyl)ethyl)-3-methyl-1H-benzo[d]imidazol-2(3H)-one

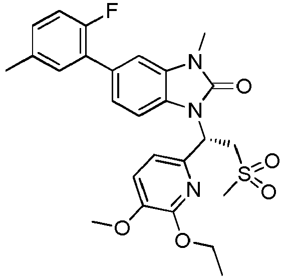

The compound was synthesized by referring to the method of Example 19.

¹HNMR (400 MHz, DMSO-D6) δ 7.35-7.24(m,4H), 7.18-7.15(q,3H), 6.86-6.84(d,1H), 6.10-6.06(q,1H), 4.42-4.25(m,4H), 3.74(s,3H), 3.39(s,3H), 3.05(s,3H), 2.33(s,3H), 1.33-1.29(t,3H).

ESI MS m/z: 514.17[M+1]⁺

### Example 177. (S)-5-(2-fluoro-3-methylphenyl)-1-(1-(6-ethoxy-5-methoxypyridi n-2-yl)-2-(methylsulfonyl)ethyl)-1H-benzo[d]imidazol-2(3H)-one

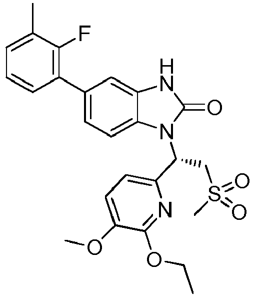

The compound was synthesized by referring to the method of Example 2.

¹H NMR (400 MHz, DMSO-D6)δ11.11(s,1H), 7.29-7.22(m,4H), 7.16-7.08(m,3H), 6.85-6.83(d,1H), 6.06-6.02(q,1H), 4.41-4.33(m,3H), 4.28-4.26(d,1H), 3.75(s,3H), 3.05(s,3H), 2.29-2.28(d,3H), 1.32-1.29(q,3H).

ESI MS m/z: 500.16[M+1]⁺

### Example 178. (S)-5-(2-fluoro-3-methylphenyl)-1-(1-(6-ethoxy-5-methoxypyridi n-2-yl)-2-(methylsulfonyl)ethyl)-3-methyl-1H-benzo[d]imidazol-2(3H)-one

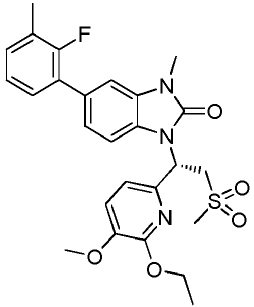

The compound was synthesized by referring to the method of Example 19.

¹HNMR (400 MHz, DMSO-D6) δ 7.31-7.22(m,5H), 7.18-7.11(q,2H), 6.87-6.83(t,1H), 6.10-6.06(q,1H), 4.42-4.29(m,4H), 3.75(s,3H), 3.38(s,3H), 3.05(s,3H), 2.29(s,3H), 1.31-1.27(t,3H).

ESI MS m/z: 514.17[M+1]+

### Example 179. (S)-5-(2-fluoro-4-methylphenyl)-1-(1-(6-ethoxy-5-methoxypyridi n-2-yl)-2-(methylsulfonyl)ethyl)-1H-benzo[d]imidazol-2(3H)-one

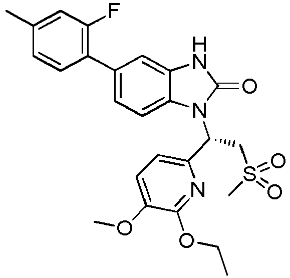

The compound was synthesized by referring to the method of Example 2.

¹H NMR (400 MHz, DMSO-D6) δ11.01(s,1H), 7.25-7.20(t,1H), 7.12-7.10(d,2H), 7.09-7.07(d,2H), 6.84-6.82(d,1H), 6.05-6.02(q,1H), 4.40-4.33(m,3H), 4.28-4.22(q,1H), 3.75(s,3H), 3.05-3.01(d,3H), 2.34(s,3H), 1.33-1.29(q,3H).

ESI MS m/z: 500.16[M+1]⁺

### Example 180. (S)-5-(2-fluoro-4-methylphenyl)-1-(1-(6-ethoxy-5-methoxypyridi n-2-yl)-2-(methylsulfonyl)ethyl)-3-methyl-1H-benzo[d]imidazol-2(3H)-one

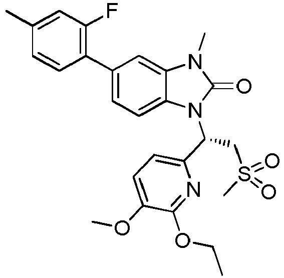

The compound was synthesized by referring to the method of Example 19.

¹HNMR (400 MHz, DMSO-D6) δ 7.44-7.40(t,1H), 7.31-7.24(m,3H), 7.15-7.09(q,3H), 6.86-6.84(d,1H), 6.09-6.06(q,1H), 4.38-4.24(m,4H), 3.74(s,3H), 3.38(s,3H), 3.04(s,3H), 2.35(s,3H), 1.31-1.28(q,3H).

ESI MS m/z: 514.17[M+1]⁺

### Example 181. (S)-5-(2-fluoro-5-methoxyphenyl)-1-(1-(6-ethoxy-5-methoxypyri din-2-yl)-2-(methylsulfonyl)ethyl)-1H-benzo[d]imidazol-2(3H)-one

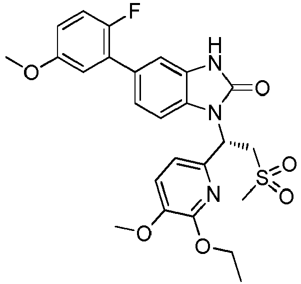

The compound was synthesized by referring to the method of Example 2.

¹H NMR (400 MHz, DMSO-D6) δ11.11(s,1H), 7.27-7.18(m,3H), 7.13-7.11(d,2H), 6.99-6.97(q,1H), 6.06-6.03(q,1H), 4.40-4.35(m,3H), 4.28-4.22(q,1H), 3.78-3.75(d,6H), 3.05(s,3H), 1.33-1.29(t,3H).

ESI MS m/z: 516.15[M+1]⁺

### Example 182. (S)-5-(2-fluoro-5-methoxyphenyl)-1-(1-(6-ethoxy-5-methoxypyri din-2-yl)-2-(methylsulfonyl)ethyl)-3-methyl-1H-benzo[d]imidazol-2(3H)-one

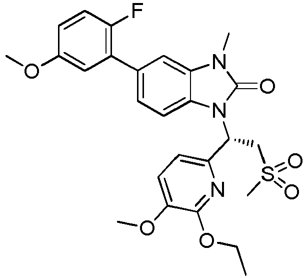

The compound was synthesized by referring to the method of Example 19.

¹HNMR (400 MHz, DMSO-D6) δ 7.29-7.08(m,7H), 6.86-6.84(d,1H), 6.10-6.06(q,1H), 4.43-4.24(m,4H), 3.75-3.74(d,6H), 3.37(s,3H), 3.06(s,3H), 1.33-1.29(t,3H).

ESI MS m/z: 530.17[M+1]⁺

### Example 183. (S)-5-(2-fluoro-3-methoxyphenyl)-1-(1-(6-ethoxy-5-methoxypyri din-2-yl)-2-(methylsulfonyl)ethyl)-1H-benzo[d]imidazol-2(3H)-one

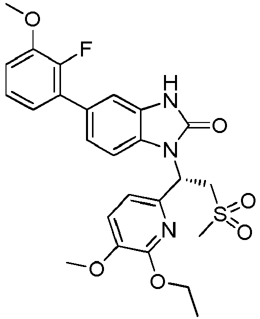

The compound was synthesized by referring to the method of Example 2.

ESI MS m/z: 516.15[M+1]⁺

### Example 184. (S)-5-(2-fluoro-4-methoxyphenyl)-1-(1-(6-ethoxy-5-methoxypyri din-2-yl)-2-(methylsulfonyl)ethyl)-1H-benzo[d]imidazol-2(3H)-one

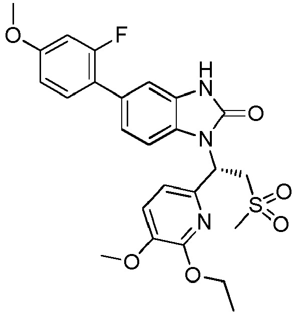

The compound was synthesized by referring to the method of Example 2.

¹H NMR (400 MHz, DMSO-D6) δ11.07(s,1H), 7.25-7.20(d,1H), 7.18(s,1H), 7.07-7.04(t,1H), 6.93-6.90(t,2H), 6.89-6.82(m,3H), 6.05-6.02(q,1H), 4.40-4.35(m,3H), 4.27-4.21(q,1H), 3.80-3.75(t,3H), 3.05(s,3H), 1.33-1.29(t,3H).

ESI MS m/z: 516.15[M+1]⁺

### Example 185. (S)-5-(2-fluoro-4-methoxyphenyl)-1-(1-(6-ethoxy-5-methoxypyri din-2-yl)-2-(methylsulfonyl)ethyl)-3-methyl-1H-benzo[d]imidazol-2(3H)-one

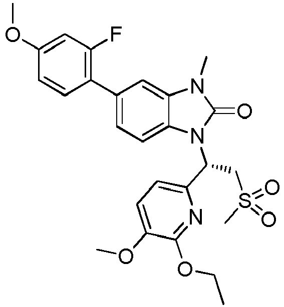

The compound was synthesized by referring to the method of Example 19.

¹H NMR (400MHz,DMSO-D6)7.48-7.43(t,1H), 7.28-7.24(q,3H), 7.13-7.11(d,1H), 6.95-6.84(m,3H), 6.10-6.06(q,1H), 4.42-4.33(m,3H), 4.30-4.24(q,1H), 3.81-3.75(t,6H), 3.38(s,3H), 3.05(s,3H), 1.32-1.28(t,3H)..

ESI MS m/z: 530.17[M+1]⁺

### Example 186. (S)-5-(5-fluoro-2-methoxyphenyl)-1-(1-(6-ethoxy-5-methoxypyri din-2-yl)-2-(methylsulfonyl)ethyl)-1H-benzo[d]imidazol-2(3H)-one

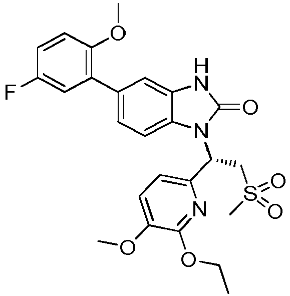

The compound was synthesized by referring to the method of Example 2.

¹H NMR (400 MHz, DMSO-D6) δ11.04(s,1H), 7.27-7.10(m,1H), 7.09-7.03(m,6H), 6.84-6.82(d,1H), 6.05-6.02(q,1H), 4.41-4.34(m,3H), 4.27-4.21(q,1H), 3.75-3.73(d,6H), 3.05(s,3H), 1.34-1.24(q,3H)..

ESI MS m/z: 516.15[M+1]⁺

### Example 187. (S)-5-(5-fluoro-2-methoxyphenyl)-1-(1-(6-ethoxy-5-methoxypyri din-2-yl)-2-(methylsulfonyl)ethyl)-3-methyl-1H-benzo[d]imidazol-2(3H)-one

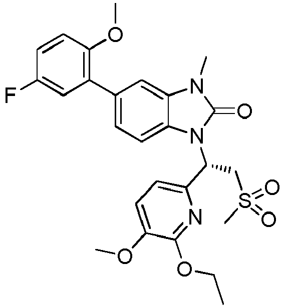

The compound was synthesized by referring to the method of Example 19.

¹HNMR (400 MHz, DMSO-D6) δ 7.36(s,1H), 7.30-7.18(m,4H), 7.06-7.03(q,1H), 6.95-6.91(m,1H), 6.87-6.85(d,1H), 6.11-6.07(q,1H), 4.39-4.25(m,4H), 3.79(s,3H), 3.75(s,3H), 3.40-3.37(d,3H), 3.05(s,3H)1.32-1.18(t,3H)..

ESI MS m/z: 530.17[M+1]⁺

### Example 188. (S)-5-(4-fluoro-2-methoxyphenyl)-1-(1-(6-ethoxy-5-methoxypyri din-2-yl)-2-(methylsulfonyl)ethyl)-1H-benzo[d]imidazol-2(3H)-one

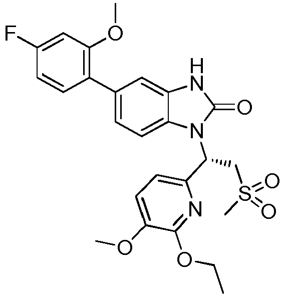

The compound was synthesized by referring to the method of Example 2.

¹H NMR (400 MHz, DMSO-D6)δ11.11(s,1H), 7.29-7.22(m,4H), 7.16-7.08(m,3H), 6.85-6.83(d,1H), 6.06-6.02(q,1H), 4.41-4.33(m,3H), 4.28-4.26(d,1H), 3.75(s,3H), 3.05(s,3H), 2.29-2.28(d,3H), 1.32-1.29(q,3H)..

ESI MS m/z: 516.15[M+1]⁺

### Example 189. (S)-5-(4-fluoro-2-methoxyphenyl)-1-(1-(6-ethoxy-5-methoxypyri din-2-yl)-2-(methylsulfonyl)ethyl)-3-methyl-1H-benzo[d]imidazol-2(3H)-one

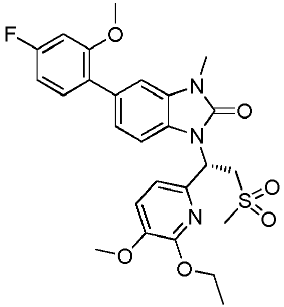

The compound was synthesized by referring to the method of Example 19.

¹HNMR (400 MHz, DMSO-D6) δ 7.20(s,4H), 7.06-7.01(m,2H), 6.99-6.81(m,2H), 6.09-6.05(q,1H), 4.42-4.24(m,4H), 3.77-3.75(d,6H), 3.36(s,3H), 3.07-3.05(d,3H), 1.32-1.29(t,3H).

ESI MS m/z: 530.17[M+1]⁺

### Example 190. (S)-5-(6-fluoro-2-methoxyphenyl)-1-(1-(6-ethoxy-5-methoxypyri din-2-yl)-2-(methylsulfonyl)ethyl)-1H-benzo[d]imidazol-2(3H)-one

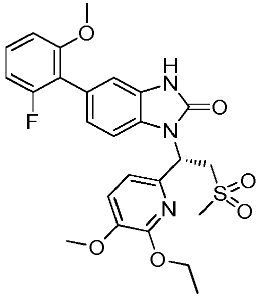

The compound was synthesized by referring to the method of Example 2.

¹H NMR (400 MHz, DMSO-D6) δ11.01(s,1H), 7.28-7.26(d,1H), 7.18-7.16(d,1H), 6.89-6.85(t,5H), 6.06-6.02(q,1H), 4.42-4.34(m,3H), 4.28-4.22(q,1H),3.76-3.72(q,6H), 3.10-3.06(d,3H), 1.32-1.27(q,3H).

ESI MS m/z: 516.15[M+1]⁺

### Example 191. (S)-5-(6-fluoro-2-methoxyphenyl)-1-(1-(6-ethoxy-5-methoxypyri din-2-yl)-2-(methylsulfonyl)ethyl)-3-methyl-1H-benzo[d]imidazol-2(3H)-one

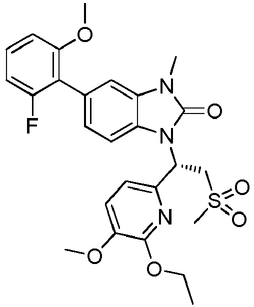

The compound was synthesized by referring to the method of Example 19.

¹HNMR (400 MHz, DMSO-D6) δ 7.39-7.34(q,1H), 7.27-7.24(q,2H), 7.12(s,1H), 6.97-6.87(m,4H), 6.10-6.07(q,1H), 4.39-4.28(m,4H), 3.77-3.73(q,6H), 3.34(s,3H), 3.07(s,3H)1.31-1.28(t,3H).

ESI MS m/z: 530.17[M+1]⁺

### Example 192. (S)-1-(1-(1-(6-ethoxy-5-methoxypyridin-2-yl)-2-(methylsulfonyl) ethyl)-5-(2-ethoxyphenyl)-1H-benzo[d]imidazol-2(3H)-one

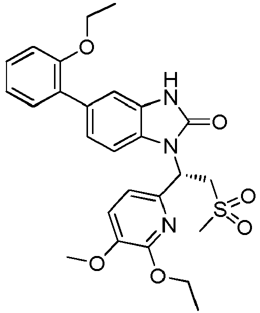

The compound was synthesized by referring to the method of Example 2.

ESI MS m/z: 512.18[M+1]⁺

### Example 193. (R)-1-(1-(1-(6-ethoxy-5-methoxypyridin-2-yl)-2-(methylsulfonyl) ethyl)-5-(2-ethoxyphenyl)-1H-benzo[d]imidazol-2(3H)-one

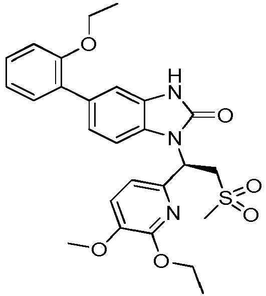

The compound was synthesized by referring to the method of Example 2.

ESI MS m/z: 512.18[M+1]⁺

### Example 194. (S)-1-(1-(1-(6-ethoxy-5-methoxypyridin-2-yl)-2-(methylsulfonyl) ethyl)-5-(pyridin-2-yl)-1H-benzo[d]imidazol-2(3H)-one

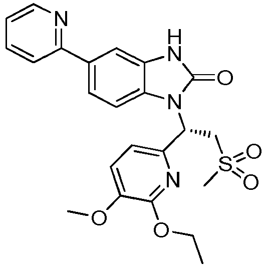

The compound was synthesized by referring to the method of Example 2.

ESI MS m/z: 469.15[M+1]⁺

### Example 195. (R)-1-(1-(1-(6-ethoxy-5-methoxypyridin-2-yl)-2-(methylsulfonyl) ethyl)-5-(pyridin-2-yl)-1H-benzo[d]imidazol-2(3H)-one

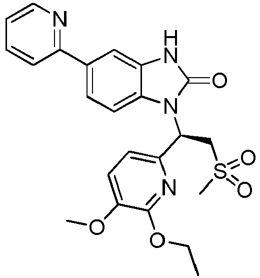

The compound was synthesized by referring to the method of Example 2.

ESI MS m/z: 469.15[M+1]⁺

### Example 196. (S)-1-(1-(6-ethoxy-5-methoxypyridin-2-yl)-2-(methylsulfonyl)eth yl)-3-methyl-5-(pyridin-2-yl)-1H-benzo[d]imidazol-2(3H)-one

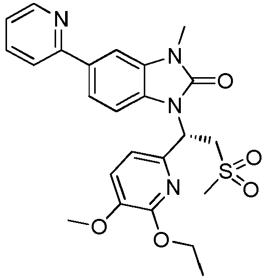

The compound was synthesized by referring to the method of Example 19.

ESI MS m/z: 483.16[M+1]⁺

### Example 197. (S)-1-(1-(1-(6-ethoxy-5-methoxypyridin-2-yl)-2-(methylsulfonyl) ethyl)-3-ethyl-5-(pyridin-2-yl)-1H-benzo[d]imidazol-2(3H)-one

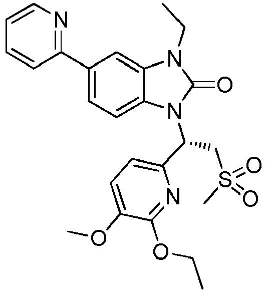

The compound was synthesized by referring to the method of Example 19.

ESI MS m/z: 497.18[M+1]⁺

### Example 198. (S)-1-(1-(6-ethoxy-5-methoxypyridin-2-yl)-2-(methylsulfonyl)eth yl)-3-(2-methoxyethyl)-5-(pyridin-2-yl)-1H-benzo[d]imidazol-2(3H)-one

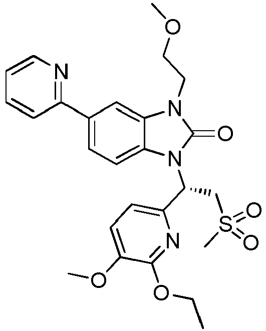

The compound was synthesized by referring to the method of Example 19.

ESI MS m/z: 527.19[M+1]⁺

### Example 199. (S)-1-(1-(6-ethoxy-5-methoxypyridin-2-yl)-2-(methylsulfonyl)eth yl)-5-(4-fluoro-2-methylphenyl)-1H-benzo[d]imidazol-2(3H)-one

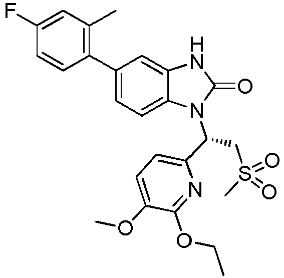

The compound was synthesized by referring to the method of Example 2.

¹H NMR (400 MHz, DMSO-D6) δ11.05(s,1H), 7.18-7.28(m,1H), 7.12(t,3H), 7.04(d,1H), 6.86(t,3H), 6.04(q,1H), 4.28-4.38(m,3H), 4.27(d,1H), 3.75(s,3H), 3.05(s,3H), 2.21(s,3H), 1.27(q,3H)

ESI MS m/z: 500.16 [M+1]⁺

### Example 200. (S)-1-(1-(6-ethoxy-5-methoxypyridin-2-yl)-2-(methylsulfonyl)eth yl)-5-(4-fluoro-2-methylphenyl)-3-methyl-1H-benzo[d]imidazol-2(3H)-one

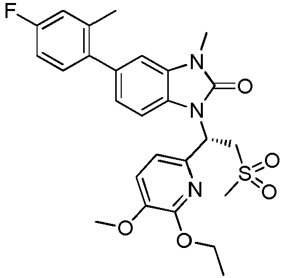

The compound was synthesized by referring to the method of Example 19.

¹H NMR (400 MHz, DMSO-D6) δ7.23-7.28(m,3H), 7.17(t,2H), 7.04-7.09(m,1H), 6.91(q,2H), 6.06(q,1H), 4.25-4.44(m,4H), 3.74(s,3H), 3.34(t,3H), 3.05(s,3H), 2.23(s,3H), 1.26(q,3H).

ESI MS m/z: 500.16 [M+1]⁺

### Example 201. (S)-1-(1-(6-ethoxy-5-methoxypyridin-2-yl)-2-(methylsulfonyl)eth yl)-5-(2-fluoro-5-methylphenyl)-1H-benzo[d]imidazol-2(3H)-one

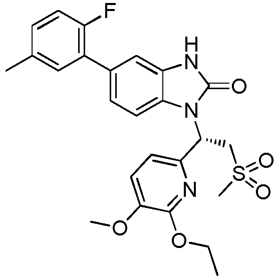

The compound was synthesized by referring to the method of Example 2.

ESI MS m/z: 500.16 [M+1]⁺

### Example 202. (S)-1-(1-(6-ethoxy-5-methoxypyridin-2-yl)-2-(methylsulfonyl)eth yl)-5-(2-fluoro-5-methylphenyl)-3-methyl-1H-benzo[d]imidazol-2(3H)-one

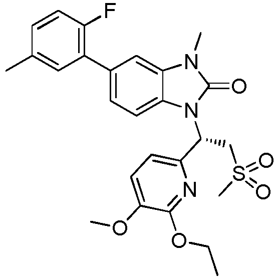

The compound was synthesized by referring to the method of Example 19.

ESI MS m/z: 514.17 [M+1]⁺

### Example 203. (S)-1-(1-(1-(6-ethoxy-5-methoxypyridin-2-yl)-2-(methylsulfonyl) ethyl)-4-(2-methoxyphenyl)-1H-benzo[d]imidazol-2(3H)-one

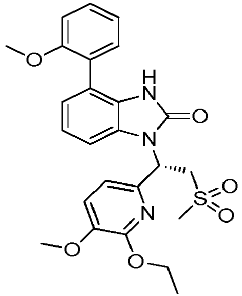

The compound was synthesized by referring to the method of Example 2.

ESI MS m/z: 498.16[M+1]⁺

### Example 204. (S)-1-(1-(1-ethoxy-5-methoxypyridin-2-yl)-2-(methylsulfonyl)eth yl)-4-(o-methylphenyl)-1H-benzo[d]imidazol-2(3H)-one

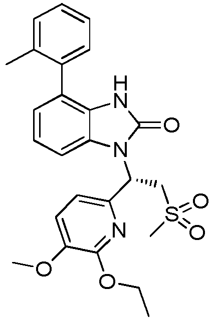

The compound was synthesized by referring to the method of Example 2.

ESI MS m/z: 482.17[M+1]⁺

### Example 205. (S)-1-(1-(1-(6-ethoxy-5-methoxypyridin-2-yl)-2-(methylsulfonyl) ethyl)-4-(2-fluorophenyl)-1H-benzo[d]imidazol-2(3H)-one

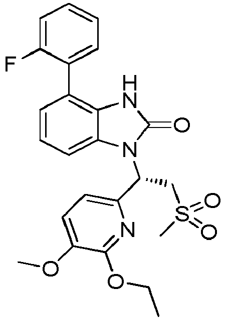

The compound was synthesized by referring to the method of Example 2.

ESI MS m/z: 486.14[M+1]⁺

### Example 206. (S)-1-(1-(1-(6-ethoxy-5-methoxypyridin-2-yl)-2-(methylsulfonyl) ethyl)-4-phenyl-1H-benzo[d]imidazol-2(3H)-one

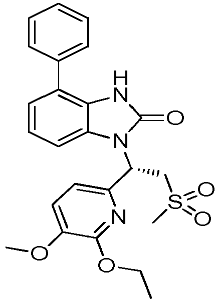

The compound was synthesized by referring to the method of Example 2.

ESI MS m/z: 468.15[M+1]⁺

### Example 207. (S)-1-(1-(1-(6-ethoxy-5-methoxypyridin-2-yl)-2-(methylsulfonyl) ethyl)-4-(2-fluorobenzyl)-1H-benzo[d]imidazol-2(3H)-one

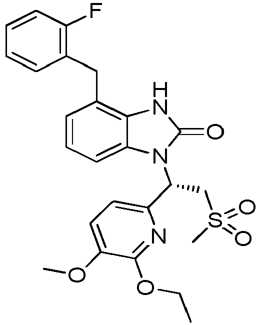

The compound was synthesized by referring to the method of Example 2.

ESI MS m/z: 500.16[M+1]⁺

### Example 208. (S)-3-cyclopropyl-1-(1-(6-ethoxy-5-methoxypyridin-2-yl)-2-(met hylsulfonyl)ethyl)-5-(2-fluorophenyl)-1H-benzo[d]imidazol-2(3H)-one

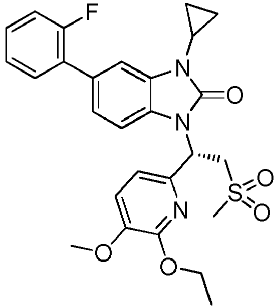

The compound was synthesized by referring to the method of Example 19.

ESI MS m/z: 526.17[M+1]⁺

### Example 209. (S)-3-cyclopropyl-1-(1-(6-ethoxy-5-methoxypyridin-2-yl)-2-(met hylsulfonyl)ethyl)-5-methyl-1H-benzo[d]imidazol-2(3H)-one

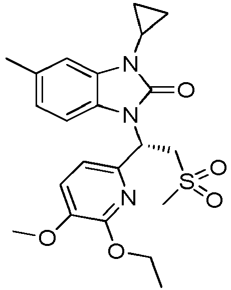

The compound was synthesized by referring to the method of Example 19.

ESI MS m/z: 446.17[M+1]⁺

### Example 210. (S)-5-bromo-1-(1-(6-ethoxy-5-methoxypyridin-2-yl)-2-(methylsul fonyl)ethyl)-3-(2-(methylsulfonyl)ethyl)-1H-benzo[d]imidazol-2(3H)-one

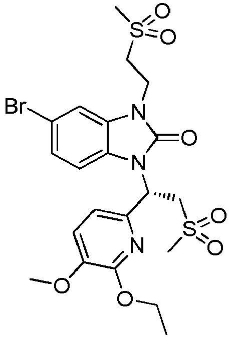

The compound was synthesized by referring to the method of Example 19.

ESI MS m/z: 576.04[M+1]⁺

### Example 211. (S)-3-cyclopropyl-1-(1-(6-ethoxy-5-methoxypyridin-2-yl)-2-(met hylsulfonyl)ethyl)-5-(trifluoromethyl)-1H-benzo[d]imidazol-2(3H)-one

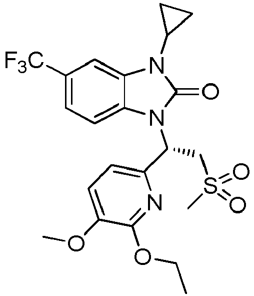

The compound was synthesized by referring to the method of Example 19.

ESI MS m/z: 500.14[M+1]⁺

### Example 212. (S)-ethyl 1-(1-(1-(6-ethoxy-5-methoxypyridin-2-yl)-2-(methylsul fonyl)ethyl)-2-oxo-2,3-dihydro-1H-benzo[d]imidazole-5-carboxylate

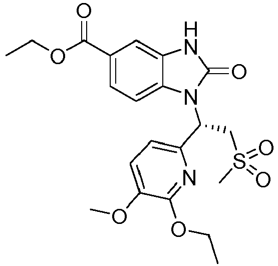

The compound was synthesized by referring to the method of Example 2.

ESI MS m/z: 464.14[M+1]⁺

### Example 213. (S)-isopropyl 1-(1-(1-(6-ethoxy-5-methoxypyridin-2-yl)-2-(meth ylsulfonyl)ethyl)-2-oxo-2,3-dihydro-1H-benzo[d]imidazole-5-carboxylate

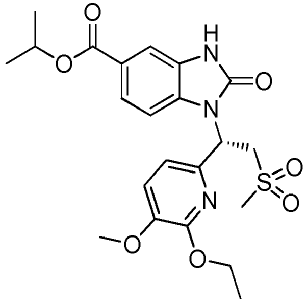

The compound was synthesized by referring to the method of Example 2.

ESI MS m/z: 478.16[M+1]⁺

### Example 214. (S)-2,2,2-trifluoroethyl 1-(1-(1-ethoxy-5-methoxypyridin-2-yl)-2 -(methylsulfonyl)ethyl)-2-oxo-2,3-dihydro-1H-benzo[d]imidazole-5-carboxylate

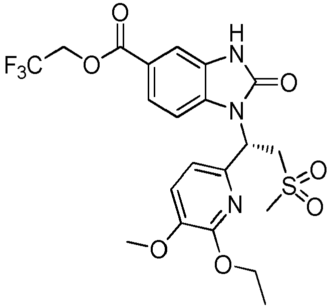

The compound was synthesized by referring to the method of Example 2.

ESI MS m/z: 518.11[M+1]⁺

### Example 215. (S)-N-(2-(1-(6-ethoxy-5-methoxypyridin-2-yl)-2-(methylsulfonyl) ethyl)-3-oxoisoindolin-4-yl)acetamide

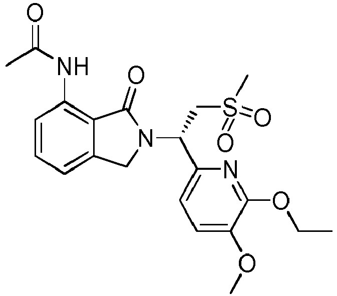

### Step 1. (S)-methyl 2-(((1-(6-ethoxy-5-methoxypyridin-2-yl)-2-(methylsulfonyl)eth yl)amino)methyl)-6-nitrobenzoate

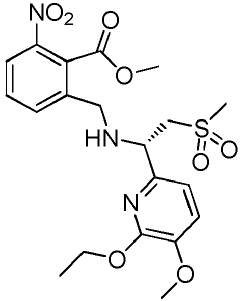

Methyl 2-(bromomethyl)-6-nitro benzoate (200 mg, 0.73 mmol) was dissolved in 2.0 mL of acetonitrile, and (S)-1-(6-ethoxy-5-methoxypyridin-2-yl)-2-(methylsulfonyl) ethylamine (215 mg, 0.78 mg) and potassium carbonate (201 mg, 1.45 mmol) were added. The mixture was heated to 80°C at reflux and reacted for 2 h. The system was concentrated, extracted with ethyl acetate (50 mL x 3). The organic phases were combined, washed with saturated saline, dried over anhydrous sodium sulfate, and filtered. The filtrate was concentrated to obtain an oil, which was dissolved with a small amount of ethyl acetate, and then a large amount of petroleum ether was added to precipitate solids. The solid was filtered and washed twice with methanol to obtain 210 mg of yellow solid.

### Step 2, (S)-2-(1-(6-ethoxy-5-methoxypyridin-2-yl)-2-(methylsulfonyl)ethyl)-7-nitro isoindolin-1-one.

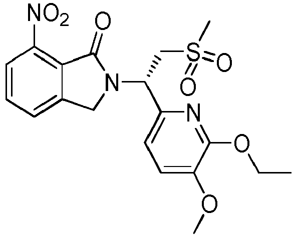

The product of Step 1 (200 mg, 0.43 mmol) was dissolved in 4.0 mL of solvent (acetonitrile : ethanol = 1 : 1), and 0.2 mL of glacial acetic acid was added. The mixture was heated to 90 °C at reflux for reaction. The system was concentrated, extracted with ethyl acetate (50 mL x 3). The organic phases were combined, washed with saturated saline, dried over anhydrous sodium sulfate and filtered. The filtrate was concentrated to obtain 150 mg of the target material.

### Step 3, (S)-7-amino-2-(1-(6-ethoxy-5-methoxypyridin-2-yl)-2-(methylsulfonyl)ethy l)isoindolin-1-one

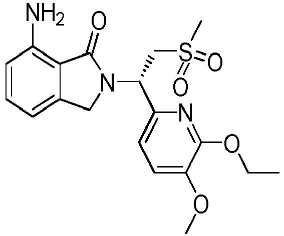

Reductive iron powder was added to 125 mL of solvent of ethanol/water (4:1), and 4 drops of glacial acetic acid were added. The mixture was heated to 70-80°C at reflux to be activated for 30 min. The product of Step 2 (150 mg, 0.345 mmol) was added, and the mixture was reacted at reflux for 2 hours. The system was filtered through diatomaceous earth with suction. The filtrate was concentrated and extracted with ethyl acetate (50 mL x 3). The organic phases were combined, washed with saturated saline, dried over anhydrous sodium sulfate, and filtered. The filtrate was concentrated to obtain 100 mg of the target compound.

### Step 4, (S)-N-(2-(1-(6-ethoxy-5-methoxypyridin-2-yl)-2-(methylsulfonyl)ethyl)-3-o xoisoindolin-4-yl)acetamide

The product of Step 3 (100 mg, 0.25 mmol) was dissolved in 4.0 mL of tetrahydrofuran, and 0.1 mL of acetyl chloride was added. The reaction was heated at reflux for 2 hours. The system was concentrated, and then extracted with ethyl acetate (50 mL x 3). The organic phases were combined, washed with saturated saline, dried over anhydrous sodium sulfate, and filtered. The filtrate was concentrated to obtain 80 mg of the target compound.

¹H NMR (400 MHz, DMSO-D6) δ 10.23 (s, 1H), 8.28-8.26 (d, 1H), 7.55-7.51 (t, 1H), 7.29-7.22 (q,2H), 6.94-6.92 (d, 1H), 5.88-5.85 (q, 1H), 4.73-4.68(d,1H), 4.41-4.31(m,3H), 4.14-4.00(m,2H)3.76(s,3H), 3.03(s,3H), 2.17(s,3H), 1.32-1.29(t,3H).

ESI MS m/z: 448.15[M+1]⁺

### Example 216. (R)-N-(2-(1-(6-ethoxy-5-methoxypyridin-2-yl)-2-(methylsulfonyl) ethyl)-3-oxoisoindolin-4-yl)acetamide

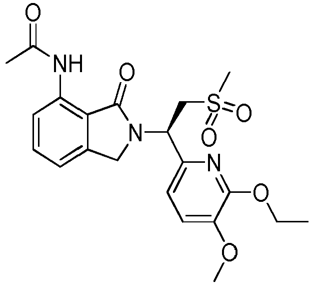

The compound was synthesized by referring to the method of Example 215.

ESI MS m/z: 448.15[M+1]⁺

### Example 217. (S)-N-(2-(1-(6-ethoxy-5-methoxypyridin-2-yl)-2-(methylsulfonyl) ethyl)-3-oxoisoindolin-4-yl)cyclopropanecarboxamide

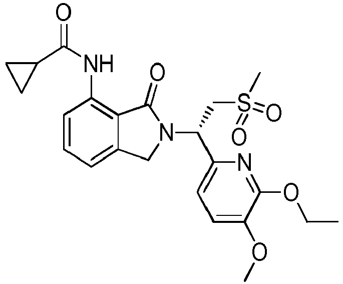

The compound was synthesized by referring to the method of Example 215.

ESI MS m/z: 474.16[M+1]⁺

### Example 218. (R)-N-(2-(1-(6-ethoxy-5-methoxypyridin-2-yl)-2-(methylsulfonyl) ethyl)-3-oxoisoindolin-4-yl)cyclopropanecarboxamide

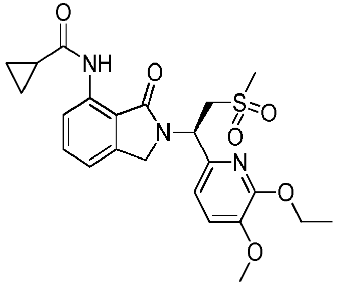

The compound was synthesized by referring to the method of Example 215.

ESI MS m/z: 474.16[M+1]⁺

### Example 219. (S)-N-(2-(1-(6-ethoxy-5-methoxypyridin-2-yl)-2-(methylsulfonyl) ethyl)-3-oxoisoindolin-4-yl)propionamide

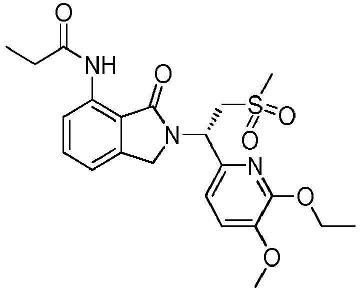

The compound was synthesized by referring to the method of Example 215.

ESI MS m/z: 462.16[M+1]⁺

### Example 220. (R)-N-(2-(1-(6-ethoxy-5-methoxypyridin-2-yl)-2-(methylsulfonyl) ethyl)-3-oxoisoindolin-4-yl)propionamide

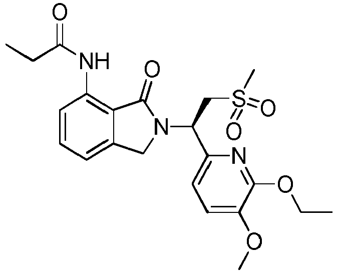

The compound was synthesized by referring to the method of Example 215.

ESI MS m/z: 462.16[M+1]⁺

### Example 221. 5-(1-(6-ethoxy-5-methoxypyridin-2-yl)-2-(methylsulfonyl)ethyl)-1-nitro-4H-thieno[3,4-c]pyrrol-4,6(5H)-dione

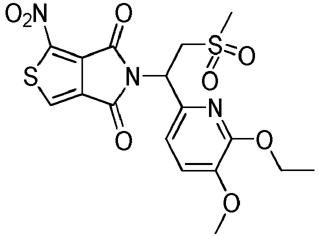

### Step 1: 2-nitrothiophene-3,4-dicarboxylic acid

To a 250 mL round-bottom flask equipped with an electromagnetic stirrer and a drying tube was added 20 mL of fuming nitric acid (95%). The sysrem was cooled to 0-5°C in an ice bath, and 5 g of thieno[3,4-c]furan-1,3-dione was added in batches. The mixture was then reacted at this temperature for 30 min. The reaction mixture was poured into 40 mL of ice-water mixture and extracted with ethyl acetate. All the ethyl acetate layers were combined, washed with water (50 mL x 2) and saturated saline successively, dried over anhydrous sodium sulfate, and filtered. The solvent was evaporated to dryness, and 3.5 g of solid was obtained.

### Step 2: 4-nitrothieno[3,4-c]furan-1,3-dione

To a 50 mL round-bottom flask equipped with an electromagnetic stirrer, a reflux condenser and a drying tube were added 2 g of compound of Step 1 and 2 mL of acetic anhydride. The mixture was refluxed for 3 h. The solvent was evaporated to dryness, and 1.2 g of the target compound was obtained.

### Step 3: 5-(1-(6-ethoxy-5-methoxypyridin-2-yl)-2-(methylsulfonyl)ethyl)-1-nitro-4H -thieno[3,4-c]pyrrol-4,6 (5H)-dione

To a 250 mL round-bottom flask equipped with an electromagnetic stirrer and a drying tube were added 1.0 g of compound of Step 2, 1.36 g of 1-(6-ethoxy-5-methoxypyridin-2-yl)-2-(methylsulfonyl)ethylamine (synthesized by reference to the method disclosed in WO2018157779), and 10 mL THF. The mixture was stirred overnight at room temperature. 1.2 g of CDI was added and the mixture was refluxed in an oil bath for 2 hours. The mixture was cooled to room temperature, and 30 mL of ethyl acetate and 150 mL of water were added. The mixture was extracted, and the layers were separated. The organic layer was washed with 100 mL of 0.5N hydrochloric acid, washed with 100 mL of saturated saline, dried over anhydrous magnesium sulfate, and filtered. The filtrate was evaporated to remove the solvent, and purified by column chromatography to obtain 0.52 g of the target compound. ESI MS m/z: 456.05[M+1]⁺

### Example 222. (S)-5-(1-(6-ethoxy-5-methoxypyridin-2-yl)-2-(methylsulfonyl)eth yl)-1-nitro-4H-thieno[3,4-c]pyrrol-4,6(5H)-dione

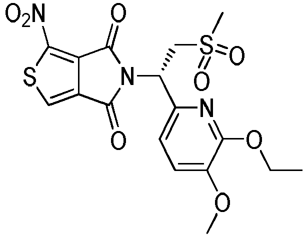

The compound was synthesized by referring to the method of Example 221.

ESI MS m/z: 456.05[M+1]⁺

### Example 223. (S)-1-amino-5-(1-(6-ethoxy-5-methoxypyridin-2-yl)-2-(methylsul fonyl)ethyl)-4H-thieno[3,4-c]pyrrol-4,6 (5H)-dione

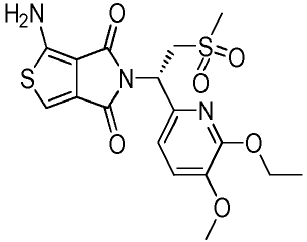

To a 250 mL round-bottom flask equipped with an electromagnetic stirrer, a reflux condenser and a drying tube were added 2.0 g of product of Example 134 and 100 mL of ethanol/water (2:1). The mixture was heated to reflux. 1.8 g of reductive iron powder was added, and the mixture was refluxed for 2 hours. The mixture was filtered with suction, and the filtrate was evaporated to dryness. 200 mL of ethyl acetate and 150 mL of water were added for extraction. The layers were separated. The organic layer was washed with 100 mL of water and 100 mL of saturated saline, dried over anhydrous sodium sulfate, and filtered. The filtrate was evaporated to remove the solvent, and purified by column chromatography to obtain 1.1 g of yellow-brown solid.

ESI MS m/z: 426.07[M+1]⁺

### Example 224. N-(5-(1-(6-ethoxy-5-methoxypyridin-2-yl)-2-(methylsulfonyl)eth yl)-4,6-dioxo-5,6-dihydro-4H-thieno[3,4-c]pyrrol-1-yl)acetamide

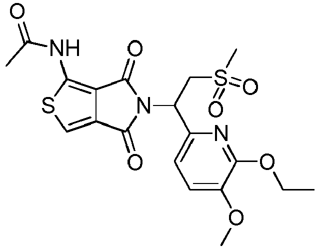

The target compound was obtained by acetylating the product of Example 22 3.

ESI MS m/z: 468.08[M+1]⁺

### Example 225. (R)-N-(5-(1-(6-ethoxy-5-methoxypyridin-2-yl)-2-(methylsulfonyl) ethyl)-4,6-dioxo-5,6-dihydro-4H-thieno[3,4-c]pyrrol-1-yl)acetamide

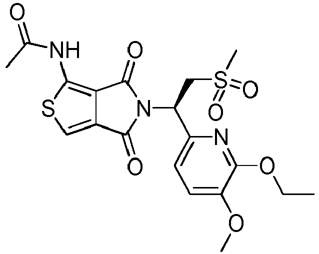

The compound was synthesized by referring to the method of Example 224.

ESI MS m/z: 468.08[M+1]⁺

### Example 226. (S)-N-(5-(1-(6-ethoxy-5-methoxypyridin-2-yl)-2-(methylsulfonyl) ethyl)-4,6-dioxo-5,6-dihydro-4H-thieno[3,4-c]pyrrol-1-yl)propionamide

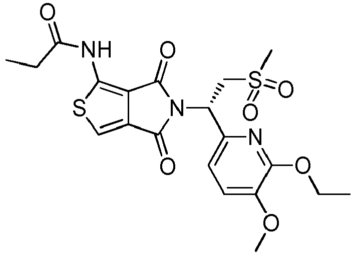

The compound was synthesized by referring to the method of Example 224.

ESI MS m/z: 482.10[M+1]⁺

### Example 227. (S)-N-(5-(1-(6-ethoxy-5-methoxypyridin-2-yl)-2-(methylsulfonyl) ethyl)-4,6-dioxo-5,6-dihydro-4H-thieno[3,4-c]pyrrol-1-yl)cyclopropanecarboxamide

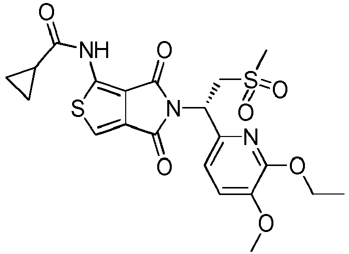

The compound was synthesized by referring to the method of Example 224.

ESI MS m/z: 494.10[M+1]⁺

### Example 228. (S)-2-(dimethylamino)-N-(5-(1-(6-ethoxy-5-methoxypyridin-2-yl) -2-(methylsulfonyl)ethyl)-4,6-dioxo-5,6-dihydro-4H-thieno[3,4-c]pyrrol-1-yl)acetamide

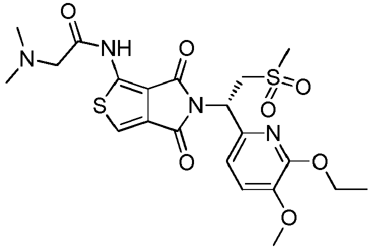

The compound was synthesized by referring to the method of Example 224.

ESI MS m/z: 511.12[M+1]⁺

### Example 229. (S)-2-(diethylamino)-N-(5-(1-(6-ethoxy-5-methoxypyridin-2-yl)-2 -(methylsulfonyl)ethyl)-4,6-dioxo-5,6-dihydro-4H-thieno[3,4-c]pyrrol-1-yl)acetamide

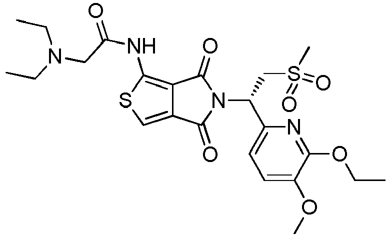

The compound was synthesized by referring to the method of Example 224.

ESI MS m/z: 539.16[M+1]⁺

### Example 230. (S)-N-(5-(1-(6-ethoxy-5-methoxypyridin-2-yl)-2-(methylsulfonyl) ethyl)-4,6-dioxo-5,6-dihydro-4H-thieno[3,4-c]pyrrol-1-yl)-2-(piperidin-1-yl)acetamide

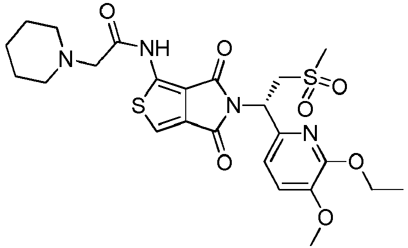

The compound was synthesized by referring to the method of Example 224.

ESI MS m/z: 551.16[M+1]⁺

### Example 231. (S)-N-(5-(1-(6-ethoxy-5-methoxypyridin-2-yl)-2-(methylsulfonyl) ethyl)-4,6-dioxo-5,6-dihydro-4H-thieno[3,4-c]pyrrol-1-yl)-2-(pyrrolidin-1-yl)acetamide

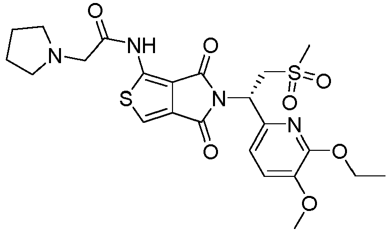

The compound was synthesized by referring to the method of Example 224.

ESI MS m/z: 537.14[M+1]⁺

### Example 232. (S)-N-(5-(1-(6-ethoxy-5-methoxypyridin-2-yl)-2-(methylsulfonyl) ethyl)-4,6-dioxo-5,6-dihydro-4H-thieno[3,4-c]pyrrol-1-yl)-2-morpholinoacetamide

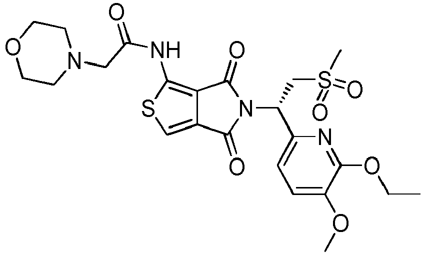

The compound was synthesized by referring to the method of Example 224.

ESI MS m/z: 553.13[M+1]⁺

### Example 233. PDE4 inhibitory activity assay

The compounds of the present disclosure were tested for their inhibitory activity (IC50 value) against PDE4A1A, PDE4B1, and PDE4D2.

### Assay materials:

Enzymes: PDE4A1A(BPS, CatNo. 60040); PDE4B1 (BPS, CatNo. 60041), and PDE4D2.

Positive compound: Trequinsin (Sigma, Cat No. T20S7).

Reaction plate: 384-well plate (Perkin Elmer, Cat No. 60077).

Instrument: Wallac Victor Muti-lable counter (Pekin Elmer)Steps of assay :
I. 1x reaction solution and reaction stopping solution were prepared;
II. PDE enzymatic reaction;
   1) PDE was dissolved in 1x reaction solution to prepare 2x enzyme solution;
   2) FAM-cAMP was dissolved in 1x reaction solution to prepare 2x substrate solution;
   3) Corresponding volume of the compound in DMSO was transferred to the reaction plate through Echo 550;
   4) 2x enzyme solution was added into the corresponding wells of the reaction plate and incubated with the compound solution for 15 minutes at room temperature;
   5) 2x substrate solution was added into the corresponding well of the reaction plate to initiate the reaction;
   6) After the reaction plate was incubated at room temperature for 30 min, the reaction was terminated by adding reaction stopping solution. The reaction plate was incubated at room temperature for another 60 minutes;
III. Read on Victor;
IV. Curve fitting;

Inhibition rates were calculated with Excel; and IC₅₀ for each compound was calculated with Graphpad prism.

| | PDE4 A1 | PDE4 B1 | PDE4 D2 | | PDE4 A1 | PDE4 B1 | PDE4 D2 | | PDE4 A1 | PDE4 B1 | PDE4 D2 |
|---|---|---|---|---|---|---|---|---|---|---|---|
| Ex. 1 | | | | Ex. 84 | + | + | + | Ex. 166 | + | + | + |
| Ex. 2 | 6.8 | 21 | 3.4 | Ex. 85 | + | + | + | Ex. 167 | + | + | + |
| Ex. 3 | 790 | 2180 | 415 | Ex. 86 | + | + | + | Ex. 168 | + | + | + |
| Ex. 4 | 17 | 54 | 8.5 | Ex. 87 | + | + | + | Ex. 169 | + | + | + |
| Ex. 5 | - | - | - | Ex. 88 | + | + | + | Ex. 170 | + | + | + |
| Ex. 6 | + | + | = | Ex. 89 | + | + | + | Ex. 171 | + | + | + |
| Ex. 7 | - | - | - | Ex. 90 | + | + | + | Ex. 172 | + | + | + |
| Ex. 8 | 4.5 | 15 | 3.8 | Ex. 91 | + | + | + | Ex. 173 | + | + | + |
| Ex. 9 | 530 | 1000 | 318 | Ex. 92 | + | + | + | Ex. 174 | + | + | + |
| Ex. 10 | 4.9 | 9.8 | 2.8 | Ex. 93 | + | + | + | Ex. 175 | + | + | + |
| Ex. 11 | + | + | + | Ex. 94 | + | + | + | Ex. 176 | + | + | + |
| Ex. 12 | 6.0 | 20 | 3.2 | Ex. 95 | + | + | + | Ex. 177 | + | + | + |
| Ex. 13 | 94 | 1244 | 396 | Ex. 96 | + | + | + | Ex. 178 | + | + | + |
| Ex. 14 | + | + | = | Ex. 97 | + | + | + | Ex. 179 | + | + | + |
| Ex. 15 | - | - | - | Ex. 98 | - | - | - | Ex. 180 | + | + | + |
| Ex. 16 | - | - | - | Ex. 99 | + | + | + | Ex. 181 | + | + | + |
| Ex. 17 | + | + | = | Ex. 100 | + | + | + | Ex. 182 | + | + | + |
| Ex. 18 | - | - | - | Ex. 101 | + | + | + | Ex. 183 | + | + | + |
| Ex. 19 | + | + | + | Ex. 102 | + | + | + | Ex. 184 | + | + | + |
| Ex. 20 | + | + | + | Ex. 103 | + | + | + | Ex. 185 | + | + | + |
| Ex. 22 | - | - | - | Ex. 104 | + | + | + | Ex. 186 | + | + | + |
| Ex. 23 | + | + | + | Ex. 105 | + | + | + | Ex. 187 | + | + | + |
| Ex. 24 | - | - | - | Ex. 106 | + | + | + | Ex. 188 | + | + | + |
| Ex. 25 | + | + | + | Ex. 107 | + | + | + | Ex. 189 | + | + | + |
| Ex. 26 | - | - | - | Ex. 108 | + | + | + | Ex. 190 | + | + | + |
| Ex. 27 | 6.7 | 13 | 3.7 | Ex. 109 | + | + | + | Ex. 191 | + | + | + |
| Ex. 28 | + | + | + | Ex. 110 | + | + | + | Ex. 192 | + | + | + |
| Ex. 29 | + | + | + | Ex. 111 | + | + | + | Ex. 193 | - | - | - |
| Ex. 30 | - | - | - | Ex. 112 | + | + | + | Ex. 194 | + | + | + |
| Ex. 31 | 3.6 | 6.8 | 4.1 | Ex. 113 | + | + | + | Ex. 195 | - | - | - |
| Ex. 32 | + | + | + | Ex. 114 | + | + | + | Ex. 196 | + | + | + |
| Ex. 33 | + | + | + | Ex. 115 | + | + | + | Ex. 197 | + | + | + |
| Ex. 34 | + | + | + | Ex. 116 | + | + | + | Ex. 198 | + | + | + |
| Ex. 35 | 4.1 | 8.5 | 3.4 | Ex. 117 | + | + | + | Ex. 199 | + | + | + |
| Ex. 36 | + | + | + | Ex. 118 | + | + | + | Ex. 200 | + | + | + |
| Ex. 37 | + | + | + | Ex. 119 | + | + | + | Ex. 201 | + | + | + |
| Ex. 38 | + | + | + | Ex. 120 | + | + | + | Ex. 202 | + | + | + |
| Ex. 39 | + | + | + | Ex. 121 | + | + | + | Ex. 203 | + | + | + |
| Ex. 40 | + | + | + | Ex. 122 | + | + | + | Ex. 204 | + | + | + |
| Ex. 41 | + | + | + | Ex. 123 | + | + | + | Ex. 205 | + | + | + |
| Ex. 42 | + | + | + | Ex. 124 | + | + | + | Ex. 206 | + | + | + |
| Ex. 43 | + | + | + | Ex. 125 | + | + | + | Ex. 207 | + | + | + |
| Ex. 44 | + | + | + | Ex. 126 | + | + | + | Ex. 208 | + | + | + |
| Ex. 45 | + | + | + | Ex. 127 | + | + | + | Ex. 209 | + | + | + |
| Ex. 46 | + | + | + | Ex. 128 | + | + | + | Ex. 210 | - | - | - |
| Ex. 47 | - | - | - | Ex. 129 | + | + | + | Ex. 211 | + | + | + |
| Ex. 48 | + | + | + | Ex. 130 | + | + | + | Ex. 212 | + | + | + |
| Ex. 49 | - | - | - | Ex. 131 | + | + | + | Ex. 213 | + | + | + |
| Ex. 50 | + | + | + | Ex. 132 | + | + | + | Ex. 214 | + | + | + |
| Ex. 51 | + | + | + | Ex. 133 | + | + | + | Ex. 215 | + | + | + |
| Ex. 52 | - | - | - | Ex. 134 | + | + | + | Ex. 216 | + | + | + |
| Ex. 53 | + | + | + | Ex. 135 | + | + | + | Ex. 217 | + | + | + |
| Ex. 54 | - | - | - | Ex. 136 | + | + | + | Ex. 218 | + | + | + |
| Ex. 55 | + | + | + | Ex. 137 | + | + | + | Ex. 219 | + | + | + |
| Ex. 56 | + | + | + | Ex. 138 | + | + | + | Ex. 220 | - | - | - |
| Ex. 57 | + | + | + | Ex. 139 | + | + | + | Ex. 221 | + | + | + |
| Ex. 58 | + | + | + | Ex. 140 | + | + | + | Ex. 222 | + | + | + |
| Ex. 59 | + | + | + | Ex. 141 | + | + | + | Ex. 223 | + | + | + |
| Ex. 60 | + | + | + | Ex. 142 | + | + | + | Ex. 224 | + | + | + |
| Ex. 61 | + | + | + | Ex. 143 | + | + | + | Ex. 225 | - | - | - |
| Ex. 62 | - | - | - | Ex. 144 | + | + | + | Ex. 226 | + | + | + |
| Ex. 63 | + | + | + | Ex. 145 | - | - | - | Ex. 227 | + | + | + |
| Ex. 64 | + | + | + | Ex. 146 | + | + | + | Ex. 228 | + | + | + |
| Ex. 65 | + | + | + | Ex. 147 | + | + | + | Ex. 229 | + | + | + |
| Ex. 66 | + | + | + | Ex. 148 | + | + | + | Ex. 230 | + | + | + |
| Ex. 67 | + | + | + | Ex. 149 | + | + | + | Ex. 231 | + | + | + |
| Ex. 68 | + | + | + | Ex. 150 | + | + | + | apremilast | 21 | 80 | 9.4 |
| Ex. 69 | + | + | + | Ex. 151 | + | + | + | ^{∗}WX063 | 4.2 | 12 | 1.0 |
| Ex. 70 | + | + | + | Ex. 152 | + | + | + | ^{∗}WX011 | 14 | 26 | 6.8 |
| Ex. 71 | + | + | + | Ex. 153 | + | + | + | | | | |
| Ex. 72 | + | + | + | Ex. 154 | + | + | + | | | | |
| Ex. 73 | + | + | + | Ex. 155 | + | + | + | | | | |
| Ex. 74 | + | + | + | Ex. 156 | + | + | + | | | | |
| Ex. 75 | + | + | + | Ex. 157 | + | + | + | | | | |
| Ex. 76 | + | + | + | Ex. 158 | + | + | + | | | | |
| Ex. 77 | + | + | + | Ex. 159 | + | + | + | | | | |
| Ex. 78 | + | + | + | Ex. 160 | + | + | + | | | | |
| Ex. 79 | 10 | 15 | 4.7 | Ex. 161 | + | + | + | | | | |
| Ex. 80 | 14 | 26 | 6.8 | Ex. 162 | + | + | + | | | | |
| Ex. 81 | + | + | + | Ex. 163 | + | + | + | | | | |
| Ex. 82 | + | + | + | Ex. 164 | + | + | + | | | | |
| Ex. 83 | + | + | + | Ex. 165 | + | + | + | | | | |

| | | | | | | | | | | | |
|---|---|---|---|---|---|---|---|---|---|---|---|
| ^{∗}WX011: Example 11 of WO2018036470, which was synthesized according to the method disclosed therein; ^{∗}WX063: Example 63 of WO2018036469, which was synthesized according to the method disclosed therein; +: significant inhibitory effect at 20nM; -: no significant inhibitory effect at 20nM; Ex.^{∗}: indicates Example ^{∗} | | | | | | | | | | | |

Conclusion: The Examples 2, 8, 10, 12, and 30 of the present disclosure had significantly enhanced inhibitory activity against PDE4A1A, PDE4B1, and PDE4D2, compared with Apremilast and Example 11 of WO2018036470 (WX011). Compounds of the present disclosure had lower inhibitory activity against PDE4D2 compared with Example 63 of WO2018036469 (WX063), indicating less vomiting side effect of compounds of the present disclosure (Osamu Suzuki et al. J. Pharmacol. Sci. 2013, 123: 219-226).

## Claims

1. A compound of formula (I) and an isomer, solvate, deuterated derivative or pharmaceutically acceptable salt thereof: 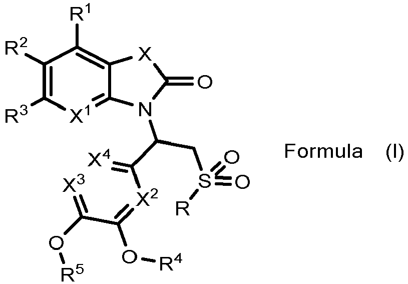 wherein:
X¹ is selected from N or CH;
one of X², X³, and X⁴ is selected from N, and the others are CH;
X is selected from O, -N(R⁶), and-CH(R⁶)-;
R is selected from hydrogen, C₁-C₃ alkyl, and C₃-C₆ cycloalkyl, and may be substituted with halogen;
R¹, R², and R³ are each independently selected from hydrogen, halogen, hydroxyl, -CN, -NH₂, C1-C6 alkyl, C1-6 alkoxy, C1-C6 alkylamino, C2-6 alkenyl, C3-6 cycloalkenyl, 3- to 6-membered heterocycloalkenyl, C3-C6 cycloalkyl, 3- to 6-membered heterocycloalkyl, phenyl, 5- to 6-membered heteroaryl, and -COOR⁸, which may be substituted with halogen, C1-C6 alkyl, optionally halogenated C1-C6 alkyl, C3-C6 cycloalkyl, or optionally halogenated C3-C6 cycloalkyl;
R⁴ and R⁵are independently selected from C1-C6 alkyl and C3-C6 cycloalkyl, and may be optionally substituted with deuterium, tritium, or halogen;
R⁶ is selected from H, halogen, hydroxyl, CN, -NH₂, COOH, and R⁷-L¹-, or selected from C1-6 alkyl, C1-C6 heteroalkyl, C3-C6 cycloalkyl, 3- to 6-membered heterocycloalkyl, phenyl, 5- to 6-membered heteroaryl, -COOR⁹, -SO₂R¹⁰, -SO₂NHR¹¹, and -NHCONHR¹², which may be substituted with halogen, C1-C6 alkyl, optionally halogenated C1-C6 alkyl, C3-C6 cycloalkyl, or optionally halogenated C3-C6 cycloalkyl;
R⁷ is selected from C3-C6 cycloalkyl, 3- to 6-membered heterocycloalkyl, 3- to 6-membered heterocycloalkenyl, phenyl, 5- to 6-membered heteroaryl, and -COOR⁸, and may be substituted with halogen, C1-C6 alkyl, or C3-C6 cycloalkyl;
L¹ is selected from -CH₂-, -CH₂CH₂-, O, S, and NH;
R⁸, R⁹, R¹⁰, R¹¹, and R¹² are C1-C6 alkyl, halogenated C1-C6 alkyl, or C3-C6 cycloalkyl.

2. The compound of formula (I) and the isomer, solvate, deuterated derivative or pharmaceutically acceptable salt thereof according to claim 1, wherein X is selected from:

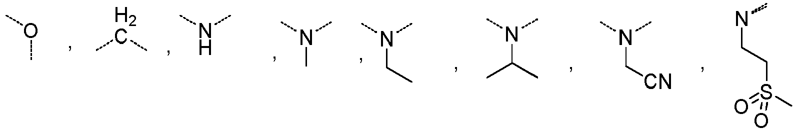

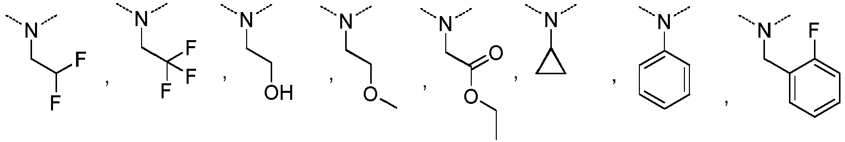

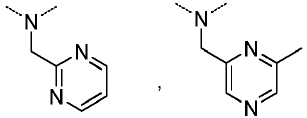

3. The compound of formula (I) and the isomer, solvate, deuterated derivative or pharmaceutically acceptable salt thereof according to claim 1 or 2, wherein R¹, R², and R³ are independently selected from the following groups: 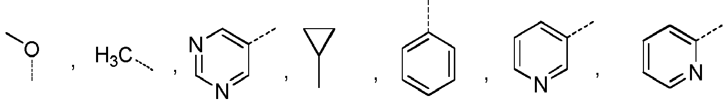 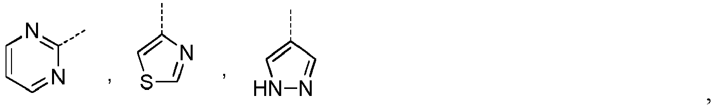 which may be substituted with halogen, -OH, -NH₂, -CN, C1-C6 alkyl, optionally halogenated C1-C6 alkyl, C3-C6 cycloalkyl, or optionally halogenated C3-C6 cycloalkyl.

4. The compound of formula (I) and the isomer, solvate, deuterated derivative or pharmaceutically acceptable salt thereof according to any one of claims 1-3, wherein the compound of formula (I) comprises the following structures:
3-(1-(6-ethoxy-5-methoxypyridin-2-yl)-2-(methylsulfonyl)ethyl)-6-(2-fluorophenyl)-7-methyl-1H-imidazo[4,5-b]pyridin-2(3H)-one;
(S)-3-(1-(6-ethoxy-5-methoxypyridin-2-yl)-2-(methylsulfonyl)ethyl)-6-(2-fluorophenyl) -7-methyl-1H-imidazo[4,5-b]pyridin-2(3H)-one;
(R)-3-(1-(6-ethoxy-5-methoxypyridin-2-yl)-2-(methylsulfonyl)ethyl)-6-(2-fluorophenyl) -7-methyl-1H-imidazo[4,5-b]pyridin-2(3H)-one;
(S)-3-(1-(6-ethoxy-5-methoxypyridin-2-yl)-2-(methylsulfonyl)ethyl)-7-methyl-6-phenyl -1H-imidazo[4,5-b]pyridin-2(3H)-one;
(R)-3-(1-(6-ethoxy-5-methoxypyridin-2-yl)-2-(methylsulfonyl)ethyl)-7-methyl-6-phenyl -1H-imidazo[4,5-b]pyridin-2(3H)-one;
(S)-3-(1-(6-ethoxy-5-methoxypyridin-2-yl)-2-(methylsulfonyl)ethyl)-6-(4-fluorophenyl) -7-methyl-1H-imidazo[4,5-b]pyridin-2(3H)-one;
(R)-3-(1-(6-ethoxy-5-methoxypyridin-2-yl)-2-(methylsulfonyl)ethyl)-6-(4-fluorophenyl) -7-methyl-1H-imidazo[4,5-b]pyridin-2(3H)-one;
(S)-3-(1-(6-ethoxy-5-methoxypyridin-2-yl)-2-(methylsulfonyl)ethyl)-7-methyl-6-(*o*-met hylphenyl)-1H-imidazo[4,5-b]pyridin-2(3H)-one;
(R)-3-(1-(6-ethoxy-5-methoxypyridin-2-yl)-2-(methylsulfonyl)ethyl)-7-methyl-6-(*o*-met hylphenyl)-1H-imidazo[4,5-b]pyridin-2(3H)-one;
(S)-3-(1-(6-ethoxy-5-methoxypyridin-2-yl)-2-(methylsulfonyl)ethyl)-6-(2-ethylphenyl)-7-methyl-1H-imidazo[4,5-b]pyridin-2(3H)-one;
(S)-3-(1-(6-ethoxy-5-methoxypyridin-2-yl)-2-(methylsulfonyl)ethyl)-6-(2-isopropylphen yl)-7-methyl-1H-imidazo[4,5-b]pyridin-2(3H)-one;
(S)-3-(1-(6-ethoxy-5-methoxypyridin-2-yl)-2-(methylsulfonyl)ethyl)-6-(2-methoxyphen yl)-7-methyl-1H-imidazo[4,5-b]pyridin-2(3H)-one;
(R)-3-(1-(6-ethoxy-5-methoxypyridin-2-yl)-2-(methylsulfonyl)ethyl)-6-(2-methoxyphen yl)-7-methyl-1H-imidazo[4,5-b]pyridin-2(3H)-one;
(S)-3-(1-(6-ethoxy-5-methoxypyridin-2-yl)-2-(methylsulfonyl)ethyl)-7-methyl-6-(2-(trifl uoromethyl)phenyl)-1H-imidazo[4,5-b]pyridin-2(3H)-one;
(S)-2-(3-(1-(6-ethoxy-5-methoxypyridin-2-yl)-2-(methylsulfonyl)ethyl)-7-methyl-2-oxo-2,3-dihydro-1H-imidazo[4,5-b]pyridin-6-yl)benzonitrile;
(R)-2-(3-(1-(6-ethoxy-5-methoxypyridin-2-yl)-2-(methylsulfonyl)ethyl)-7-methyl-2-oxo -2,3-dihydro-1H-imidazo[4,5-b]pyridin-6-yl)benzonitrile;
(S)-3-(1-(6-ethoxy-5-methoxypyridin-2-yl)-2-(methylsulfonyl)ethyl)-7-methyl-6-(pyridi n-2-yl)-1H-imidazo[4,5-b]pyridin-2(3H)-one;
(R)-3-(1-(6-ethoxy-5-methoxypyridin-2-yl)-2-(methylsulfonyl)ethyl)-7-methyl-6-(pyridi n-2-yl)-1H-imidazo[4,5-b]pyridin-2(3H)-one;
(S)-3-(1-(6-ethoxy-5-methoxypyridin-2-yl)-2-(methylsulfonyl)ethyl)-1,7-dimethyl-6-(py ridin-2-yl)-1H-imidazo[4,5-b]pyridin-2(3H)-one;
(S)-3-(1-(6-ethoxy-5-methoxypyridin-2-yl)-2-(methylsulfonyl)ethyl)-1-(2-methoxyethyl )-7-methyl-6-(pyridin-2-yl)-1H-imidazo[4,5-b]pyridin-2(3H)-one;
(S)-3-(1-(6-ethoxy-5-methoxypyridin-2-yl)-2-(methylsulfonyl)ethyl)-6-(2-fluorophenyl) -1,7-dimethyl-1H-imidazo[4,5-b]pyridin-2(3H)-one;
(R)-3-(1-(6-ethoxy-5-methoxypyridin-2-yl)-2-(methylsulfonyl)ethyl)-6-(2-fluorophenyl) -1,7-dimethyl-1H-imidazo[4,5-b]pyridin-2(3H)-one;
(S)-6-(2,6-difluorophenyl)-3-(1-(6-ethoxy-5-methoxypyridin-2-yl)-2-(methylsulfonyl)et hyl)-7-methyl-1H-imidazo[4,5-b]pyridin-2(3H)-one;
(R)(S)-6-(2,6-difluorophenyl)-3-(1-(6-ethoxy-5-methoxypyridin-2-yl)-2-(methylsulfonyl )ethyl)-7-methyl-1H-imidazo[4,5-b]pyridin-2(3H)-one;
(S)-6-(2,3-difluorophenyl)-3-(1-(6-ethoxy-5-methoxypyridin-2-yl)-2-(methylsulfonyl)et hyl)-7-methyl-1H-imidazo[4,5-b]pyridin-2(3H)-one;
(R)-6-(2,3-difluorophenyl)-3-(1-(6-ethoxy-5-methoxypyridin-2-yl)-2-(methylsulfonyl)et hyl)-7-dimethyl-1H-imidazo[4,5-b]pyridin-2(3H)-one;
(S)-6-(2,5-difluorophenyl)-3-(1-(6-ethoxy-5-methoxypyridin-2-yl)-2-(methylsulfonyl)et hyl)-7-methyl-1H-imidazo[4,5-b]pyridin-2(3H)-one;
(S)-6-(2,4-difluorophenyl)-3-(1-(6-ethoxy-5-methoxypyridin-2-yl)-2-(methylsulfonyl)et hyl)-7-methyl-1H-imidazo[4,5-b]pyridin-2(3H)-one;
(S)-6-(2,4,5-trifluorophenyl)-3-(1-(6-ethoxy-5-methoxypyridin-2-yl)-2-(methylsulfonyl) ethyl)-7-methyl-1H-imidazo[4,5-b]pyridin-2(3H)-one;
(R)-6-(2,5-difluorophenyl)-3-(1-(6-ethoxy-5-methoxypyridin-2-yl)-2-(methylsulfonyl)et hyl)-7-methyl-1H-imidazo[4,5-b]pyridin-2(3H)-one;
(S)-6-(2,4-dimethylphenyl)-3-(1-(6-ethoxy-5-methoxypyridin-2-yl)-2-(methylsulfonyl)et hyl)-7-methyl-1H-imidazo[4,5-b]pyridin-2(3H)-one;
(S)-6-(2,5-dimethylphenyl)-3-(1-(6-ethoxy-5-methoxypyridin-2-yl)-2-(methylsulfonyl)et hyl)-7-methyl-1H-imidazo[4,5-b]pyridin-2(3H)-one;
(S)-6-(2,3-dimethylphenyl)-3-(1-(6-ethoxy-5-methoxypyridin-2-yl)-2-(methylsulfonyl)et hyl)-7-methyl-1H-imidazo[4,5-b]pyridin-2(3H)-one;
(S)-6-(2,6-dimethylphenyl)-3-(1-(6-ethoxy-5-methoxypyridin-2-yl)-2-(methylsulfonyl)et hyl)-7-methyl-1H-imidazo[4,5-b]pyridin-2(3H)-one;
(S)-6-(4-fluoro-2-methylphenyl)-3-(1-(6-ethoxy-5-methoxypyridin-2-yl)-2-(methylsulfo nyl)ethyl)-7-methyl-1H-imidazo[4,5-b]pyridin-2(3H)-one;
(S)-6-(5-fluoro-2-methylphenyl)-3-(1-(6-ethoxy-5-methoxypyridin-2-yl)-2-(methylsulfo nyl)ethyl)-7-methyl-1H-imidazo[4,5-b]pyridin-2(3H)-one;
(S)-6-(2-fluoro-5-methylphenyl)-3-(1-(6-ethoxy-5-methoxypyridin-2-yl)-2-(methylsulfo nyl)ethyl)-7-methyl-1H-imidazo[4,5-b]pyridin-2(3H)-one;
(S)-6-(2-fluoro-3-methylphenyl)-3-(1-(6-ethoxy-5-methoxypyridin-2-yl)-2-(methylsulfo nyl)ethyl)-7-methyl-1H-imidazo[4,5-b]pyridin-2(3H)-one;
(S)-6-(2-fluoro-4-methylphenyl)-3-(1-(6-ethoxy-5-methoxypyridin-2-yl)-2-(methylsulfo nyl)ethyl)-7-methyl-1H-imidazo[4,5-b]pyridin-2(3H)-one;
(S)-6-(2-fluoro-5-methoxyphenyl)-3-(1-(6-ethoxy-5-methoxypyridin-2-yl)-2-(methylsulf onyl)ethyl)-7-methyl-1H-imidazo[4,5-b]pyridin-2(3H)-one;
(S)-6-(2-fluoro-3-methoxyphenyl)-3-(1-(6-ethoxy-5-methoxypyridin-2-yl)-2-(methylsulf onyl)ethyl)-7-methyl-1H-imidazo[4,5-b]pyridin-2(3H)-one;
(S)-6-(2-fluoro-4-methoxyphenyl)-3-(1-(6-ethoxy-5-methoxypyridin-2-yl)-2-(methylsulf onyl)ethyl)-7-methyl-1H-imidazo[4,5-b]pyridin-2(3H)-one;
(S)-6-(5-fluoro-2-methoxyphenyl)-3-(1-(6-ethoxy-5-methoxypyridin-2-yl)-2-(methylsulf onyl)ethyl)-7-methyl-1H-imidazo[4,5-b]pyridin-2(3H)-one;
(S)-6-(4-fluoro-2-methoxyphenyl)-3-(1-(6-ethoxy-5-methoxypyridin-2-yl)-2-(methylsulf onyl)ethyl)-7-methyl-1H-imidazo[4,5-b]pyridin-2(3H)-one;
(S)-6-(6-fluoro-2-methoxyphenyl)-3-(1-(6-ethoxy-5-methoxypyridin-2-yl)-2-(methylsulf onyl)ethyl)-7-methyl-1H-imidazo[4,5-b]pyridin-2(3H)-one;
(R)-3-(1-(6-ethoxy-5-methoxypyridin-2-yl)-2-(methylsulfonyl)ethyl)-1-ethyl-7-methyl-6 -phenyl-1H-imidazo[4,5-b]pyridin-2(3H)-one;
(S)-3-(1-(6-ethoxy-5-methoxypyridin-2-yl)-2-(methylsulfonyl)ethyl)-1-ethyl-6-(4-fluoro phenyl)-7-methyl-1H-imidazo[4,5-b]pyridin-2(3H)-one;
(R)-3-(1-(6-ethoxy-5-methoxypyridin-2-yl)-2-(methylsulfonyl)ethyl)-1-ethyl-6-(4-fluoro phenyl)-7-methyl-1H-imidazo[4,5-b]pyridin-2(3H)-one;
(S)-3-(1-(6-ethoxy-5-methoxypyridin-2-yl)-2-(methylsulfonyl)ethyl)-1-ethyl-7-methyl-6 -(*o*-methylphenyl)-1H-imidazo[4,5-b]pyridin-2(3H)-one;
(S)-3-(1-(6-ethoxy-5-methoxypyridin-2-yl)-2-(methylsulfonyl)ethyl)-6-(2-fluorophenyl) -7-methyl-1-(2,2,2-trifluoroethyl)-1H-imidazo[4,5-b]pyridin-2(3H)-one;
(R)-3-(1-(6-ethoxy-5-methoxypyridin-2-yl)-2-(methylsulfonyl)ethyl)-6-(2-fluorophenyl) -7-methyl-1-(2,2,2-trifluoroethyl)-1H-imidazo[4,5-b]pyridin-2(3H)-one;
(S)-3-(1-(6-ethoxy-5-methoxypyridin-2-yl)-2-(methylsulfonyl)ethyl)-7-methyl-6-phenyl -1-(2,2,2-trifluoroethyl)-1H-imidazo[4,5-b]pyridin-2(3H)-one;
(R)-3-(1-(6-ethoxy-5-methoxypyridin-2-yl)-2-(methylsulfonyl)ethyl)-7-methyl-6-phenyl -1-(2,2,2-trifluoroethyl)-1H-imidazo[4,5-b]pyridin-2(3H)-one;
(S)-3-(1-(6-ethoxy-5-methoxypyridin-2-yl)-2-(methylsulfonyl)ethyl)-7-methyl-6-(*o*-met hylphenyl)-1-(2,2,2-trifluoroethyl)-1H-imidazo[4,5-b]pyridin-2(3H)-one;
(S)-1-(2,2-difluoroethyl)-3-(1-(6-ethoxy-5-methoxypyridin-2-yl)-2-(methylsulfonyl)ethy l)-6-(2-fluorophenyl)-7-methyl-1H-imidazo[4,5-b]pyridin-2(3H)-one;
(S)-1-(2,2-difluoroethyl)-3-(1-(6-ethoxy-5-methoxypyridin-2-yl)-2-(methylsulfonyl)ethy l)-7-methyl-6-phenyl-1H-imidazo[4,5-b]pyridin-2(3H)-one;
(S)-1-(2,2-difluoroethyl)-3-(1-(6-ethoxy-5-methoxypyridin-2-yl)-2-(methylsulfonyl)ethy l)-6-(4-fluorophenyl)-7-methyl-1H-imidazo[4,5-b]pyridin-2(3H)-one;
(S)-1-(2,2-difluoroethyl)-3-(1-(6-ethoxy-5-methoxypyridin-2-yl)-2-(methylsulfonyl)ethy l)-7-methyl-6-(*o*-methylphenyl)-1H-imidazo[4,5-b]pyridin-2(3H)-one;
(S)-3-(1-(5-(difluoromethoxy)-6-ethoxypyridin-2-yl)-2-(methylsulfonyl)ethyl)-6-(2-fluo rophenyl)-7-methyl-1H-imidazo[4,5-b]pyridin-2(3H)-one;
(S)-3-(1-(5-(difluoromethoxy)-6-ethoxypyridin-2-yl)-2-(methylsulfonyl)ethyl)-7-methyl -6-phenyl-1H-imidazo[4,5-b]pyridin-2(3H)-one;
(R)-3-(1-(5-(difluoromethoxy)-6-ethoxypyridin-2-yl)-2-(methylsulfonyl)ethyl)-7-methyl -6-phenyl-1H-imidazo[4,5-b]pyridin-2(3H)-one;
(S)-3-(1-(5-(difluoromethoxy)-6-ethoxypyridin-2-yl)-2-(methylsulfonyl)ethyl)-6-(4-fluo rophenyl)-7-methyl-1H-imidazo[4,5-b]pyridin-2(3H)-one;
(S)-3-(1-(5-(difluoromethoxy)-6-ethoxypyridin-2-yl)-2-(methylsulfonyl)ethyl)-7-methyl -6-(*o*-methylphenyl)-1H-imidazo[4,5-b]pyridin-2(3H)-one;
(S)-3-(1-(5-(difluoromethoxy)-6-ethoxypyridin-2-yl)-2-(methylsulfonyl)ethyl)-6-(2-fluo rophenyl)-1,7-dimethyl-1H-imidazo[4,5-b]pyridin-2(3H)-one;
(S)-3-(1-(5-(difluoromethoxy)-6-ethoxypyridin-2-yl)-2-(methylsulfonyl)ethyl)-1,7-dimet hyl-6-phenyl-1H-imidazo[4,5-b]pyridin-2(3H)-one;
(S)-3-(1-(5-(difluoromethoxy)-6-ethoxypyridin-2-yl)-2-(methylsulfonyl)ethyl)-6-(4-fluo rophenyl)-1,7-dimethyl-1H-imidazo[4,5-b]pyridin-2(3H)-one;
(S)-3-(1-(5-(difluoromethoxy)-6-ethoxypyridin-2-yl)-2-(methylsulfonyl)ethyl)-1,7-dimet hyl-6-(*o*-methylphenyl)-1H-imidazo[4,5-b]pyridin-2(3H)-one;
(S)-1-(2,2-difluoroethyl)-3-(1-(6-ethoxy-5-isopropoxypyridin-2-yl)-2-(methylsulfonyl)et hyl)-6-(2-fluorophenyl)-7-methyl-1H-imidazo[4,5-b]pyridin-2(3H)-one;
(S)-1-(2,2-difluoroethyl)-3-(1-(6-ethoxy-5-isopropoxypyridin-2-yl)-2-(methylsulfonyl)et hyl)-7-methyl-6-phenyl-1H-imidazo[4,5-b]pyridin-2(3H)-one;
(S)-1-(2,2-difluoroethyl)-3-(1-(6-ethoxy-5-isopropoxypyridin-2-yl)-2-(methylsulfonyl)et hyl)-6-(4-fluorophenyl)-7-methyl-1H-imidazo[4,5-b]pyridin-2(3H)-one;
(S)-1-(2,2-difluoroethyl)-3-(1-(6-ethoxy-5-isopropoxypyridin-2-yl)-2-(methylsulfonyl)et hyl)-7-methyl-6-(o-methylphenyl)-1H-imidazo[4,5-b]pyridin-2(3H)-one;
(S)-3-(1-(6-ethoxy-5-methoxypyridin-2-yl)-2-(methylsulfonyl)ethyl)-6-bromo-7-methyl-1H-imidazo[4,5-b]pyridin-2(3H)-one;
(S)-3-(1-(6-ethoxy-5-methoxypyridin-2-yl)-2-(methylsulfonyl)ethyl)-6-(2-fluorophenyl) -1H-imidazo[4,5-b]pyridin-2(3H)-one;
(S)-3-(1-(6-ethoxy-5-methoxypyridin-2-yl)-2-(methylsulfonyl)ethyl)-6-(2-fluorophenyl) -1-methyl-1H-imidazo[4,5-b]pyridin-2(3H)-one;
(S)-3-(1-(6-ethoxy-5-methoxypyridin-2-yl)-2-(methylsulfonyl)ethyl)-6-phenyl-1H-imida zo[4,5-b]pyridin-2(3H)-one;
(S)-3-(1-(6-ethoxy-5-methoxypyridin-2-yl)-2-(methylsulfonyl)ethyl)-6-phenyl-1-methyl -1H-imidazo[4,5-b]pyridin-2(3H)-one;
(S)-3-(1-(6-ethoxy-5-methoxypyridin-2-yl)-2-(methylsulfonyl)ethyl)-6-(4-fluorophenyl) -1H-imidazo[4,5-b]pyridin-2(3H)-one;
(S)-3-(1-(6-ethoxy-5-methoxypyridin-2-yl)-2-(methylsulfonyl)ethyl)-6-(*o*-methylphenyl )-1H-imidazo[4,5-b]pyridin-2(3H)-one;
(S)-3-(1-(6-ethoxy-5-methoxypyridin-2-yl)-2-(methylsulfonyl)ethyl)-1-methyl-6-(*o*-met hylphenyl)-1H-imidazo[4,5-b]pyridin-2(3H)-one;
(S)-3-(1-(6-ethoxy-5-methoxypyridin-2-yl)-2-(methylsulfonyl)ethyl)-6-(2-ethylphenyl)-1H-imidazo[4,5-b]pyridin-2(3H)-one;
(S)-3-(1-(6-ethoxy-5-methoxypyridin-2-yl)-2-(methylsulfonyl)ethyl)-6-(2-ethylphenyl)-1-methyl-1H-imidazo[4,5-b]pyridin-2(3H)-one;
(S)-3-(1-(6-ethoxy-5-methoxypyridin-2-yl)-2-(methylsulfonyl)ethyl)-6-(2-methoxyphen yl)-1H-imidazo[4,5-b]pyridin-2(3H)-one;
(S)-3-(1-(6-ethoxy-5-methoxypyridin-2-yl)-2-(methylsulfonyl)ethyl)-6-(2-methoxyphen yl)-1-methyl-1H-imidazo[4,5-b]pyridin-2(3H)-one;
(S)-3-(1-(6-ethoxy-5-methoxypyridin-2-yl)-2-(methylsulfonyl)ethyl)-6-(2-(trifluorometh yl)phenyl)-1H-imidazo[4,5-b]pyridin-2(3H)-one;
(S)-3-(1-(6-ethoxy-5-methoxypyridin-2-yl)-2-(methylsulfonyl)ethyl)-6-(2-(trifluorometh yl)phenyl)-1-methyl-1H-imidazo[4,5-b]pyridin-2(3H)-one;
(S)-6-(2-cyclopropylphenyl)-3-(1-(6-ethoxy-5-methoxypyridin-2-yl)-2-(methylsulfonyl) ethyl)-1H-imidazo[4,5-b]pyridin-2(3H)-one;
(S)-6-(2,6-difluorophenyl)-3-(1-(6-ethoxy-5-methoxypyridin-2-yl)-2-(methylsulfonyl)et hyl)-1H-imidazo[4,5-b]pyridin-2(3H)-one;
(S)-6-(2,6-difluorophenyl)-3-(1-(6-ethoxy-5-methoxypyridin-2-yl)-2-(methylsulfonyl)et hyl)-1-methyl-1H-imidazo[4,5-b]pyridin-2(3H)-one;
(S)-6-(2,3-difluorophenyl)-3-(1-(6-ethoxy-5-methoxypyridin-2-yl)-2-(methylsulfonyl)et hyl)-1H-imidazo[4,5-b]pyridin-2(3H)-one;
(S)-6-(2,3-difluorophenyl)-3-(1-(6-ethoxy-5-methoxypyridin-2-yl)-2-(methylsulfonyl)et hyl)-1-methyl-1H-imidazo[4,5-b]pyridin-2(3H)-one;
(S)-6-(2,5-difluorophenyl)-3-(1-(6-ethoxy-5-methoxypyridin-2-yl)-2-(methylsulfonyl)et hyl)-1H-imidazo[4,5-b]pyridin-2(3H)-one;
(S)-6-(2,5-difluorophenyl)-3-(1-(6-ethoxy-5-methoxypyridin-2-yl)-2-(methylsulfonyl)et hyl)-1-methyl-1H-imidazo[4,5-b]pyridin-2(3H)-one;
(S)-6-(2,4-difluorophenyl)-3-(1-(6-ethoxy-5-methoxypyridin-2-yl)-2-(methylsulfonyl)et hyl)-1H-imidazo[4,5-b]pyridin-2(3H)-one;
(S)-6-(2,4-difluorophenyl)-3-(1-(6-ethoxy-5-methoxypyridin-2-yl)-2-(methylsulfonyl)et hyl)-1-methyl-1H-imidazo[4,5-b]pyridin-2(3H)-one;
(S)-6-(2,4,5-trifluorophenyl)-3-(1-(6-ethoxy-5-methoxypyridin-2-yl)-2-(methylsulfonyl) ethyl)-1H-imidazo[4,5-b]pyridin-2(3H)-one;
(S)-6-(2,4,5-trifluorophenyl)-3-(1-(6-ethoxy-5-methoxypyridin-2-yl)-2-(methylsulfonyl) ethyl)-1-methyl-1H-imidazo[4,5-b]pyridin-2(3H)-one;
(R)-6-(2,5-difluorophenyl)-3-(1-(6-ethoxy-5-methoxypyridin-2-yl)-2-(methylsulfonyl)et hyl)-1H-imidazo[4,5-b]pyridin-2(3H)-one;
(S)-6-(2,4-dimethylphenyl)-3-(1-(6-ethoxy-5-methoxypyridin-2-yl)-2-(methylsulfonyl)et hyl)-1H-imidazo[4,5-b]pyridin-2(3H)-one;
(S)-6-(2,4-dimethylphenyl)-3-(1-(6-ethoxy-5-methoxypyridin-2-yl)-2-(methylsulfonyl)et hyl)-1-methyl-1H-imidazo[4,5-b]pyridin-2(3H)-one;
(S)-6-(2,5-dimethylphenyl)-3-(1-(6-ethoxy-5-methoxypyridin-2-yl)-2-(methylsulfonyl)et hyl)-1H-imidazo[4,5-b]pyridin-2(3H)-one;
(S)-6-(2,5-dimethylphenyl)-3-(1-(6-ethoxy-5-methoxypyridin-2-yl)-2-(methylsulfonyl)et hyl)-1-methyl-1H-imidazo[4,5-b]pyridin-2(3H)-one;
(S)-6-(2,3-dimethylphenyl)-3-(1-(6-ethoxy-5-methoxypyridin-2-yl)-2-(methylsulfonyl)et hyl)-1H-imidazo[4,5-b]pyridin-2(3H)-one;
(S)-6-(2,3-dimethylphenyl)-3-(1-(6-ethoxy-5-methoxypyridin-2-yl)-2-(methylsulfonyl)et hyl)-1-methyl-1H-imidazo[4,5-b]pyridin-2(3H)-one;
(S)-6-(2,6-dimethylphenyl)-3-(1-(6-ethoxy-5-methoxypyridin-2-yl)-2-(methylsulfonyl)et hyl)-1H-imidazo[4,5-b]pyridin-2(3H)-one;
(S)-6-(2,6-dimethylphenyl)-3-(1-(6-ethoxy-5-methoxypyridin-2-yl)-2-(methylsulfonyl)et hyl)-1-methyl-1H-imidazo[4,5-b]pyridin-2(3H)-one;
(S)-6-(4-fluoro-2-methylphenyl)-3-(1-(6-ethoxy-5-methoxypyridin-2-yl)-2-(methylsulfo nyl)ethyl)-1H-imidazo[4,5-b]pyridin-2(3H)-one;
(S)-6-(4-fluoro-2-methylphenyl)-3-(1-(6-ethoxy-5-methoxypyridin-2-yl)-2-(methylsulfo nyl)ethyl)-1-methyl-1H-imidazo[4,5-b]pyridin-2(3H)-one;
(S)-6-(5-fluoro-2-methylphenyl)-3-(1-(6-ethoxy-5-methoxypyridin-2-yl)-2-(methylsulfo nyl)ethyl)-1H-imidazo[4,5-b]pyridin-2(3H)-one;
(S)-6-(5-fluoro-2-methylphenyl)-3-(1-(6-ethoxy-5-methoxypyridin-2-yl)-2-(methylsulfo nyl)ethyl)-1-methyl-1H-imidazo[4,5-b]pyridin-2(3H)-one;
(S)-6-(3-fluoro-2-methylphenyl)-3-(1-(6-ethoxy-5-methoxypyridin-2-yl)-2-(methylsulfo nyl)ethyl)-1H-imidazo[4,5-b]pyridin-2(3H)-one;
(S)-6-(3-fluoro-2-methylphenyl)-3-(1-(6-ethoxy-5-methoxypyridin-2-yl)-2-(methylsulfo nyl)ethyl)-1-methyl-1H-imidazo[4,5-b]pyridin-2(3H)-one;
(S)-6-(2-fluoro-5-methylphenyl)-3-(1-(6-ethoxy-5-methoxypyridin-2-yl)-2-(methylsulfo nyl)ethyl)-1H-imidazo[4,5-b]pyridin-2(3H)-one;
(S)-6-(2-fluoro-5-methylphenyl)-3-(1-(6-ethoxy-5-methoxypyridin-2-yl)-2-(methylsulfo nyl)ethyl)-1-methyl-1H-imidazo[4,5-b]pyridin-2(3H)-one;
(S)-6-(2-fluoro-4-methylphenyl)-3-(1-(6-ethoxy-5-methoxypyridin-2-yl)-2-(methylsulfo nyl)ethyl)-1H-imidazo[4,5-b]pyridin-2(3H)-one;
(S)-6-(2-fluoro-4-methylphenyl)-3-(1-(6-ethoxy-5-methoxypyridin-2-yl)-2-(methylsulfo nyl)ethyl)-1-methyl-1H-imidazo[4,5-b]pyridin-2(3H)-one;
(S)-6-(5-fluoro-2-methoxyphenyl)-3-(1-(6-ethoxy-5-methoxypyridin-2-yl)-2-(methylsulf onyl)ethyl)-1H-imidazo[4,5-b]pyridin-2(3H)-one;
(S)-6-(5-fluoro-2-methoxyphenyl)-3-(1-(6-ethoxy-5-methoxypyridin-2-yl)-2-(methylsulf onyl)ethyl)-1-methyl-1H-imidazo[4,5-b]pyridin-2(3H)-one;
(S)-6-(4-fluoro-2-methoxyphenyl)-3-(1-(6-ethoxy-5-methoxypyridin-2-yl)-2-(methylsulf onyl)ethyl)-1H-imidazo[4,5-b]pyridin-2(3H)-one;
(S)-6-(4-fluoro-2-methoxyphenyl)-3-(1-(6-ethoxy-5-methoxypyridin-2-yl)-2-(methylsulf onyl)ethyl)-1-methyl-1H-imidazo[4,5-b]pyridin-2(3H)-one;
(S)-6-(6-fluoro-2-methoxyphenyl)-3-(1-(6-ethoxy-5-methoxypyridin-2-yl)-2-(methylsulf onyl)ethyl)-1H-imidazo[4,5-b]pyridin-2(3H)-one;
(S)-6-(pyridin-2-yl)-3-(1-(6-ethoxy-5-methoxypyridin-2-yl)-2-(methylsulfonyl)ethyl)-1 H-imidazo[4,5-b]pyridin-2(3H)-one;
(S)-3-(1-(6-ethoxy-5-methoxypyridin-2-yl)-2-(methylsulfonyl)ethyl)-1-methyl-6-(pyridi n-2-yl)-1H-imidazo[4,5-b]pyridin-2(3H)-one;
(S)-3-(1-(6-ethoxy-5-methoxypyridin-2-yl)-2-(methylsulfonyl)ethyl)-1-ethyl-6-(pyridin-2-yl)-1H-imidazo[4,5-b]pyridin-2(3H)-one;
(S)-3-(1-(6-ethoxy-5-methoxypyridin-2-yl)-2-(methylsulfonyl)ethyl)-1-(2-methoxyethyl )-6-(pyridin-2-yl)-1H-imidazo[4,5-b]pyridin-2(3H)-one;
(S)-3-(1-(6-ethoxy-5-methoxypyridin-2-yl)-2-(methylsulfonyl)ethyl)-6-(2-fluorophenyl) -1-methyl-1H-imidazo[4,5-b]pyridin-2(3H)-one;
(S)-3-(1-(6-ethoxy-5-methoxypyridin-2-yl)-2-(methylsulfonyl)ethyl)-1-methyl-6-phenyl -1H-imidazo[4,5-b]pyridin-2(3H)-one;
(S)-3-(1-(6-ethoxy-5-methoxypyridin-2-yl)-2-(methylsulfonyl)ethyl)-6-(4-fluorophenyl) -1-methyl-1H-imidazo[4,5-b]pyridin-2(3H)-one;
(S)-5-bromo-1-(1-(6-ethoxy-5-methoxypyridin-2-yl)-2-(methylsulfonyl)ethyl)-1H-benzo [d]imidazol-2(3H)-one;
(S)-1-(1-(1-(6-ethoxy-5-methoxypyridin-2-yl)-2-(methylsulfonyl)ethyl)-5-phenyl-1H-be nzo[d]imidazol-2(3H)-one;
(S)-1-(1-(1-(6-ethoxy-5-methoxypyridin-2-yl)-2-(methylsulfonyl)ethyl)-5-phenyl-3-met hyl-1H-benzo[d]imidazol-2(3H)-one;
(S)-1-(1-(1-(6-ethoxy-5-methoxypyridin-2-yl)-2-(methylsulfonyl)ethyl)-5-(2-fluorophen yl)-1H-benzo[d]imidazol-2(3H)-one;
(S)-1-(1-(1-(6-ethoxy-5-methoxypyridin-2-yl)-2-(methylsulfonyl)ethyl)-5-(2-fluorophen yl)-3 -methyl-1H-benzo[d]imidazol-2(3H)-one;
(R)-1-(1-(1-(6-ethoxy-5-methoxypyridin-2-yl)-2-(methylsulfonyl)ethyl)-5-(2-fluorophen yl)-1H-benzo[d]imidazol-2(3H)-one;
(S)-1-(1-(1-(6-ethoxy-5-methoxypyridin-2-yl)-2-(methylsulfonyl)ethyl)-5-(4-fluorophen yl)-1H-benzo[d]imidazol-2(3H)-one;
(R)-1-(1-(1-(6-ethoxy-5-methoxypyridin-2-yl)-2-(methylsulfonyl)ethyl)-5-(4-fluorophen yl)-1H-benzo[d]imidazol-2(3H)-one;
(S)-1-(1-(1-(6-ethoxy-5-methoxypyridin-2-yl)-2-(methylsulfonyl)ethyl)-3-methyl-5-(4-fl uorophenyl)-1H-benzo[d]imidazol-2(3H)-one;
(R)-1-(1-(1-(6-ethoxy-5-methoxypyridin-2-yl)-2-(methylsulfonyl)ethyl)-3-methyl-5-(4-f luorophenyl)-1H-benzo[d]imidazol-2(3H)-one;
(S)-1-(1-(6-ethoxy-5-methoxypyridin-2-yl)-2-(methylsulfonyl)ethyl)-5-(o-methylphenyl )-1H-benzo[d]imidazol-2(3H)-one;
(S)-1-(1-(6-ethoxy-5-methoxypyridin-2-yl)-2-(methylsulfonyl)ethyl)-5-(o-methylphenyl )-3-methyl-1H-benzo[d]imidazol-2(3H)-one;
(R)-1-(1-(6-ethoxy-5-methoxypyridin-2-yl)-2-(methylsulfonyl)ethyl)-5-(o-methylphenyl )-1H-benzo[d]imidazol-2(3H)-one;
(S)-5-(2-chlorophenyl)-1-(1-(6-ethoxy-5-methoxypyridin-2-yl)-2-(methylsulfonyl)ethyl) -1H-benzo[d]imidazol-2(3H)-one;
(S)-1-(1-(1-(6-ethoxy-5-methoxypyridin-2-yl)-2-(methylsulfonyl)ethyl)-5-(2-methoxyph enyl)-1H-benzo[d]imidazol-2(3H)-one;
(S)-1-(1-(1-(6-ethoxy-5-methoxypyridin-2-yl)-2-(methylsulfonyl)ethyl)-5-(2-methoxyph enyl)-3-methyl-1H-benzo[d]imidazol-2(3H)-one;
(R)-1-(1-(1-(6-ethoxy-5-methoxypyridin-2-yl)-2-(methylsulfonyl)ethyl)-5-(2-methoxyph enyl)-1H-benzo[d]imidazol-2(3H)-one;
(S)-1-(1-(1-(6-ethoxy-5-methoxypyridin-2-yl)-2-(methylsulfonyl)ethyl)-5-(2-(trifluorom ethyl)phenyl)-1H-benzo[d]imidazol-2(3H)-one;
(S)-1-(1-(1-(6-ethoxy-5-methoxypyridin-2-yl)-2-(methylsulfonyl)ethyl)-5-(2-(trifluorom ethyl)phenyl)-3-methyl-1H-benzo[d]imidazol-2(3H)-one;
(S)-5-(2-cyclopropylphenyl)-1-(1-(6-ethoxy-5-methoxypyridin-2-yl)-2-(methylsulfonyl) ethyl)-1H-benzo[d]imidazol-2(3H)-one;
(S)-1-(1-(1-(6-ethoxy-5-methoxypyridin-2-yl)-2-(methylsulfonyl)ethyl)-5-(2-isopropylp henyl)-4-methyl-1H-benzo[d]imidazol-2(3H)-one;
(S)-1-(1-(6-ethoxy-5-methoxypyridin-2-yl)-2-(methylsulfonyl)ethyl)-5-(2-ethylphenyl)-1H-benzo[d]imidazol-2(3H)-one;
(S)-1-(1-(6-ethoxy-5-methoxypyridin-2-yl)-2-(methylsulfonyl)ethyl)-5-(2-ethylphenyl)-3 -methyl-1H-benzo[d]imidazol-2(3H)-one;
(S)-5-(2,3-difluorophenyl)-1-(1-(6-ethoxy-5-methoxypyridin-2-yl)-2-(methylsulfonyl)et hyl)-1H-benzo[d]imidazol-2(3H)-one;
(S)-5-(2,3-difluorophenyl)-1-(1-(6-ethoxy-5-methoxypyridin-2-yl)-2-(methylsulfonyl)et hyl)-3 -methyl-1H-benzo[d]imidazol-2(3H)-one;
(S)-5-(2,6-difluorophenyl)-1-(1-(6-ethoxy-5-methoxypyridin-2-yl)-2-(methylsulfonyl)et hyl)-1H-benzo[d]imidazol-2(3H)-one;
(S)-5-(2,6-difluorophenyl)-1-(1-(6-ethoxy-5-methoxypyridin-2-yl)-2-(methylsulfonyl)et hyl)-3 -methyl-1H-benzo[d]imidazol-2(3H)-one;
(S)-5-(2,4-difluorophenyl)-1-(1-(6-ethoxy-5-methoxypyridin-2-yl)-2-(methylsulfonyl)et hyl)-1H-benzo[d]imidazol-2(3H)-one;
(S)-5-(2,4-difluorophenyl)-1-(1-(6-ethoxy-5-methoxypyridin-2-yl)-2-(methylsulfonyl)et hyl)-3 -methyl-1H-benzo[d]imidazol-2(3H)-one;
(S)-5-(2,4,5-trifluorophenyl)-1-(1-(6-ethoxy-5-methoxypyridin-2-yl)-2-(methylsulfonyl) ethyl)-1H-benzo[d]imidazol-2(3H)-one;
(S)-5-(2,4,5-trifluorophenyl)-1-(1-(6-ethoxy-5-methoxypyridin-2-yl)-2-(methylsulfonyl) ethyl)-3-methyl-1H-benzo[d]imidazol-2(3H)-one;
(S)-5-(2,5-difluorophenyl)-1-(1-(6-ethoxy-5-methoxypyridin-2-yl)-2-(methylsulfonyl)et hyl)-1H-benzo[d]imidazol-2(3H)-one;
(S)-5-(2,5-difluorophenyl)-1-(1-(6-ethoxy-5-methoxypyridin-2-yl)-2-(methylsulfonyl)et hyl)-3 -methyl-1H-benzo[d]imidazol-2(3H)-one;
(S)-5-(2,4-dimethylphenyl)-1-(1-(6-ethoxy-5-methoxypyridin-2-yl)-2-(methylsulfonyl)et hyl)-1H-benzo[d]imidazol-2(3H)-one;
(S)-5-(2,4-dimethylphenyl)-1-(1-(6-ethoxy-5-methoxypyridin-2-yl)-2-(methylsulfonyl)et hyl)-3 -methyl-1H-benzo[d]imidazol-2(3H)-one;
(S)-5-(2,5-dimethylphenyl)-1-(1-(6-ethoxy-5-methoxypyridin-2-yl)-2-(methylsulfonyl)et hyl)-1H-benzo[d]imidazol-2(3H)-one;
(S)-5-(2,5-dimethylphenyl)-1-(1-(6-ethoxy-5-methoxypyridin-2-yl)-2-(methylsulfonyl)et hyl)-3 -methyl-1H-benzo[d]imidazol-2(3H)-one;
(S)-5-(2,3-dimethylphenyl)-1-(1-(6-ethoxy-5-methoxypyridin-2-yl)-2-(methylsulfonyl)et hyl)-1H-benzo[d]imidazol-2(3H)-one;
(S)-5-(2,3-dimethylphenyl)-1-(1-(6-ethoxy-5-methoxypyridin-2-yl)-2-(methylsulfonyl)et hyl)-3 -methyl-1H-benzo[d]imidazol-2(3H)-one;
(S)-5-(2,6-dimethylphenyl)-1-(1-(6-ethoxy-5-methoxypyridin-2-yl)-2-(methylsulfonyl)et hyl)-1H-benzo[d]imidazol-2(3H)-one;
(S)-5-(4-fluoro-2-methylphenyl)-1-(1-(6-ethoxy-5-methoxypyridin-2-yl)-2-(methylsulfo nyl)ethyl)-1H-benzo[d]imidazol-2(3H)-one;
(S)-5-(4-fluoro-2-methylphenyl)-1-(1-(6-ethoxy-5-methoxypyridin-2-yl)-2-(methylsulfo nyl)ethyl)-3-methyl-1H-benzo[d]imidazol-2(3H)-one;
(S)-5-(5-fluoro-2-methylphenyl)-1-(1-(6-ethoxy-5-methoxypyridin-2-yl)-2-(methylsulfo nyl)ethyl)-1H-benzo[d]imidazol-2(3H)-one;
(S)-5-(5-fluoro-2-methylphenyl)-1-(1-(6-ethoxy-5-methoxypyridin-2-yl)-2-(methylsulfo nyl)ethyl)-3-methyl-1H-benzo[d]imidazol-2(3H)-one;
(S)-5-(3-fluoro-2-methylphenyl)-1-(1-(6-ethoxy-5-methoxypyridin-2-yl)-2-(methylsulfo nyl)ethyl)-1H-benzo[d]imidazol-2(3H)-one;
(S)-5-(3-fluoro-2-methylphenyl)-1-(1-(6-ethoxy-5-methoxypyridin-2-yl)-2-(methylsulfo nyl)ethyl)-3-methyl--1H-benzo[d]imidazol-2(3H)-one;
(S)-5-(2-fluoro-5-methylphenyl)-1-(1-(6-ethoxy-5-methoxypyridin-2-yl)-2-(methylsulfo nyl)ethyl)-1H-benzo[d]imidazol-2(3H)-one;
(S)-5-(2-fluoro-5-methylphenyl)-1-(1-(6-ethoxy-5-methoxypyridin-2-yl)-2-(methylsulfo nyl)ethyl)-3-methyl-1H-benzo[d]imidazol-2(3H)-one;
(S)-5-(2-fluoro-3-methylphenyl)-1-(1-(6-ethoxy-5-methoxypyridin-2-yl)-2-(methylsulfo nyl)ethyl)-1H-benzo[d]imidazol-2(3H)-one;
(S)-5-(2-fluoro-3-methylphenyl)-1-(1-(6-ethoxy-5-methoxypyridin-2-yl)-2-(methylsulfo nyl)ethyl)-3-methyl-1H-benzo[d]imidazol-2(3H)-one;
(S)-5-(2-fluoro-4-methylphenyl)-1-(1-(6-ethoxy-5-methoxypyridin-2-yl)-2-(methylsulfo nyl)ethyl)-1H-benzo[d]imidazol-2(3H)-one;
(S)-5-(2-fluoro-4-methylphenyl)-1-(1-(6-ethoxy-5-methoxypyridin-2-yl)-2-(methylsulfo nyl)ethyl)-3-methyl-1H-benzo[d]imidazol-2(3H)-one;
(S)-5-(2-fluoro-5-methoxyphenyl)-1-(1-(6-ethoxy-5-methoxypyridin-2-yl)-2-(methylsulf onyl)ethyl)-1H-benzo[d]imidazol-2(3H)-one;
(S)-5-(2-fluoro-5-methoxyphenyl)-1-(1-(6-ethoxy-5-methoxypyridin-2-yl)-2-(methylsulf onyl)ethyl)-3-methyl-1H-benzo[d]imidazol-2(3H)-one;
(S)-5-(2-fluoro-3-methoxyphenyl)-1-(1-(6-ethoxy-5-methoxypyridin-2-yl)-2-(methylsulf onyl)ethyl)-1H-benzo[d]imidazol-2(3H)-one;
(S)-5-(2-fluoro-4-methoxyphenyl)-1-(1-(6-ethoxy-5-methoxypyridin-2-yl)-2-(methylsulf onyl)ethyl)-1H-benzo[d]imidazol-2(3H)-one;
(S)-5-(2-fluoro-4-methoxyphenyl)-1-(1-(6-ethoxy-5-methoxypyridin-2-yl)-2-(methylsulf onyl)ethyl)-3-methyl-1H-benzo[d]imidazol-2(3H)-one;
(S)-5-(5-fluoro-2-methoxyphenyl)-1-(1-(6-ethoxy-5-methoxypyridin-2-yl)-2-(methylsulf onyl)ethyl)-1H-benzo[d]imidazol-2(3H)-one;
(S)-5-(5-fluoro-2-methoxyphenyl)-1-(1-(6-ethoxy-5-methoxypyridin-2-yl)-2-(methylsulf onyl)ethyl)-3-methyl-1H-benzo[d]imidazol-2(3H)-one;
(S)-5-(4-fluoro-2-methoxyphenyl)-1-(1-(6-ethoxy-5-methoxypyridin-2-yl)-2-(methylsulf onyl)ethyl)-1H-benzo[d]imidazol-2(3H)-one;
(S)-5-(4-fluoro-2-methoxyphenyl)-1-(1-(6-ethoxy-5-methoxypyridin-2-yl)-2-(methylsulf onyl)ethyl)-3-methyl-1H-benzo[d]imidazol-2(3H)-one;
(S)-5-(6-fluoro-2-methoxyphenyl)-1-(1-(6-ethoxy-5-methoxypyridin-2-yl)-2-(methylsulf onyl)ethyl)-1H-benzo[d]imidazol-2(3H)-one;
(S)-5-(6-fluoro-2-methoxyphenyl)-1-(1-(6-ethoxy-5-methoxypyridin-2-yl)-2-(methylsulf onyl)ethyl)-3-methyl-1H-benzo[d]imidazol-2(3H)-one;
(S)-1-(1-(1-(6-ethoxy-5-methoxypyridin-2-yl)-2-(methylsulfonyl)ethyl)-5-(2-ethoxyphe nyl)-1H-benzo[d]imidazol-2(3H)-one;
(R)-1-(1-(1-(6-ethoxy-5-methoxypyridin-2-yl)-2-(methylsulfonyl)ethyl)-5-(2-ethoxyphe nyl)-1H-benzo[d]imidazol-2(3H)-one;
(S)-1-(1-(1-(6-ethoxy-5-methoxypyridin-2-yl)-2-(methylsulfonyl)ethyl)-5-(pyridin-2-yl) -1H-benzo[d]imidazol-2(3H)-one;
(R)-1-(1-(1-(6-ethoxy-5-methoxypyridin-2-yl)-2-(methylsulfonyl)ethyl)-5-(pyridin-2-yl) -1H-benzo[d]imidazol-2(3H)-one;
(S)-1-(1-(6-ethoxy-5-methoxypyridin-2-yl)-2-(methylsulfonyl)ethyl)-3-methyl-5-(pyridi n-2-yl)-1H-benzo[d]imidazol-2(3H)-one;
(S)-1-(1-(1-(6-ethoxy-5-methoxypyridin-2-yl)-2-(methylsulfonyl)ethyl)-3-ethyl-5-(pyrid in-2-yl)-1H-benzo[d]imidazol-2(3H)-one;
(S)-1-(1-(6-ethoxy-5-methoxypyridin-2-yl)-2-(methylsulfonyl)ethyl)-3-(2-methoxyethyl )-5-(pyridin-2-yl)-1H-benzo[d]imidazol-2(3H)-one;
(S)-1-(1-(6-ethoxy-5-methoxypyridin-2-yl)-2-(methylsulfonyl)ethyl)-5-(4-fluoro-2-meth ylphenyl)-1H-benzo[d]imidazol-2(3H)-one;
(S)-1-(1-(6-ethoxy-5-methoxypyridin-2-yl)-2-(methylsulfonyl)ethyl)-5-(4-fluoro-2-meth ylphenyl)-3-methyl-1H-benzo[d]imidazol-2(3H)-one;
(S)-1-(1-(6-ethoxy-5-methoxypyridin-2-yl)-2-(methylsulfonyl)ethyl)-5-(2-fluoro-5-meth ylphenyl)-1H-benzo[d]imidazol-2(3H)-one;
(S)-1-(1-(6-ethoxy-5-methoxypyridin-2-yl)-2-(methylsulfonyl)ethyl)-5-(2-fluoro-5-meth ylphenyl)-3-methyl-1H-benzo[d]imidazol-2(3H)-one;
(S)-1-(1-(1-(6-ethoxy-5-methoxypyridin-2-yl)-2-(methylsulfonyl)ethyl)-4-(2-methoxyph enyl)-1H-benzo[d]imidazol-2(3H)-one;
(S)-1-(1-(1-ethoxy-5-methoxypyridin-2-yl)-2-(methylsulfonyl)ethyl)-4-(o-methylphenyl )-1H-benzo[d]imidazol-2(3H)-one;
(S)-1-(1-(1-(6-ethoxy-5-methoxypyridin-2-yl)-2-(methylsulfonyl)ethyl)-4-(2-fluorophen yl)-1H-benzo[d]imidazol-2(3H)-one;
(S)-1-(1-(1-(6-ethoxy-5-methoxypyridin-2-yl)-2-(methylsulfonyl)ethyl)-4-phenyl-1H-be nzo[d]imidazol-2(3H)-one;
(S)-1-(1-(1-(6-ethoxy-5-methoxypyridin-2-yl)-2-(methylsulfonyl)ethyl)-4-(2-fluorobenz yl)-1H-benzo[d]imidazol-2(3H)-one;
(S)-3-cyclopropyl-1-(1-(6-ethoxy-5-methoxypyridin-2-yl)-2-(methylsulfonyl)ethyl)-5-(2 -fluorophenyl)-1H-benzo[d]imidazol-2(3H)-one;
(S)-3-cyclopropyl-1-(1-(6-ethoxy-5-methoxypyridin-2-yl)-2-(methylsulfonyl)ethyl)-5-m ethyl-1H-benzo[d]imidazol-2(3H)-one;
(S)-5-bromo-1-(1-(6-ethoxy-5-methoxypyridin-2-yl)-2-(methylsulfonyl)ethyl)-3-(2-(met hylsulfonyl)ethyl)-1H-benzo[d]imidazol-2(3H)-one;
(S)-3-cyclopropyl-1-(1-(6-ethoxy-5-methoxypyridin-2-yl)-2-(methylsulfonyl)ethyl)-5-(tr ifluoromethyl)-1H-benzo[d]imidazol-2(3H)-one;
(S)-ethyl 1-(1-(1-(6-ethoxy-5-methoxypyridin-2-yl)-2-(methylsulfonyl)ethyl)-2-oxo-2,3 -dihydro-1H-benzo[d]imidazole-5-carboxylate;
(S)-isopropyl 1-(1-(1-(6-ethoxy-5-methoxypyridin-2-yl)-2-(methylsulfonyl)ethyl)-2-oxo -2,3-dihydro-1H-benzo[d]imidazole-5-carboxylate; and
(S)-2,2,2-trifluoroethyl 1-(1-(1-ethoxy-5-methoxypyridin-2-yl)-2-(methylsulfonyl)ethyl) -2-oxo-2,3-dihydro-1H-benzo[d]imidazole-5-carboxylate.

5. A compound of formula (II) and an isomer, solvate, deuterated derivative or pharmaceutically acceptable salt thereof: 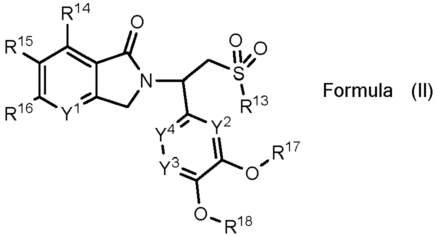 wherein:
Y¹ is selected from N or CH;
one of Y², Y³, and Y⁴ is selected from N, and the others are CH;
R¹³ is selected from C1-C3 alkyl and C1-C6 cycloalkyl, and may be substituted with halogen;
R¹⁴, R¹⁵, and R¹⁶ are independently selected from hydrogen, halogen, hydroxyl, CN, -NO₂, -NH₂, C1-C6 alkyl, C1-C6 alkoxy, C1-C6 alkylamino, C1-C6 alkylamido, C3-C6 cycloalkylamido, C2-C6 alkenyl, C3-C6 cycloalkenyl, 3- to 6-membered heterocycloalkenyl, C3-C6 cycloalkyl, 3- to 6-membered heterocyclyl, phenyl, and 5- to 6-membered heteroaryl, which may be optionally substituted with halogen, C3-C6 cycloalkyl, C1-C8 alkoxy, C3-C8 cycloalkoxy, 3- to 12-membered heterocyclyl, C1-C8 alkylamino, di(C1-C8 alkyl) substituted amino, phenyl, or 5- to 6-membered heteroaryl;
R¹⁷ and R¹⁸ are independently selected from C1-C6 alkyl and C3-C6 cycloalkyl, and may be optionally substituted with deuterium, tritium, or halogen.

6. The compound of formula (II) and the isomer, solvate, deuterated derivative or pharmaceutically acceptable salt thereof according to claim 5, wherein the compound of formula (II) comprises the following structures:
(S)-*N*-(2-(1-(6-ethoxy-5-methoxypyridin-2-yl)-2-(methylsulfonyl)ethyl)-3-oxoisoindolin -4-yl)acetamide;
(R)-*N*-(2-(1-(6-ethoxy-5-methoxypyridin-2-yl)-2-(methylsulfonyl)ethyl)-3-oxoisoindolin -4-yl)acetamide;
(S)-*N*-(2-(1-(6-ethoxy-5-methoxypyridin-2-yl)-2-(methylsulfonyl)ethyl)-3-oxoisoindolin -4-yl)cyclopropanecarboxamide;
(R)-*N*-(2-(1-(6-ethoxy-5-methoxypyridin-2-yl)-2-(methylsulfonyl)ethyl)-3-oxoisoindolin -4-yl)cyclopropanecarboxamide;
(S)-*N*-(2-(1-(6-ethoxy-5-methoxypyridin-2-yl)-2-(methylsulfonyl)ethyl)-3-oxoisoindolin -4-yl)propionamide; and
(R)-*N*-(2-(1-(6-ethoxy-5-methoxypyridin-2-yl)-2-(methylsulfonyl)ethyl)-3-oxoisoindolin -4-yl)propionamide.

7. A compound of formula (III) and an isomer, solvate, deuterated derivative or pharmaceutically acceptable salt thereof: 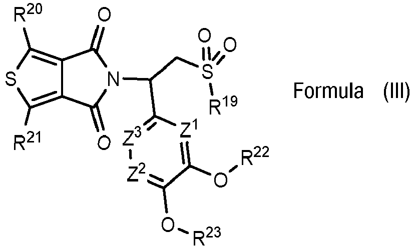 wherein:
R¹⁹ is selected from C₁-C₃ alkyl and C₃-C₆ cycloalkyl, and may be substituted with halogen;
R²⁰ and R²¹ are each selected from hydrogen, halogen, C1-C8 alkyl, C1-C8 alkoxy, hydroxyl, cyano, nitro, -NHC(O)R²⁶, -NHSO₂R²⁶, or -NR²⁴R²⁵;
R²² and R²³ are independently selected from C1-C6 alkyl, and may be optionally substituted with deuterium, tritium, or halogen;
R²⁴ and R²⁵ are each selected from hydrogen, C1-C8 alkyl, C6-C12 aryl, C(O)R²⁶, and SO₂R²⁶; alternatively, R²⁴ and R²⁵ are taken together with the N to which they are attached to form a 4- to 8-membered ring or -CH₂CH₂ZCH₂CH₂-;
Z is selected from O, S, and NR²⁶;
R²⁶ is hydrogen, C1-C6 alkyl, C3-C6 cycloalkyl, or C1-C8 alkylamino, and may be optionally substituted with halogen, C1-C8 alkoxy, C3-C8 cycloalkoxy, 3- to 12-membered heterocyclyl, C1-C8 alkylamino, or di(C1-C8 alkyl) substituted amino.

8. The compound of formula (III) and the isomer, solvate, deuterated derivative or pharmaceutically acceptable salt thereof according to claim 7, wherein the compound of formula (III) comprises the following structures:
5-(1-(6-ethoxy-5-methoxypyridin-2-yl)-2-(methylsulfonyl)ethyl)-1-nitro-4H-thieno[3,4-c]pyrrol-4,6 (5H)-dione;
(S)-5-(1-(6-ethoxy-5-methoxypyridin-2-yl)-2-(methylsulfonyl)ethyl)-1-nitro-4H-thieno[ 3,4-c]pyrrol-4,6 (5H)-dione;
(S)-1-amino-5-(1-(6-ethoxy-5-methoxypyridin-2-yl)-2-(methylsulfonyl)ethyl)-4H-thieno [3,4-c]pyrrol-4,6 (5H)-dione;
N-(5-(1-(6-ethoxy-5-methoxypyridin-2-yl)-2-(methylsulfonyl)ethyl)-4,6-dioxo-5,6-dihy dro-4H-thieno[3,4-c]pyrrol-1-yl)acetamide;
(S)-N-(5-(1-(6-ethoxy-5-methoxypyridin-2-yl)-2-(methylsulfonyl)ethyl)-4,6-dioxo-5,6-d ihydro-4H-thieno[3,4-c]pyrrol-1-yl)acetamide;
(R)-N-(5-(1-(6-ethoxy-5-methoxypyridin-2-yl)-2-(methylsulfonyl)ethyl)-4,6-dioxo-5,6-d ihydro-4H-thieno[3,4-c]pyrrol-1-yl)acetamide;
(S)-N-(5-(1-(6-ethoxy-5-methoxypyridin-2-yl)-2-(methylsulfonyl)ethyl)-4,6-dioxo-5,6-d ihydro-4H-thieno[3,4-c]pyrrol-1-yl)propionamide;
(S)-N-(5-(1-(6-ethoxy-5-methoxypyridin-2-yl)-2-(methylsulfonyl)ethyl)-4,6-dioxo-5,6-d ihydro-4H-thieno[3,4-c]pyrrol-1-yl)cyclopropanecarboxamide;
(S)-2-(dimethylamino)-N-(5-(1-(6-ethoxy-5-methoxypyridin-2-yl)-2-(methylsulfonyl)et hyl)-4,6-dioxo-5,6-dihydro-4H-thieno[3,4-c]pyrrol-1-yl)acetamide;
(S)-2-(diethylamino)-N-(5-(1-(6-ethoxy-5-methoxypyridin-2-yl)-2-(methylsulfonyl)ethy l)-4,6-dioxo-5,6-dihydro-4H-thieno[3,4-c]pyrrol-1-yl)acetamide;
(S)-N-(5-(1-(6-ethoxy-5-methoxypyridin-2-yl)-2-(methylsulfonyl)ethyl)-4,6-dioxo-5,6-d ihydro-4H-thieno[3,4-c]pyrrol-1-yl)-2-(piperidin-1-yl)acetamide;
(S)-N-(5-(1-(6-ethoxy-5-methoxypyridin-2-yl)-2-(methylsulfonyl)ethyl)-4,6-dioxo-5,6-d ihydro-4H-thieno[3,4-c]pyrrol-1-yl)-2-(pyrrolidin-1-yl)acetamide; and
(S)-N-(5-(1-(6-ethoxy-5-methoxypyridin-2-yl)-2-(methylsulfonyl)ethyl)-4,6-dioxo-5,6-d ihydro-4H-thieno[3,4-c]pyrrol-1-yl)-2-morpholinoacetamide.

9. Use of the compound and the isomer, solvate, deuterated derivative or pharmaceutically acceptable salt thereof according to any one of claims 1-8 in the manufacture of a medicament for treating a disease related to PDE4.

10. The use according to claim 9, wherein the disease related to PDE4 is immune inflammatory and autoimmune diseases or conditions comprising systemic lupus erythematosus (SLE), lupus nephritis, psoriasis, cutaneous lupus, Crohn's Disease, ulcerative colitis, type 1 diabetes, rheumatoid arthritis, systemic-onset juvenile idiopathic arthritis, atopic dermatitis, ankylosing spondylitis, or multiple sclerosis.
